# EUROPEAN PATENT APPLICATION

(11) **EP 2 589 665 A1**
(43) Date of publication of application: **08.05.2013**
(21) Application number: 11800481.1
(22) Date of filing: 30.03.2011
(51) Int. Cl.: C12Q 1/68, C12N 15/09, G01N 33/53

(54) **METHOD FOR PREDICTING THERAPEUTIC EFFECT OF IMMUNOTHERAPY ON CANCER PATIENT, AND GENE SET AND KIT TO BE USED IN THE METHOD**

(30) Priority: 29.06.2010 JP 2010147797
(71) Applicant: Kurume University, Kurume-shi Fukuoka 8300011 (JP); Kyushu University National University Corporation, Higashi-ku Fukuoka-shi Fukuoka 812-8581 (JP); Kurume Research Park Co., Ltd., Fukuoka 839-0864 (JP)
(72) Inventor: ITOH, Kyogo, Kurume-shi Fukuoka 830-0011 (JP); NOGUCHI, Masanori, Kurume-shi Fukuoka 830-0011 (JP); KUHARA, Satoru, Fukuoka-shi Fukuoka 812-0053 (JP); YAMADA, Akira, Kurume-shi Fukuoka 830-0011 (JP); SHICHIJO, Shigeki, Kurume-shi Fukuoka 830-0011 (JP); KOMATSU, Nobukazu, Kurume-shi Fukuoka 830-0011 (JP); TASHIRO, Kosuke, Fukuoka-shi Fukuoka 812-0053 (JP)
(74) Representative: Vossius & Partner
(86) International application number: PCT/JP2011/058094
(87) International publication number: WO 2012/002011

(57) **Abstract**

Provided is a gene set which is useful for predicting the therapeutic effect of an immunotherapy on a cancer patient. Also provided is a method for examining whether an immunotherapy is efficacious or not, said method comprising quantifying the expression amount of each of the genes constituting the aforesaid gene set. This examination method is useful for determining a therapeutic strategy for the cancer patient.

## Description

### Technical Field

The present invention relates to a method for predicting therapeutic effect of immunotherapy on a cancer patient and a gene set and a kit for use in the method, etc.

### Background Art

Immunotherapy for various cancers has not yet been the optimum treatment for every patient, although it is effective in some cases. One reason for that is because the immunotherapy is mediated by immunological competence, which differs by individuals, in order to suppress the growth of cancer cells. So far, a method for predicting effect of cancer immunotherapy has not been found. Thus, the efficacy cannot be evaluated until the treatment is actually performed. Although a method for predicting effect of chemotherapy on a breast cancer patient has been known to be performed by measuring a gene expression level, the method is complicated method that requires combining gene expression with other factors, and also, the method is intended for predicting only effect of chemotherapy for breast cancer (Patent Document 1). A method for predicting therapeutic effect of cancer immunotherapy or prognosis of a patient after immunotherapy has been unknown so far.
Patent Document 1
   JP-A-2008-536094

### Disclosure of the Invention

### Problem to be Solved by the Invention

An object of the present invention is to provide a method for accurately predicting therapeutic effect of immunotherapy on a cancer patient.

### Means for Solving the Problem

The present inventors attempted to predict therapeutic effect of cancer immunotherapy based on the results which had been obtained by conducting peptide vaccine therapy for prostate cancer patients over long years. First, gene expression profiles in prostate cancer patients before or after peptide vaccine therapy were analyzed with DNA microarrays. Subsequently, the patients were classified into a good prognosis group and a poor prognosis group on the basis of a survival time after the treatment, and a gene or a gene group was selected for accurately predicting whether the patient belongs to the good prognosis group or the poor prognosis group based on the expression level before the treatment. Then, it was confirmed that the therapeutic effect of immunotherapy could be predicted from the expression level(s) of the selected gene(s). In this way, the present invention has been completed.

Accordingly, in one aspect, the present invention provides a method for predicting effect of immunotherapy on a cancer patient, comprising a step of (1) measuring an expression level of each gene included in a gene set in a sample obtained from the cancer patient before or after the immunotherapy, wherein the gene set consists of at least one gene selected from the group of genes shown in Table 1, 19, 34, or 35.
The method for predicting effect of immunotherapy on a cancer patient may further comprise a step of (2) determining prognosis of the patient by discriminant analysis using the expression level.

In an another aspect, the present invention provides a gene set for predicting effect of immunotherapy on a cancer patient, consisting of at least one gene selected from the group of genes shown in Table 1, 19, 34 or 35, and a biomarker for predicting effect of immunotherapy on a cancer patient, consisting of at least one gene selected from the group of genes shown in Table 1, 19, 34 or 35.

In a further aspect, the present invention provides a probe and a primer for each gene included in the gene set, and a kit for predicting effect of immunotherapy on a cancer patient, comprising a probe and primers for each gene included in the gene set and/or an antibody specifically recognizing an expression product of each gene included in the gene set.

In a still further aspect, the present invention provides a method for screening for a cancer patient for whom immunotherapy is effective, comprising the step of (1) measuring the expression level of each gene included in a gene set consisting of at least one gene selected from the group of genes shown in Table 1, 19, 34, or 35 in a sample obtained from the cancer patient before the immunotherapy.
The method for screening for a cancer patient may further comprise a step of
(2) determining prognosis of the patient by discriminant analysis using the expression level.
In a still further aspect, the present invention provides a method for predicting effect of immunotherapy on a cancer patient, comprising a step of measuring an expression level of IL-6 protein in blood obtained from the cancer patient before or after the immunotherapy.
The level of IL-6 protein in blood can also serve as a biomarker for predicting effect of immunotherapy on a cancer patient.

### Effects of the Invention

According to the present invention, effect of immunotherapy on a patient can be predicted by determination of gene expression profiles of the cancer patient before the start of immunotherapy. The present invention enables prediction of patients for whom immunotherapy is not effective (poor prognosis group), and the present invention provides useful information for choosing a treatment method for cancer patients.

### Brief Description of the Drawings

Figure 1 shows the distribution of 16968 genes in t-test. The ordinate represents the level of significance between two groups, and the abscissa represents the logarithm of the gene expression ratio between two groups. n = 40 (20 short-lived individuals and 20 long-lived individuals);
Figure 2 shows the distribution of 16968 genes in Wilcoxon test. The ordinate represents the level of significance between two groups, and the abscissa represents the gene expression ratio between two groups. n = 40 (20 short-lived individuals and 20 long-lived individuals);
Figure 3 shows the distribution of 13 genes in t-test. The ordinate represents the level of significance between two groups, and the abscissa represents the logarithm of the gene expression ratio between two groups. n = 40 (20 short-lived individuals and 20 long-lived individuals);
Figure 4 shows the distribution of 13 genes in Wilcoxon test. The ordinate represents the level of significance between two groups, and the abscissa represents the gene expression ratio between two groups. n = 40 (20 short-lived individuals and 20 long-lived individuals);
Figure 5 shows genes advantageous for discrimination. The ordinate represents the frequencies of the genes for use in gene sets that offers a discrimination rate of 80% or more;
Figure 6 shows the difference in gene expression in peripheral blood mononuclear cells (PBMCs) between a long-lived group and a short-lived group before vaccination (A) or after vaccination (B);
Figure 7 shows results of determining the gene expression levels of DEFA1 (A), DEFA4 (B), CEACAM8 (C) and MPO (D) in the peripheral blood mononuclear cells (PBMCs) of a long-lived group (Long) and a short-lived group (Short) after vaccination by real-time PCR. The expression level of each gene was determined with GAPDH as an internal standard; and
Figure 8 shows results of determining the level of IL-6 protein in the plasmas of patients in a long-lived group (Long) and a short-lived group (Short). Mann-Whitney test was used as a statistical test.

### Best Mode for Carrying Out the Invention

In one aspect, the present invention provides a method for predicting effect of immunotherapy on a cancer patient, comprising measuring the expression levels of one or more genes in a sample obtained from the cancer patient. For the above-described prediction method, a gene set which is available for accurate prediction of whether a cancer patient after given immunotherapy belongs to a good prognosis group or a poor prognosis group based on the expression level is utilized. For the above-described prediction method, an expression level of at least one gene selected from the group of genes shown in Table 1, 19, 34, or 35 is utilized. The above-described prediction method can be used as a method for predicting a patient for whom immunotherapy is not expected to be effective; a patient for whom immunotherapy is expected to be effective; or a patient who is resistant to immunotherapy, and determining whether immunotherapy is applicable or not.

A gene set, which is used for the above-described prediction method provided as one aspect of the present invention, can be selected arbitrarily from 54 genes shown in Table 1, 100 genes shown in Table 19, 36 genes shown in Table 34, 19 genes shown in Table 35 and/or IL-6 gene without being limited by the number and the kind of the gene.

Preferably, the gene set for use in the above-described prediction method provided as one aspect of the present invention consists of 1, 2, 3, 4, 5, 6, 7, 8, 9, 10, 11, 12, or 13 genes selected from 13 genes shown in Table 2. The gene set preferably comprises 4 genes: LOC653600, TNFRSF19, P4HA1, and SONE1. Specific examples of the gene set include a gene set consisting of LOC653600, TNFRSF19, P4HA1 and SYNE1; a gene set consisting of LOC653600, TNFRSF19, G3BP2, ZNF83, C6orf222, ZBTB20, P4HA1, GP1BA, HLA-A29.1, SYNE1 and NAP1L1; and gene sets represented by No. 1 of Table 10, Nos. 1 to 18 of Table 11, Nos. 1 to 55 of Table 12, Nos. 1 to 71 of Table 13, Nos. 1 to 63 of Table 14, Nos. 1 to 45 of Table 15, Nos. 1 to 22 of Table 16 or Nos. 1 to 7 of Table 17.

In another preferable embodiment, the gene set for use in the above-described prediction method provided as one aspect of the present invention consists of 1 to 29 genes (e.g. 1, 2, 3, 4, 5, 6, 7, 8, 9, 10 and 11 genes), selected from 29 genes shown in Table 20. Specific examples of the gene set include gene sets shown in Tables 23 to 33. Those skilled in the art can appropriately select even a gene set comprising 12 or more genes according to the description of Examples.

In another preferable embodiment, the gene set for use in the above-described prediction method provided as one aspect of the present invention consists of at least one gene selected from genes shown in Table 34. The gene set preferably comprises 4 genes: DEFA1, DEFA4, CEACAM8 and MPO. Specific examples of the gene set include a gene set consisting of DEFA1, DEFA4, CEACAM8 and MPO, and a gene set consisting of at least one gene selected from the group consisting of DEFA1, DEAF3, DEFA4, ELA2, CSTG, CAMP, MPO, MUMP9 and CEACAM8.

In another preferable embodiment, the gene set for use in the above-described prediction method provided as one aspect of the present invention consists of at least one gene selected from genes shown in Table 35 (PRKAR1A, LRRN3, PCDH17, TTN, LAIR2, RNASE3, CEACAM6, AZU1, HIST1H4C, PGLYRP1, CEACAM8, LCN2, MPO, CAMP, DEFA1, DEFA3, CTSG, DEFA4 and ELA2). The gene set preferably comprises 4 genes: LRRN3, PCDH17, HIST1H9C and PGLYRP1. Specific examples of the gene set include a gene set consisting of LRRN3, PCDH17, HIST1H9C and PGLYRP1, and a gene set consisting of at least one gene selected from the group consisting of LRRN3, PCDH17, HIST1H4C and PGLYRP1.
In another preferable embodiment, the gene set for use in the above-described prediction method provided as one aspect of the present invention consists of IL-6 gene or comprises IL-6 gene.
In another preferable embodiment, examples of the gene set for use in the above-described prediction method provided as one aspect of the present invention include a gene set consisting of at least one gene selected from the group consisting of LOC653600, TNFRSF19, P4HA1, SYNE1, DEFA1, DEFA4, CEACAM8, MPO, LRRN3, PCDH17, HIST1H4C and PGLYRP1, and a gene set comprising at least one gene selected from the group consisting of LOC653600, TNFRSF19, P4HA1, SYNE1, DEFA1, DEFA4, CEACAM8, MPO, LRRN3, PCDH17, HIST1H4C and PGLYRP1.

In the present specification, a cancer patient is not particularly limited as long as the cancer patient is a human. Examples thereof include patients with prostate cancer, pancreatic cancer, breast cancer, liver cancer or the like. The prostate cancer may be progressive recurrent prostate cancer.

In the present specification, an immunotherapy means a method for treating cancer by activating immune response to tumor antigen proteins in the cancer patient. Examples of immunotherapy include peptide vaccine therapy using tumor antigen peptides; adoptive immunotherapy using lymphocytes such as cytotoxic T cells or natural killer cells; DNA vaccine therapy which involves introducing viral vectors expressing tumor antigen proteins or tumor antigen peptides into organisms; and dendritic cell vaccine therapy which involves administering dendritic cells displaying tumor antigen peptides. One preferable example of the immunotherapy, includes peptide vaccine therapy.

An expression level of each gene can be measured by a conventional method. Examples of the method for measuring the expression level include DNA microarray, DNA chip, PCR (including real-time PCR) and Northern blot methods. One preferable example of the method for measuring the expression level includes a DNA microarray method.

A DNA microarray method is performed using a microarray comprising a probe for a gene to be measured. One example of the available microarray includes HumanWG-6 v3.0 Expression BeadChip manufactured by Illumina, Inc. Alternatively, a probe for a gene to be measured may be synthesized and immobilized on an appropriate substrate such as slide glass to prepare a desired microarray. The method for preparing a microarray is well known in the art. The analysis of microarray data is also well known and can be performed with reference to, for example, "Microarrays for an Integrative Genomics" (translated by Yujin Hoshida, published by Springer-Verlag Tokyo, Inc.).

A sample of human patient for use in measurement of a gene expression level is not limited, and, for example, peripheral blood obtained from a patient can be used. Myeloid dendritic cells (MDCs), granalocytic MDSCs, peripheral blood mononuclear cells (PBMCs), granulocytes or erythrocytes in the peripheral blood may be used for the measurement of the gene expression level. Also, the patient-derived sample may be a sample obtained before immunotherapy, a sample obtained after immunotherapy, or samples obtained before and after immunotherapy. The above-described prediction method provided as one aspect of the present invention may be carried out using the sample obtained before immunotherapy in order to predict whether immunotherapy is not expected to be effective for the patient or immunotherapy is expected to be effective for the patient, and determine whether immunotherapy is applicable or not. Alternatively, the above-described prediction method provided as one aspect of the present invention may be carried out using the sample obtained after immunotherapy in order to predict whether or not after the sample is obtained, immunotherapy is expected to be effective for the patient, and determine whether further immunotherapy is applicable or not.

A measurement of a gene expression level using a DNA microarray method is, for example, described as follows. Firstly, total RNA is extracted from the peripheral blood of a patient and purified. Subsequently, biotinylated cRNA is synthesized using Illumina TotalPrep RNA Amplification Kit (manufactured by Life Technologies Corp. (Ambion)) or the like. This biotinylated cRNA is hybridized to a microarray and then reacted with Cy3-labeled streptavidin. The microarray after the reaction is scanned with a specific scanner. The Cy3 fluorescence of each spot can be quantified using specific software such as BeadStudio to obtain an expression level of each gene.

When a gene expression level is measured by PCR, for example, cDNA can be prepared from mRNA in a sample and used as a template in PCR to determine the gene expression level in the sample. For determination of a gene expression level by PCR, real-time PCR may be used. Primers for use in PCR can be designed appropriately by those skilled in the art to be capable of specifically hybridizing to a gene of interest. Also, for the real-time PCR, a probe that specifically hybridizes to a gene of interest and is bound with a fluorescent dye to allow determination of a PCR product is used. The probe can be designed appropriately by those skilled in the art. The real-time PCR may be performed using a fluorescent dye such as SYBR (registered trademark) Green.

Alternatively, an expression level of each gene may be measured by measuring an expression level of a protein which is an expression product of the gene. A protein localized in a cell membrane or a cytoplasm can be measured by flow cytometry using an antibody labeled with a fluorescent dye. Alternatively, an enzyme antibody method (ELISA), a Western blot method, or the like may be used.

The method for predicting effect of immunotherapy on a cancer patient provided as one aspect of the present invention comprises measuring an expression level of one or more genes in a sample obtained from the cancer patient and may further comprise conducting discriminant analysis using the measured expression level. As a result of the discriminant analysis, prognosis of the patient, such as whether the effect of immunotherapy would be observed, can be determined. The discriminant analysis can be carried out, for example, as described in Examples. Specifically, genes for use in the determination are selected, and discriminant functions based on known data (training data) are obtained. Then, the expression levels of the genes in a patient as a subject are applied thereto to calculate the probability of 1 (long life) or 0 (short life) for the patient. The prognosis of the patient is determined according to results in which the probability exceeds 50% (long life (good prognosis) or short life (poor prognosis)). When the probability of long life (good prognosis) exceeds 50%, the patient is predicted to respond to the immunotherapy. By contrast, when the probability of short life (poor prognosis) exceeds 50%, the patient is predicted not to respond to the immunotherapy.

The discriminant analysis can be conducted using statistical analysis software SAS (SAS Institute Japan Ltd.), statistical analysis software JMP (SAS Institute Japan Ltd.) or the like. The number of training data is not particularly limited. Those skilled in the art can appropriately determine the number of training data that achieves prediction.

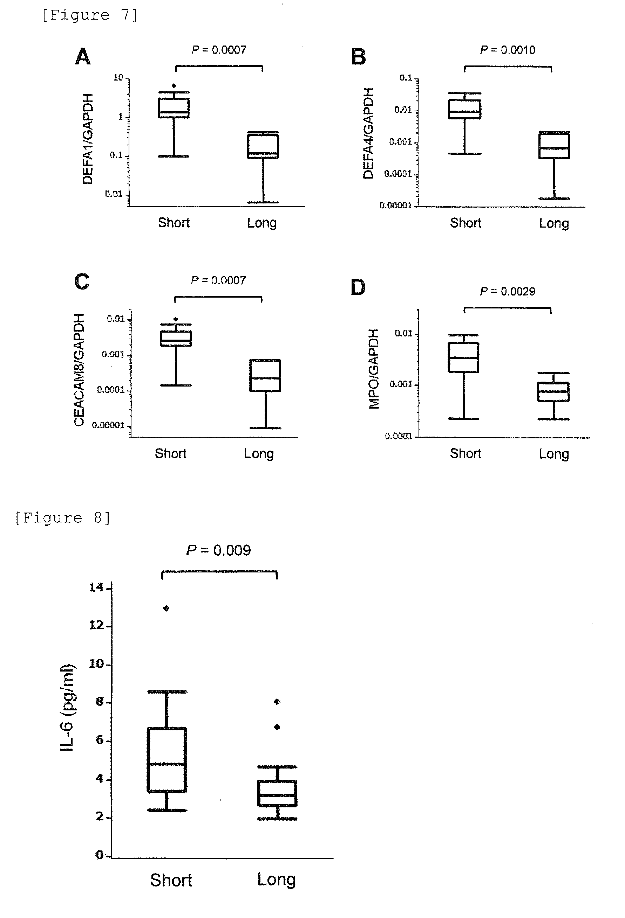

Alternatively, a standard expression level (standard values of expression levels in long-lived or short-lived individuals) of genes for use in determination may be preliminarily determined for each of long-lived (good prognosis) and short-lived (poor prognosis) individuals in a sufficient number of cases (for example, 100 or 1000 cases). The standard value may be compared with the expression level of the gene in a patient as a subject to determine the prognosis of the patient. For example, when expression levels of DEFA1, DEFA4, CEACAM8 and MPO genes are higher than the standard values of the long-lived group, the patient is a patient for whom immunotherapy cannot be expected to be effective and is determined not to have good prognosis. The standard values of respective expression levels of DEFA1, DEFA4, CEACAM8 and MPO genes can be selected, for example, as shown in Figure 7.

In Examples described below, genes were selected on the basis of a survival time of 480 days, and the accuracy of the determination was confirmed on the basis of a survival time of 480 days. However, 40 prostate cancer patients of Examples were only patients with a survival time of 900 days or longer or a survival time of 300 days or shorter. Therefore, the same gene set as that of the present invention can be selected even on the basis of any of survival times of 301 to 899 days. Accordingly, the number of days on which the definition of long life or short life is based may be any number of days within the survival time range of 301 to 899 days and is not limited to 480 days.

The above-described method for predicting effect of immunotherapy on a cancer patient provided as one aspect of the present invention can be used as a method for predicting whether immunotherapy is not expected to be effective for a patient, or whether immunotherapy is expected to be effective for a patient, and determine whether immunotherapy is applicable or not. Accordingly, the above-described method for predicting effect of immunotherapy on a cancer patient may be a method for screening for a cancer patient predicted to respond to immunotherapy.
In addition, a physician can determine a therapeutic strategy for a cancer patient on the basis of results obtained by the above-described method for predicting effect of immunotherapy on a cancer patient. Accordingly, in one aspect, the present invention provides a method for diagnosing or treating cancer, comprising performing the above-described method for predicting effect of immunotherapy on a cancer patient.

In one aspect, the present invention also provides a gene set that is used for predicting effect of immunotherapy on a cancer patient. The gene set is the same as the gene set for use in the above-described method for predicting effect of immunotherapy on a cancer patient provided as one aspect of the present invention and can be used for preparing a probe for a DNA. microarray, a primer for PCR, or the like that is used in the prediction method of the present invention.

In one aspect, the present invention also provides a biomarker for predicting effect of immunotherapy on a cancer patient. In the present specification, the biomarker can serve as an index for predicting effect of immunotherapy. The biomarker may be a gene or may be a protein expressed therefrom. The gene set for use in the above-described method for predicting effect of immunotherapy on a cancer patient provided as one aspect of the present invention can be used as the biomarker. For example, the biomarker may be at least one gene selected from the group of genes shown in Table 1, 19, 34 or 35; a gene set comprising LOC653600, TNFRSF19, P4HA1 and SONE1; a gene set comprising DEFA1, DEFA4, CEACAM8 and MPO; or a gene set comprising LRRN3, PCDH17, HIST1H4C and PGLYRP1. When DEFA1, DEFA4, CEACAM8 and MPO are used as biomarkers, the higher expression levels of these genes than the standard values of the long-lived group can serve as an index for predicting the patient not to respond to the immunotherapy.
Alternatively, a protein which is expression product of the gene set for use in the above-described method for predicting effect of immunotherapy on a cancer patient provided as one aspect of the present invention may be used as a biomarker for predicting effect of immunotherapy on a cancer patient. For example, IL-6 protein in blood may be used as a biomarker. The larger level of the IL-6 protein in the blood of a patient than the standard value of the long-lived group can serve as an index for predicting the patient not to respond to the immunotherapy. The standard value of the level of IL-6 protein in blood may be set to, for example, 4.8 pg/ml for the short-lived group and 3.3 pg/ml for the long-lived group with reference to Figure 8.

In one aspect, the present invention also provides a probe, primers or an antibody that is available for measurement of the expression level of each gene in the gene set of the present invention or its gene expression product. The probe and the primers for each gene can be synthesized by a conventional method on the basis of sequence information about the gene. The probe and the primers have, for example, a sequence partially complementary to the sequence of each gene and can specifically hybridize to the gene.
As used herein, the term "specifically hybridize" refers to, for example, "hybridizing under stringent conditions". The "stringent conditions" can be determined appropriately by those skilled in the art with reference to, for example, Molecular Cloning: A Laboratory Manual, 3rd edition (2001). Examples thereof include 0.2 x SSC, 0.1% SDS, and 65°C.
The primers can be designed to be capable of being used in PCR for amplifying each gene or a portion thereof. The sequence information about each gene can be obtained according to GenBank Accession numbers described in the tables of the present specification. Also, a method for preparing the antibody is well known in the art ("Antibodies: A Laboratory Manual", Lane, H. D. et al. eds., Cold Spring Harbor Laboratory Press, New York, 1989).
For example, the respective probes and primers of DEFA1, DEFA4, CEACAM8 and MPO may be the oligonucleotides of SEQ ID NOs: 1 to 8 described in Examples.
The antibody may be a polyclonal antibody or may be a monoclonal antibody. Alternatively, the antibody may be an antibody fragment such as Fab, F(ab')₂ and Fv.

In one aspect, the present invention also provides a kit for predicting effect of immunotherapy on a cancer patient, comprising the above-described probe, the above-described primers and/or the above-described antibody.
The kit of the present invention is a kit that is used for, for example, DNA microarray, DNA chip, PCR (including real-time PCR), Northern blot, fluorescent antibody, enzyme antibody and Western blot methods. Examples of the kit for the DNA microarray method include those comprising a microarray comprising the above-described probe immobilized on an appropriate substrate.
The kit may also comprise, for example, an anti-IL-6 polyclonal antibody or an anti-IL-6 monoclonal antibody for determining the level of IL-6 protein in blood.
The kit may also comprise other necessary reagents according to the measurement method.

In one aspect, the present invention also provides a method for selecting a gene set for predicting effect of immunotherapy on a cancer patient. The method for selecting a gene set comprises, for example, step 1: a step of determining an expression level of a gene expressed in a sample derived from the cancer patient group where immunotherapy is effective for the patient (long-lived group) and where immunotherapy is not effective for the patient (short-lived group); step 2: a step of selecting a gene capable of serving as a marker for predicting effect of immunotherapy, on the basis of the difference in gene expression level between the group where immunotherapy is effective for the patient (long-lived group) and where immunotherapy is not effective for the patient (short-lived group) and statistically significant difference thereof; and step 3: a step of determining the best combination for predicting effect of immunotherapy by variable selection from the selected genes.
The difference in gene expression level may be evaluated, for example, on the basis of a value of log₂ "expression level in a short-lived group / expression level in a long-lived group" after determining the expression level in the short-lived group / the expression level in the long-lived group.
The statistically significant difference may be determined by t-test and/or Wilcoxon test or may be determined by the Limma method (see Smyth. Linear models and empirical Bayes methods for assessing differential expression in microarray experiments. Statistical applications in genetics and molecular biology (2004) vol. 3, pp. Article).
For example, in the above-described step 2, a gene that satisfies the conditions of log₂ "expression level in the short-lived group / expression level in the long-lived group" < -1.0 or > 1.0 and P-value (limma) < 0.01 may be selected.
The variable selection can be carried out appropriately by those skilled in the art. For example, the stepwise discriminant analysis (SDA) described in Sungwoo Kwon et al: DNA Microarray Data Analysis for Cancer Classification Based on Stepwise Discriminant Analysis and Bayesian Decision Theory. Genome Informatics 12: 252-254 (2001) may be carried out. Examples of SDA include the implementation of the following (i) to (iii):
(i) when Wilk's lambda is evidently decreased by adding one gene to a gene set, adding the gene to the gene set;
(ii) when Wilk's lambda is slightly increased by removing one gene from a gene set, removing the gene from the gene set; and
(iii) repeating the steps (i) and (ii) until the Wilk's lambda no longer changes from a statistical standpoint.

Hereinafter, the present invention will be further described with reference to Examples. However, the present invention is not intended to be limited to them.

### Example

### Example 1

### 1: DNA microarray study of gene expression profile before peptide vaccine therapy

The patient-derived samples used were peripheral blood that was obtained from each prostate cancer patient who gave informed consent according to a protocol approved by the ethics committee of Kurume University when the patient was diagnosed as having recurrent prostate cancer in the past clinical trial. 40 prostate cancer patients were examined for their gene expression profiles before peptide vaccine therapy using DNA microarrays (HumanWG-6 v3.0 Expression BeadChip manufactured by Illumina, Inc.). The prostate cancer patients were 20 individuals in a good prognosis group (survival time of 900 days or longer after peptide vaccine therapy) and 20 individuals in a poor prognosis group (survival time of 300 days or shorter after peptide vaccine therapy).

### (I) RNA extraction and purification from peripheral blood of patient

1. To the peripheral blood sample of each patient, TRIzol LS (manufactured by Invitrogen Corp.) was added at a ratio of 1:3 and mixed therewith.
2. 200 µL of chloroform with respect to 750 µL of the TRIzol LS solution was added and mixed therewith, followed by centrifugation.
3. The supernatant was transferred to a new tube, to which ethanol was added in an amount of 0.55 times the volume of the supernatant.
4. The sample of 3 was placed on the column of SV Total RNA Isolation System (manufactured by Promega Corp.) and applied to a filter.
5. The filter was washed with 500 µL of Wash Buffer.
6. Total RNA was eluted with 80 µL of Nuclease Free Water.
7. The concentration of the RNA was measured using a spectrophotometer, and the quality of the RNA was checked by electrophoresis using Experion System (manufactured by Bio-Rad Laboratories, Inc.).

### (II) Synthesis of cRNA for microarray using Illumina TotalPrep RNA Amplification Kit (manufactured by Life Technologies Corp. (Ambion))

### (1) Synthesis of single-stranded cDNA by reverse transcription

1. Nuclease Free Water was added to 500 µg of each total RNA to adjust the volume to 11 µL.
2. 9 µL of Reverse Transcription Master Mix was added to the solution of 1, and the mixture was incubated at 42°C for 2 hours.

### (2) Synthesis of double-stranded cDNA

1. 80 µL of Second Strand Master Mix was added to each tube of (1)2.
2. Each tube was incubated at 16°C for 2 hours.

### (3) cDNA purification

1. 250 µL of cDNA Binding Buffer was added to each tube.
2. The solution of 1 was placed on cDNA Filter Cartridge and applied to a filter by centrifugation.
3. The filter was washed with 500 µL of Wash Buffer.
4. cDNA was eluted with 19 µL of Nuclease Free Water preheated to 50 to 55°C.

### (4) cRNA synthesis through in vitro transcription reaction

1. 7.5 µL of IVT Master Mix was added to the cDNA sample obtained in (3)4.
2. The tube of 1 was incubated at 37°C for 14 hours.
3. 75 µL of Nuclease Free Water was added to the tube of 2.

### (5) cRNA purification

1. 350 µL of cRNA Binding Buffer was added to each tube.
2. 250 µL of 100% ethanol was added to each tube and mixed.
3. The sample of 2 was placed on cRNA Filter Cartridge and applied to a filter by centrifugation.
4. The filter was washed with 650 µL of Wash Buffer.
5. cRNA was eluted with 100 µL of Nuclease Free Water preheated to 50 to 55°C.
6. The concentration of the cRNA was measured on the basis of OD, and the cRNA was then used as a hybridization sample.

### (III) Microarray hybridization

(1) Preparation of cRNA for hybridization
   1. Nuclease Free Water was added to 500 µg of each total RNA to adjust the volume to 10 µL.
   2. 20 µL of GEX-HYB was added to the solution of 1, and the mixture was incubated at 65°C for 5 minutes.
(2) Hybridization
   1. The prepared cRNA sample was applied to HumanWG-6 v3.0 Expression BeadChip loaded in a specific chamber.
   2. The lid of the specific chamber was closed, followed by incubation at 55°C for 18 hours.

### (IV) Microarray washing and staining

(1) Washing of array
   1. The cover of the microarray was removed in Wash E1BC solution.
   2. The array was immediately loaded in a slide rack and washed for 10 minutes in 1 x High-Temp Wash buffer preheated to 55°C.
   3. The array was washed for 5 minutes in Wash E1BC solution.
   4. The array was washed for 5 minutes in ethanol.
   5. The array was washed for 5 minutes in Wash E1BC solution.
   6. 4 ml of Block E1 buffer was prepared in a staining-specific tray, and each array was loaded therein one by one and blocked at room temperature for 10 minutes.
   7. 2 µL of streptavidin-Cy3 with respect to 2 ml of Block E1 buffer was added to the staining-specific tray, and each array was loaded therein one by one, followed by staining at room temperature for 10 minutes.
   8. The array was washed for 5 minutes in Wash E1BC solution and then dried by centrifugation.

### (V) Scanning and quantification

1. The array was loaded in a specific scanner manufactured by Illumina, Inc. and scanned in a standard mode.
2. After the completion of scanning, each spot on the microarray was quantified using specific software BeadStudio.

The obtained microarray data was normalized using VST (variance stabilizing transformation) and RSN (robust spline normalization). A gene expression level with presence probability < 0.05 with respect to a negative control (gene expression level measured using a probe for a gene that was absent on the microarray) was determined to be significant. Genes with presence probability < 0.05 in 70% or more of the 40 patients were used in the following experiments:

### 2: Selection of gene for use in prediction

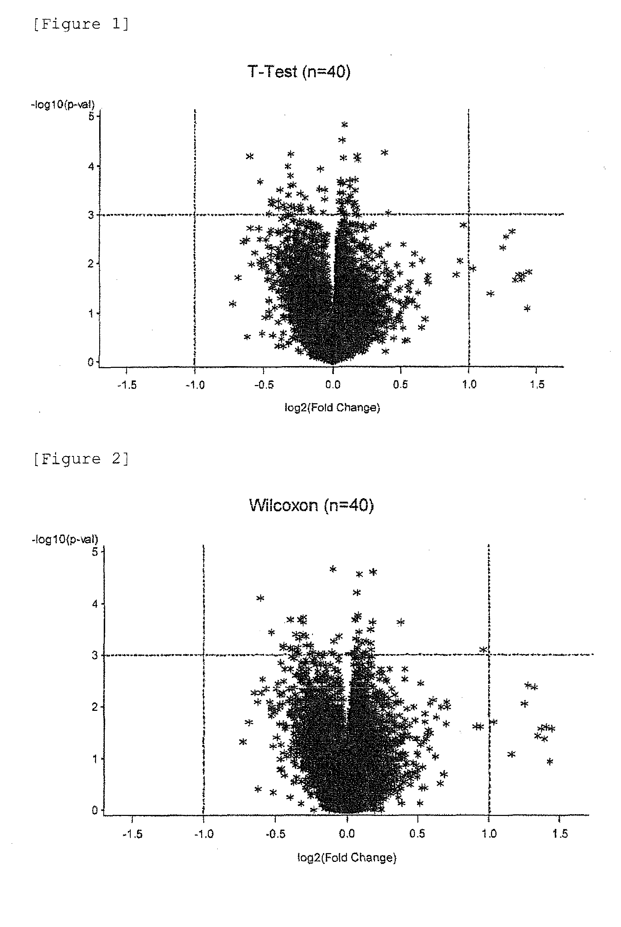

The prostate cancer patients of Example 1 were classified on the basis of a survival time after peptide vaccine therapy into a good prognosis group (long-lived group) with a survival time of 480 days or longer and a poor prognosis group (short-lived group) with a survival time shorter than 480 days. Genes that could significantly differentiate between two groups were selected. Analysis was conducted using t-test and Wilcoxon test.
Specifically, 16968 genes were subjected to t-test and Wilcoxon test in the short-lived group (S) and the long-lived group (L) (Figures 1 and 2). As a result, 54 genes with a level of significance "p <= 0.001" or "fold change of 2 or more" demonstrated by the t-test and the Wilcoxon test were extracted (Table 1). The expression levels (fluorescence reader-measured values) of these 54 genes are shown in the columns "Mean of short-lived group (S)" and "Mean of long-lived group (L)" of Table 1.

**[Table 1]**

| **OBS** | **Probe_ID** | **Symbol** | **Accession** | **Mean of short-lived group (S)** | **Mean of long-lived group (L)** | **S-L** | **Pr > \|t\|** | **P-value, Kruskal-Waills Test** |
|---|---|---|---|---|---|---|---|---|
| **2** | 150706 | UGP2 | NM_006759.3 | 7.9670 | 8.3141 | -0.3471 | 0.0008 | 0.00088 |
| **4** | 510208 | LOC643310 | XM_926856.1 | 9.2936 | 9.6892 | -0.3958 | 0.0005 | 0.00021 |
| **7** | 770400 | LOC653600 | XM_928349.1 | 9.8511 | 8.6911 | 1.1600 | 0.0416 | 0.08342 |
| **8** | 840064 | LOC645489 | XM_928514.1 | 7.2404 | 7.4395 | -0.1990 | 0.0005 | 0.00097 |
| **11** | 840601 | MEGF10 | NM_032446.1 | 6.6521 | 6.6044 | 0.0477 | 0.0005 | 0.00097 |
| **12** | 870477 | LOC728358 | NM_001042500.1 | 11.6369 | 10.2926 | 1.3443 | 0.0219 | 0.03726 |
| **13** | 990315 | TCP1 | NM_030752.2 | 8.0893 | 8.3461 | -0.2568 | 0.0008 | 0.00065 |
| **16** | 1400240 | LDHB | NM_002300.4 | 10.8411 | 11.4466 | -0.6055 | <.0001 | 0.00008 |
| **17** | 1500047 | RIN1 | NM_004292.2 | 6.8923 | 8.7602 | 0.1322 | 0.0004 | 0.00088 |
| **18** | 1500735 | CTSG | NM_001911.2 | 8.7810 | 7.5263 | 1.2547 | 0.0048 | 0.00869 |
| **19** | 1570392 | IL21R | NM_181078.1 | 6.6201 | 6.5630 | 0.0571 | 0.0002 | 0.00088 |
| **23** | 1780709 | DDX17 | NM_030881.2 | 6.6347 | 9.0780 | -0.4433 | 0.0005 | 0.00072 |
| **24** | 1780719 | PTGES3 | NM_006601.4 | 8.3710 | 8.8997 | -0.5287 | 0.0002 | 0.00036 |
| **25** | 2030332 | PTPN18 | NM_014369.2 | 6.6984 | 6.6146 | 0.0838 | <.0001 | 0.00003 |
| **28** | 2340091 | LOC646135 | XM_933437.1 | 6.8649 | 6.7536 | 0.1113 | 0.0006 | 0.00097 |
| **29** | 2360672 | TNFRSF19 | NM 148957.2 | 6.6360 | 6.5650 | 0.0710 | 0.0006 | 0.00080 |
| **30** | 2370764 | G3BP2 | NM_012297.3 | 6.6111 | 6.5557 | 0.0553 | 0.0002 | 0.00021 |
| **31** | 2480600 | LOC728358 | MM_001042500.1 | 11.7328 | 10.2900 | 1.4428 | 0.0148 | 0.02655 |
| **33** | 2680440 | | BF338865 | 6.6258 | 6.5641 | 0.0617 | 0.0005 | 0.00048 |
| **35** | 2900255 | ZBT645 | NM_032792.2 | 6.8759 | 6.7543 | 0.1246 | 0.0003 | 0.00097 |
| **37** | 2970747 | DEFA3 | NM_005217.2 | 11.8854 | 10.4815 | 1.4039 | 0.0171 | 0.02476 |
| **38** | 3060288 | NAIP | NM_004536.2 | 6.6667 | 6.5931 | 0.0736 | <.0001 | 0.00017 |
| **39** | 3130296 | AMY2A | NM_000699.2 | 7.2523 | 7.5351 | -0.2828 | 0.0007 | 0.00065 |
| **40** | 3130370 | ZNF83 | NM_018300.2 | 7.4687 | 7.7744 | -0.3057 | 0.0002 | 0.00023 |
| **41** | 3130477 | C7orf28A | XM_001133729.1 | 7.7594 | 8.1429 | -0.3834 | 0.0003 | 0.00080 |
| **43** | 3360228 | RPS20 | NM_001023.2 | 14.5907 | 14.7537 | -0.1630 | 0.0007 | 0.00097 |
| **44** | 3390368 | POP2 | NM_020786.1 | 6.6320 | 6.5632 | 0.0688 | <.0001 | 0.00006 |
| **45** | 3420136 | C6orf222 | NM_001010903.3 | 6.5946 | 6.5441 | 0.0505 | 0.0004 | 0.00097 |
| **46** | 3440189 | ZBTB20 | NM_015642.3 | 7.5534 | 7.8405 | -0.2871 | 0.0002 | 0.00044 |
| **48** | 3610521 | PCDHGB6 | NM_018928.2 | 6.8835 | 6.7067 | 0.1768 | <.0001 | 0.00023 |
| **51** | 3990608 | MAN2A1 | NM_002372.2 | 8.5259 | 8.8533 | -0.3274 | 0.0001 | 0-00021 |
| **52** | 4010632 | LOC642946 | XM_927142.1 | 6.6135 | 6.5408 | 0.0727 | 0.0002 | 0.00019 |
| **53** | 4120056 | IQSEC2 | NM_015075.1 | 5.6354 | 6.5576 | 0.0777 | 0.0002 | 0.00054 |
| **55** | 4150408 | P2RY2 | NM_002564.2 | 6.8606 | 6.5785 | 0.1821 | <.0001 | 0.00002 |
| **58** | 4220731 | P4HA1 | NM_000917.2 | 7.8835 | 8.1913 | -0.3078 | 0.0003 | 0.00039 |
| **59** | 4250154 | LOC648749 | XM_937834.2 | 6.5599 | 6.6215 | -0.0617 | 0.0003 | 0.00044 |
| **60** | 4260767 | GP1BA | NM_000173.4 | 7.0478 | 6.9170 | 0.1309 | 0.0004 | 0.00064 |
| **62** | 4540239 | DEFA1 | NM_004084.2 | 12.6997 | 11.3300 | 1.3597 | 0.0171 | 0.02655 |
| **65** | 4810072 | TUSC2 | NM_007275.1 | 7.3719 | 7.2093 | 0.1626 | 0.0002 | 0.00032 |
| **66** | 4830255 | DPP4 | NM_001935.3 | 6.8716 | 7.1790 | -0.3074 | <.0001 | 0.00019 |
| **68** | 5080692 | HLA-A29.1 | NM_001080840.1 | 11.3133 | 9.8801 | 1.4332 | 0.0823 | 0.11667 |
| **69** | 6290358 | CPT1A | NM_001031847.1 | 6.9679 | 6.8024 | 0.1656 | 0.0003 | 0.00059 |
| **70** | 5550711 | SYNE1 | NM_182961.2 | 6.6852 | 6.6097 | 0.0755 | 0.0004 | 0.00072 |
| **71** | 5860075 | CAMP | NM_004345.3 | 9.9646 | 8.9316 | 1.0328 | 0.0129 | 0.02000 |
| **72** | 6860465 | USPSY | NM_004654.3 | 6.6293 | 6.7238 | -0.0945 | 0.0001 | 0.00054 |
| **73** | 6900129 | CROP | NM_006107.2 | 7.9041 | 8.3479 | -0.4438 | 0.0006 | 0.00066 |
| **74** | 5960072 | | BY797688 | 6.6802 | 6.7772 | -0.0970 | 0.0003 | 0.00002 |
| **75** | 6110630 | HIST1H2BK | NM_080593.1 | 10.7582 | 10.3790 | 0.3791 | <.0001 | 0.00023 |
| **78** | 6420446 | CMPK1 | NM_016308.1 | 8.2399 | 8.6386 | -0.3986 | 0.0007 | 0.00072 |
| **79** | 6550154 | DEFA4 | NM_001925.1 | 8.6054 | 7.5272 | 1.2782 | 0.0029 | 0.00380 |
| **83** | 6590484 | NAP1L1 | NM_139207.1 | 7.5255 | 7.8829 | -0.3574 | 0.0006 | 0.00039 |
| **88** | 6940433 | STAT5B | NM_012448.3 | 8.5812 | 8.8213 | -0.2402 | 0.0007 | 0.00088 |
| **88** | 7150170 | LOC728358 | NM_001042500.1 | 11.6943 | 10.3009 | 1.3933 | 0.0209 | 0.04248 |
| **93** | 7650497 | ELA2 | NM_001972.2 | 8.9421 | 7.6222 | 1.3199 | 0.0022 | 0.00414 |

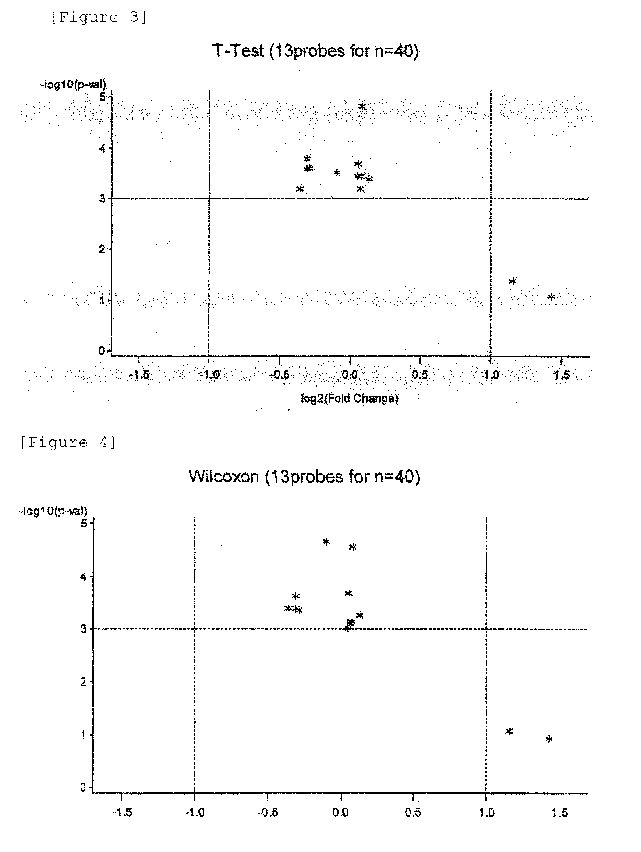

The selected 54 gene was further subjected to variable selection using t-test and Wilcoxon test (Figures 3 and 4) to select 13 genes (Table 2).

**[Table 2]**

| **Probe_ID** | **Symbol** | **Accession** | **Pr > \|t\|** | **P-value, Kruskal-WallisTest** |
|---|---|---|---|---|
| 770400 | LOC653600 | XM_928349.1 | 0.0416 | 0.08342 |
| 2030332 | PTPN18 | NM 014369.2 | <.0001 | 0.00003 |
| 2360672 | TNF RSF 19 | NM 148957.2 | 0.0006 | 0.00080 |
| 2370754 | G3BP2 | NM 012297.3 | 0.0002 | 0.00021 |
| 3130370 | ZNF83 | NM_018300.2 | 0.0002 | 0.00023 |
| 3420136 | C6orf222 | NM_001010903.3 | 0.0004 | 0.00097 |
| 3440189 | ZBTB20 | NM_015642.3 | 0.0002 | 0.00044 |
| 4220731 | P4HA1 | NM 000917.2 | 0.0003 | 0.00039 |
| 4260767 | GP1BA | NM 000173.4 | 0.0004 | 0.00054 |
| 5080692 | HLA-A29.1 | NM 001080840.1 | 0.0823 | 0.11667 |
| 5550711 | SYNE1 | NM 182961.2 | 0.0004 | 0.00072 |
| 5960072 | | BY797688 | 0.0003 | 0.00002 |
| 6590484 | NAP1L1 | NM_139207.1 | 0.0006 | 0.00039 |

### 3: Discrimination rate based on selected gene

### 3-1. Calculation of discrimination rate

The discrimination rate of the short-lived group or the long-lived group based on the selected genes was calculated. Specifically, the data set was divided into training data and test data, and subjected to cross-validation for performing model construction and tests. For the cross-validation method, leave-one-out cross-validation was used, where training for the data set except for the data of one individual is performed and a discriminant model is evaluated using this one individual that has not been used in the training data; and the above-described task is repeated for all individuals.

Results of analysis using 4 genes: probe ID Nos. 770400, 3130370, 4220731 and 5550711 shown in Table 3 are shown as an example. The analysis was conducted using statistical analysis software SAS (SAS Institute Japan Ltd.) and statistical analysis software JMP (SAS Institute Japan Ltd.).

**[Table 3]**

| **Linear discriminant function for grp** | | |
|---|---|---|
| **Variable** | **0** | **1** |
| **Constant** | -7124 | -7038 |
| **770400** | 10.47056 | 9.49131 |
| **3130370** | 148.16314 | 154.23258 |
| **4220731** | 62.46423 | 69.11172 |
| **5550711** | 1877 | 1850 |

Table 4 shows results of conducting leave-one-out cross validation with respect to the training data of 40 patients. Specifically, this table shows which group each case is predicted to belong to using the discriminant functions of Table 3. In the table, (Actual) represents a group to which each case actually belonged, and (Prediction) represents a group predicted using the discriminant functions. As is evident from the table, S2_pre was correctly predicted [0 (short-lived group) → 0 (short-lived group)], whereas S10_pre was incorrectly predicted [0 (short-lived group) → 1 (long-lived group)]. In the table, the symbol * represents that the case was predicted to belong to a group which is different from the actual one. A total of 4 individuals corresponded thereto.

**[Table 4]**

| Results of data-discrimination by means of decision rule: WORK MOTHER | | | | | |
|---|---|---|---|---|---|
| Results of cross-validation (linear discriminant function) | | | | | |
| **Posterior probability for grp** | | | | | |
| **Case Number** | **Group (Actual): grp** | **Group (Prediction): grp** | | **0** | **1** |
| **S2_pre** | **0** | **0** | | 0.9616 | 0.0384 |
| **S3_pre** | **0** | **0** | | 0.9982 | 0.0018 |
| **S4 pre** | **0** | **0** | | 0.8032 | 0.1968 |
| **S6_pre** | **0** | **0** | | 0.9994 | 0.0006 |
| **S7_pre** | **0** | **0** | | 0.9778 | 0.0222 |
| **S8_pre** | **0** | **0** | | 0.7907 | 0.2093 |
| **L3 pre** | **1** | **1** | | 0.2898 | 0.7001 |
| **L4_pre** | **1** | **1** | | 0.1652 | 0.8348 |
| **L1_pre** | **1** | **1** | | 0.3087 | 0.8913 |
| **S10_pre** | **0** | **1** | * | 0.1158 | 0.8842 |
| **S12_pre** | **0** | **0** | | 0.9168 | 0.0832 |
| **S13_pre** | **0** | **0** | | 0.9988 | 0.0011 |
| **S14_pre** | **0** | **0** | | 0.9609 | 0.0391 |
| **S15_pre** | **0** | **0** | | 0.9393 | 0.0607 |
| **S16_pre** | **0** | **0** | | 0.9869 | 0.0131 |
| **S17_pre** | **0** | **0** | | 0.9904 | 0.0096 |
| **S16_pre** | **0** | **0** | | 0.9996 | 0.0004 |
| **S20_pre** | **0** | **0** | | 0.9999 | 0.0001 |
| **S21_pre** | **0** | 1 | * | 0.4247 | 0.5753 |
| **S22_pre** | **0** | 0 | | 0.9901 | 0.0099 |
| **S23_pre** | **0** | **0** | | 0.9261 | 0.0739 |
| **S25_pre** | **0** | **0** | | 0.6318 | 0.3682 |
| **S26_pre** | **0** | **1** | * | 0.021 | 0.979 |
| **L5_pre** | **1** | **1** | | 0.0716 | 0.9284 |
| **L6_pre** | **1** | **1** | | 0.0336 | 0.9664 |
| **L7_pre** | **1** | **1** | | 0.0144 | 0.9856 |
| **L8_pre** | **1** | **1** | | 0.0152 | 0.9848 |
| **L9_pre** | **1** | **0** | * | 0.9708 | 0.0292 |
| **L10_pre** | **1** | **1** | | 0.0159 | 0.9841 |
| **L11_pre** | **1** | **1** | | 0.3711 | 0.6288 |
| **L12 pre** | **1** | **1** | | 0.0251 | 0.9749 |
| **L13_pre** | **1** | **1** | | 0.0019 | 0.9981 |
| **L14_pre** | **1** | **1** | | 0.006 | 0.995 |
| **L15_pre** | **1** | **1** | | 0.0007 | 0.9993 |
| **L16_pre** | **1** | **1** | | 0.178 | 0.822 |
| **L17_pre** | **1** | **1** | | 0.0109 | 0.9891 |
| **L18-pre** | **1** | **1** | | 0.0308 | 0.8692 |
| **L20_pre** | **1** | **1** | | 0.1248 | 0.8752 |
| **L21_pre** | **1** | **1** | | 0.0008 | 0.8992 |
| **L19_pre** | **1** | **1** | | 0.001 | 0.999 |

Table 5 summarizes the results of Table 4 in a 2 x 2 cross table.
As is evident therefrom, 17 cases of the short-lived group were correctly discriminated with [0 → 0] (17/20 = 0.85: 85%), whereas 19 cases of the long-lived group were correctly discriminated with [1 → 1] (19/20 = 0.95: 95%).
These are discrimination rates.

**[Table 5]**

| Results of data-discrimination by means of decision rule: WORK MOTHER | | | |
|---|---|---|---|
| Abstracts of cross-validation (linear discriminant function) | | | |
| **Number of observation of grp-discrimination and discrimination rate** | | | |
| **Group (Actual): grp** | **0** | **1** | **Total** |
| **0** | 17 | 3 | 20 |
| | 85 | 15 | 100 |
| **1** | 1 | 18 | 20 |
| | 5 | 95 | 100 |
| **Total** | 18 | 22 | 40 |
| | 45 | 55 | 100 |
| **Priors** | 0.5 | 0.5 | |

Table 6 shows results of conducting discriminant analysis using the discriminant functions of Table 3 with respect to the test data of 11 patients. Like Table 4, Table 6 shows the results of predicting which group each case belongs to. Only one case (4818441059_E) with the symbol * was incorrectly discriminated with [1 (long-lived group) → 0 (short-lived group)].

**[Table 6]**

| Results of discrimination of test data: WORK.TEST3 | | | | | |
|---|---|---|---|---|---|
| Results of discrimination (linear discriminant function) | | | | | |
| **Posterior probability for grp** | | | | | |
| **Case Number** | **Group (Actual): grp** | **Group (Prediction): grp** | | **0** | **1** |
| **48184411050_A** | **1** | **1** | | 0 | 1 |
| **4818441050_B** | **1** | **1** | | 0.0209 | 0.9791 |
| **48184411050_C** | **0** | **0** | | 0.8852 | 0.1048 |
| **488441050_D** | **1** | **1** | | 0.34 | 0.66 |
| **4818441050_E** | **0** | **0** | | 0.5882 | 0.4118 |
| **4818441050_F** | **0** | **0** | | 0.9995 | 0.0005 |
| **4818441052_A** | **0** | **0** | | 0.797 | 0.203 |
| **4818441059_C** | **0** | **0** | | 0.6135 | 0.3865 |
| **4818441059_D** | **0** | **0** | | 0.9108 | 0.0692 |
| **4818441059_E** | **1** | **0** | * | 0.9728 | 0.0272 |
| **4818441059_F** | **1** | **1** | | 0.2318 | 0.7684 |

Table 7 summarizes the results of Table 6 in a 2 x 2 cross table.
As is evident therefrom,
6 cases of the short-lived group were correctly discriminated with [0 (short-lived group) → 0 (short-lived group)] (6/6 = 1.00: 100%), whereas 4 cases of the long-lived group were correctly discriminated with [1 (long-lived group) → 1 (long-lived group)] (4/5 = 0.80: 80%).

**[Table 7]**

| Results of discrimination of test data: WORK.TEST3 | | | |
|---|---|---|---|
| Abstracts of discrimination (linear discriminant function) | | | |
| **Number of observation of grp-discrimination and discrimination rate** | | | |
| **Group (Actual):grp** | **0** | **1** | **Total** |
| **0** | 6 | 0 | 6 |
| | 100 | 0 | 100 |
| **1** | 1 | 4 | 5 |
| | 20 | 80 | 100 |
| **Total** | 7 | 4 | 11 |
| | 63.64 | 36.35 | 100 |
| **Priors** | 05 | 05 | |

These results demonstrated that the group to which each case belongs could be discriminated (predicted) with 80% or more accuracy for both the training data and the test data by discriminant analysis using the 4 genes, i.e., probe ID NOs. 770400, 3130370, 4220731 and 5550711.

### 3-2. Discrimination rate based on selected gene

Subsequently, the discrimination rate was calculated using combinations of the 13 genes selected in the above-described item 2.

### (1) Discrimination rate based on 1 gene

A study was made on whether or not long life or short life could be discriminated (discrimination rate: 80% or more) on the basis of one out of the 13 genes using training data (40 individuals) and test data (11 individuals).
As shown in Table 8, one gene 5960072 (BY797688) out of the 13 genes permitted prediction with a discrimination rate of 80% or more in training data (40 individuals) and test data (11 individuals) as to the discrimination of long life. Prediction based on this gene resulted in a short life discrimination rate (ccvP0) of 85% using the training data (40 individuals) and a short life discrimination rate (ctcP0) of 67% using the test data (11 individuals). On the other hand, the prediction resulted in a long life discrimination rate (ccvP1) of 85% using the training data and a long life discrimination rate (ctcP1) of 80% using the test data.

**[Table 8]**

| OBS | rep | index | pbnum | pb1 | ccvP0 | ctcP0 | ccvP1 | ctcP1 | flg0 | flg1 |
|---|---|---|---|---|---|---|---|---|---|---|
| 1 | 13 | 12 | 1 | 5960072 | 85 | 66.67 | 85 | 80 | 0 | 1 |
| 2 | 13 | 4 | 1 | 2370754 | 65 | 83.33 | 90 | 20 | 0 | 0 |
| 3 | 13 | 2 | 1 | 2030332 | 75 | 50 | 75 | 60 | 0 | 0 |
| 4 | 13 | 3 | 1 | 2360672 | 60 | 33. 33 | 75 | 60 | 0 | 0 |
| 5 | 13 | 9 | 1 | 4260767 | 70 | 100 | 75 | 20 | 0 | 0 |
| 6 | 13 | 13 | 1 | 6590484 | 85 | 0 | 70 | 80 | 0 | 0 |
| 7 | 13 | 7 | 1 | 3440189 | 70 | 66.67 | 70 | 60 | 0 | 0 |
| 8 | 13 | 8 | 1 | 4220731 | 80 | 66.67 | 70 | 40 | 0 | 0 |
| 9 | 13 | 5 | 1 | 3130370 | 75 | 83.33 | 65 | 60 | 0 | 0 |
| 10 | 13 | 6 | 1 | 3420136 | 65 | 83. 33 | 65 | 40 | 0 | 0 |
| 11 | 13 | 11 | 1 | 5550711 | 80 | 66.67 | 65 | 20 | 0 | 0 |
| 12 | 13 | 1 | 1 | 7.70400 | 45 | 16.67 | 60 | 100 | 0 | 0 |
| 13 | 13 | 10 | 1 | 5080692 | 85 | 33. 33 | 45 | 60 | 0 | 0 |

| | | | | | | | | | | |
|---|---|---|---|---|---|---|---|---|---|---|
| Pbnum: The number of probe (gene) Pb1: Probe ID NO. ccvP0: 0 (short life) → 0 (short life) discrimination rate obtained using training data (40 individuals) ctcP0: 0 (short life) → 0 (short life) discrimination rate obtained using test data (11 individuals) ccvP1: 0 (long life) → 0 (long life) discrimination rate obtained using training data ctcP1: 0 (long life) → 0 (long life) discrimination rate obtained using test data flg0: The short life discrimination rate was 80% or more for both training data and test data flg1: The long life discrimination rate was 80% or more for both training data and test data | | | | | | | | | | |

### (2) Discrimination rate based on 2 genes

The number of combinations of 2 genes from the 13 genes is 78, and some of them are shown in Table 9. For example, the sets of 2 gene probes that permitted prediction with a discrimination rate of 80% or more in training data (40 individuals) and test data (11 individuals) as to the discrimination of long life were the following 6 sets (Table 9: 1 to 6) (indicated by probe 1 / probe 2 [short life discrimination rate (ccvP0) obtained using training data (40 individuals) / short life discrimination rate (ctcP0) obtained using test data (11 individuals); long life discrimination rate (ccvP1) obtained using training data / long life discrimination rate (ctcP1) obtained using test data]: 2360672/3130370 (85/66.67; 85/80), 770400/2360672 (70/33.33; 80/80), 770400/5960072 (90/66.67; 80/80), 2360672/5080692 (65/50; 80/80), 2360672/5960072 (95/66.67; 80/80) and 2360672/6590484 (75/33.33; 80/80). On the other hand, the sets of 2 gene probes that permitted prediction with a discrimination rate of 80% or more as to the discrimination of short life were the following 15 sets (Table 9: 7 to 21) (probe 1 / probe 2 [short life discrimination rate (ccvP0) obtained using training data (40 individuals) / short life discrimination rate (ctcP0) obtained using test data (11 individuals); long life discrimination rate (ccvP1) obtained using training data / long life discrimination rate (ctcP1) obtained using test data]: 2370754/5960072 (80/83.33; 95/40), 3440189/5550711 (85/83.33; 90/60), 3420136/5960072 (90/83.33; 90/40), 2370754/4260767 (80/100; 90/0), 2030332/5550711 (90/83.33; 85/60), 3420136/3440189 (85/83.33; 85/60), 2370754/3130370 (80/83.33; 85/40), 2360672/4220731 (80/83.33; 80/60), 3440189/4220731 (8583.33/; 80/60), 4220731/4260767 (85/100; 80/20), 4220731/5550711 (85/83.33; 75/60), 3420136/4220731 (95/83.33; 75/40), 2370754/4220731 (95/83.33; 75/20), 2370754/5550711 (85/83.33; 7520/) and 4260767/6590484 (80/100; 75/20).

**[Table 9]**

| **OBS** | **rep** | **index** | **pbnum** | **pb1** | **pb2** | **ccvP0** | **ctcP0** | **ccvP1** | **ctcP1** | **flg0** | **flg1** |
|---|---|---|---|---|---|---|---|---|---|---|---|
| **1** | 78 | 25 | 2 | 2360672 | 3130370 | 85 | 66.67 | 85 | 80 | 0 | 1 |
| **2** | 78 | 2 | 2 | 770400 | 2360672 | 70 | 33.33 | 80 | 80 | 0 | 1 |
| **3** | 78 | 11 | 2 | 770400 | 5960072 | 90 | 66.67 | 80 | 80 | 0 | 1 |
| **4** | 78 | 30 | 2 | 2360672 | 5080692 | 65 | 50 | 80 | 80 | 0 | 1 |
| **5** | 78 | 32 | 2 | 2360672 | 5960072 | 95 | 66.67 | 80 | 80 | 0 | 1 |
| **6** | 78 | 33 | 2 | 2360672 | 6590484 | 75 | 33.33 | 80 | 80 | 0 | 1 |
| **7** | 78 | 41 | 2 | 2370754 | 5960072 | 80 | 83.33 | 95 | 40 | 1 | 0 |
| **8** | 78 | 61 | 2 | 3440189 | 5550711 | 85 | 83.33 | 90 | 60 | 1 | 0 |
| **9** | 78 | 56 | 2 | 3420136 | 5960072 | 90 | 83.33 | 90 | 40 | 1 | 0 |
| **10** | 78 | 38 | 2 | 2370754 | 4260767 | 80 | 100 | 90 | 0 | 1 | 0 |
| **11** | 78 | 21 | 2 | 2030332 | 5550711 | 90 | 83.33 | 85 | 60 | 1 | 0 |
| **12** | 78 | 51 | 2 | 3420136 | 3440189 | 85 | 83.33 | 85 | 60 | 1 | 0 |
| **13** | 78 | 34 | 2 | 2370754 | 3130370 | 80 | 83.33 | 85 | 40 | 1 | 0 |
| **14** | 78 | 28 | 2 | 2360672 | 4220731 | 80 | 83.33 | 80 | 60 | 1 | 0 |
| **15** | 78 | 58 | 2 | 3440189 | 4220731 | 85 | 83.33 | 80 | 60 | 1 | 0 |
| **16** | 78 | 64 | 2 | 4220731 | 4260767 | 85 | 100 | 80 | 20 | 1 | 0 |
| **17** | 78 | 66 | 2 | 4220731 | 5550711 | 85 | 83.33 | 75 | 60 | 1 | 0 |
| **18** | 78 | 52 | 2 | 3420136 | 4220731 | 95 | 83.33 | 75 | 40 | 1 | 0 |
| **19** | 78 | 37 | 2 | 2370754 | 4220731 | 95 | 83.33 | 75 | 20 | 1 | 0 |
| **20** | 78 | 40 | 2 | 2370754 | 5550711 | 85 | 83.33 | 75 | 20 | 1 | 0 |
| **21** | 78 | 72 | 2 | 4260767 | 6590484 | 80 | 100 | 75 | 20 | 1 | 0 |
| **22** | 78 | 14 | 2 | 2030332 | 2370754 | 80 | 66.67 | 95 | 60 | 0 | 0 |
| **23** | 78 | 16 | 2 | 2030332 | 3420136 | 90 | 66.67 | 95 | 60 | 0 | 0 |
| **24** | 78 | 1 | 2 | 770400 | 2030332 | 85 | 50 | 90 | 60 | 0 | 0 |
| **25** | 78 | 19 | 2 | 2030332 | 4260767 | 75 | 83.33 | 90 | 40 | 0 | 0 |
| **26** | 78 | 7 | 2 | 770400 | 4220731 | 70 | 66.67 | 85 | 60 | 0 | 0 |
| **27** | 78 | 15 | 2 | 2030332 | 3130370 | 80 | 66.67 | 85 | 60 | 0 | 0 |
| **28** | 78 | 17 | 2 | 2030332 | 3440189 | 90 | 50 | 85 | 60 | 0 | 0 |
| **29** | 78 | 22 | 2 | 2030332 | 5960072 | 95 | 50 | 85 | 60 | 0 | 0 |
| **30** | 78 | 23 | 2 | 2030332 | 6590484 | 85 | 50 | 85 | 60 | 0 | 0 |
| **31** | 78 | 31 | 2 | 2360672 | 5550711 | 75 | 83.33 | 85 | 60 | 0 | 0 |
| **32** | 78 | 78 | 2 | 5960072 | 6590484 | 80 | 50 | 85 | 60 | 0 | 0 |
| **33** | 78 | 3 | 2 | 770400 | 2370754 | 55 | 66.67 | 85 | 40 | 0 | 0 |
| **34** | 78 | 13 | 2 | 2030332 | 2360672 | 80 | 50 | 85 | -40 | 0 | 0 |
| **35** | 78 | 18 | 2 | 2030332 | 4220731 | 80 | 66.67 | 85 | 40 | 0 | 0 |
| **36** | 78 | 24 | 2 | 2360672 | 2370754 | 70 | 66.67 | 85 | 40 | 0 | 0 |
| **37** | 78 | 67 | 2 | 4220731 | 5960072 | 75 | 83.33 | 85 | 40 | 0 | 0 |
| **38** | 78 | 76 | 2 | 5550711 | 5960072 | 80 | 66.67 | 85 | 40 | 0 | 0 |
| **39** | 78 | 10 | 2 | 770400 | 5550711 | 80 | 66.67 | 80 | 60 | 0 | 0 |
| **40** | 78 | 36 | 2 | 2370754 | 3440189 | 75 | 100 | 80 | 60 | 0 | 0 |
| **41** | 78 | 48 | 2 | 3130370 | 5550711 | 65 | 100 | 80 | 60 | 0 | 0 |
| **42** | 78. | 49 | 2 | 3130370 | 5960072 | 80 | 66.67 | 80 | 60 | 0 | 0 |
| **43** | 78 | 62 | 2 | 3440189 | 5960072 | 85 | 66.67 | 80 | 60 | 0 | 0 |
| **44** | 78 | 77 | 2 | 5550711 | 6590484 | 90 | 66.67 | 80 | 60 | 0 | 0 |
| **45** | 78 | 29 | 2 | 2360672 | 4260767 | 75 | 100 | 80 | 40 | 0 | 0 |
| **46** | 78 | 45 | 2 | 3130370 | 4220731 | 75 | 66.67 | 80 | 40 | 0 | 0 |
| **47** | 78 | 55 | 2 | 3420136 | 5550711 | 70 | 83.33 | 80 | 40 | 0 | 0 |
| **48** | 78 | 59 | 2 | 3440189 | 4260767 | 75 | 100 | 80 | 40 | 0 | 0 |
| **49** | 78 | 8 | 2 | 770400 | 4260767 | 70 | 100 | 80 | 20 | 0 | 0 |

### (3) Discrimination rate based on 3 genes

The number of combinations of 3 genes from the 13 genes is 286, and some of them are shown in Table 10. For example, the set of 3 gene probes that permitted prediction with a discrimination rate of 80% or more in training data (40 individuals) and test data (11 individuals) as to the discrimination of long life or short life was only one set: 2360672/3440189/4220731 (Table 10: 1). Prediction based on this gene set resulted in a short life discrimination rate (ccvP0) of 90% using the training data (40 individuals) and a short life discrimination rate (ctcP0) of 83.33% using the test data (11 individuals). On the other hand, the prediction resulted in a long life discrimination rate (ccvP1) of 90% using the training data and a long life discrimination rate (ctcP1) of 80% using the test data. Likewise, examples of the probe sets that permit prediction of long life with a discrimination rate of 80% or more include 16 sets (Table 10: 2 to 17). In addition, examples of the probe sets that permits prediction of short life with a discrimination rate of 90% or more include the following gene probe sets: 2030332/2370754/4260767, 2030332/2370754/3440189, 3440189/5080692/5550711, 2360672/4220731/5550711, 3440189/4220731/5550711, 2370754/3440189/5550711, 2370754/3440189/4220731, 2370754/4220731/4260767, 3420136/4220731/5550711, 3420136/4220731/4260767 and 3130370/3420136/4220731.

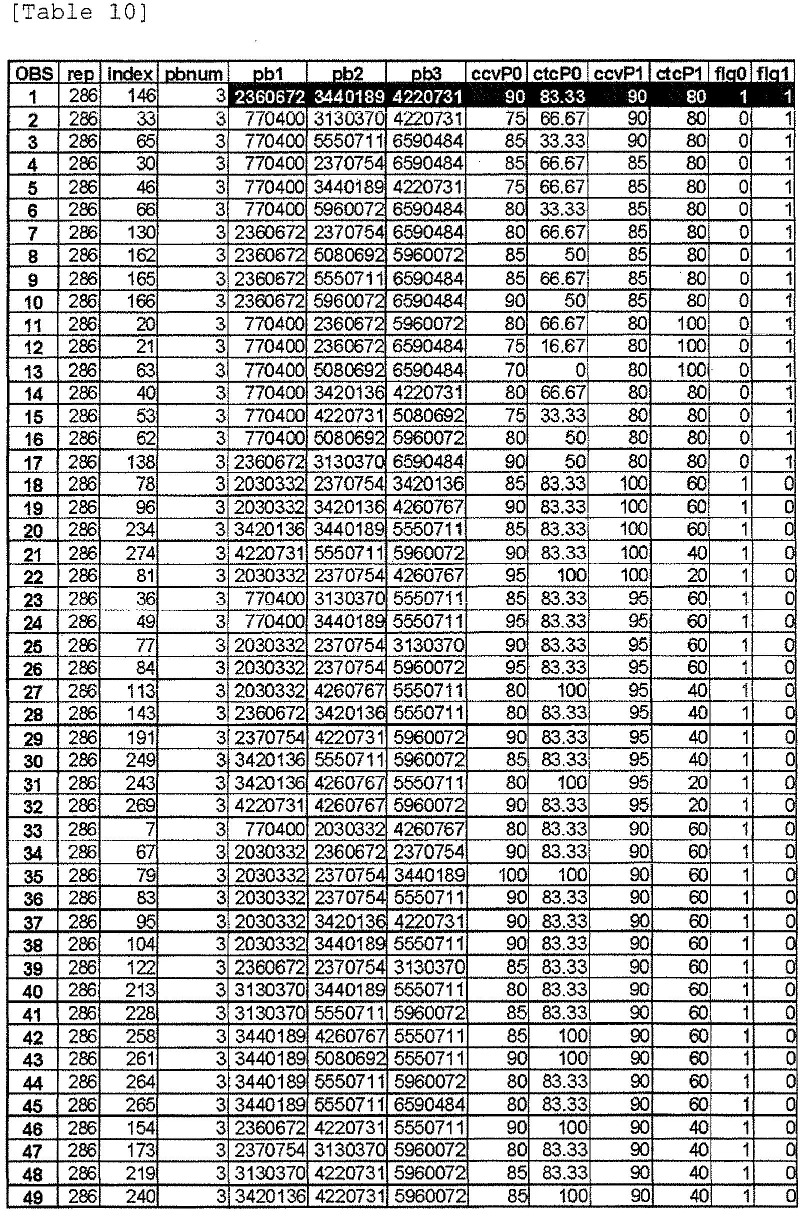

### (4) Discrimination rate based on 4 genes

The number of combinations of 4 genes from the 13 genes is 715, and some of them are shown in Table 11. For example, the sets of 4 gene probes that permit prediction with a discrimination rate of 80% or more in training data (40 individuals) and test data (11 individuals) as to the discrimination of long life or short life include 18 sets [Table 11: 1 to 18].

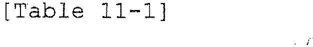

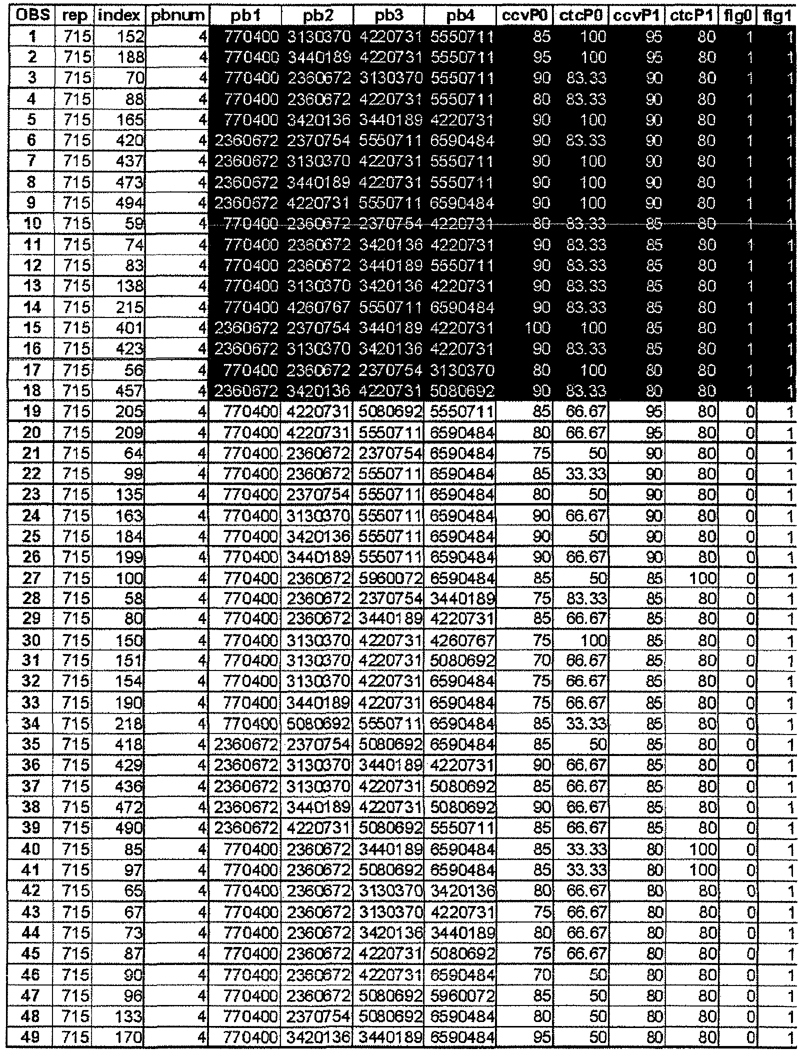

### (5) Discrimination rate based on 5 genes

The number of combinations of 5 genes from the 13 genes is 1287, and some of them are shown in Table 12. For example, the sets of 5 gene probes that permit prediction with a discrimination rate of 80% or more in training data (40 individuals) and test data (11 individuals) as to the discrimination of long life or short life include 55 sets [Table 12: 1 to 55].

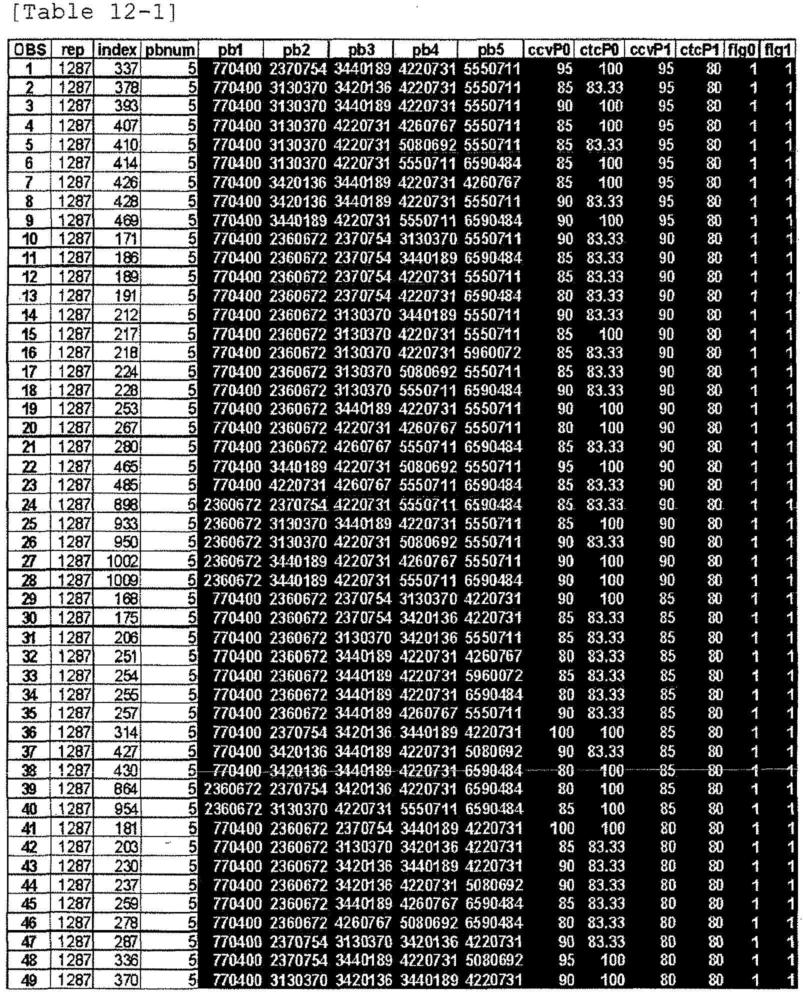

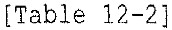

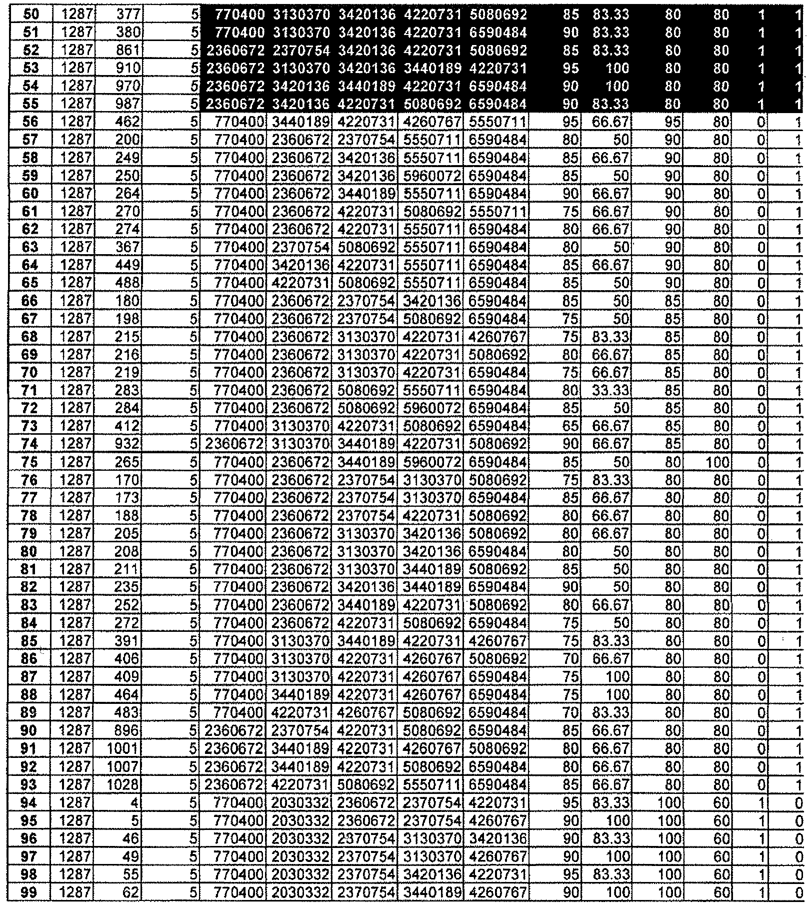

### (6) Discrimination rate based on 6 genes

The number of combinations of 6 genes from the 13 genes is 1716, and some of them are shown in Table 13. For example, the sets of 6 gene probes that permit prediction with a discrimination rate of 80% or more in training data (40 individuals) and test data (11 individuals) as to the discrimination of long life or short life include 71 sets [Table 13: 1 to 71].

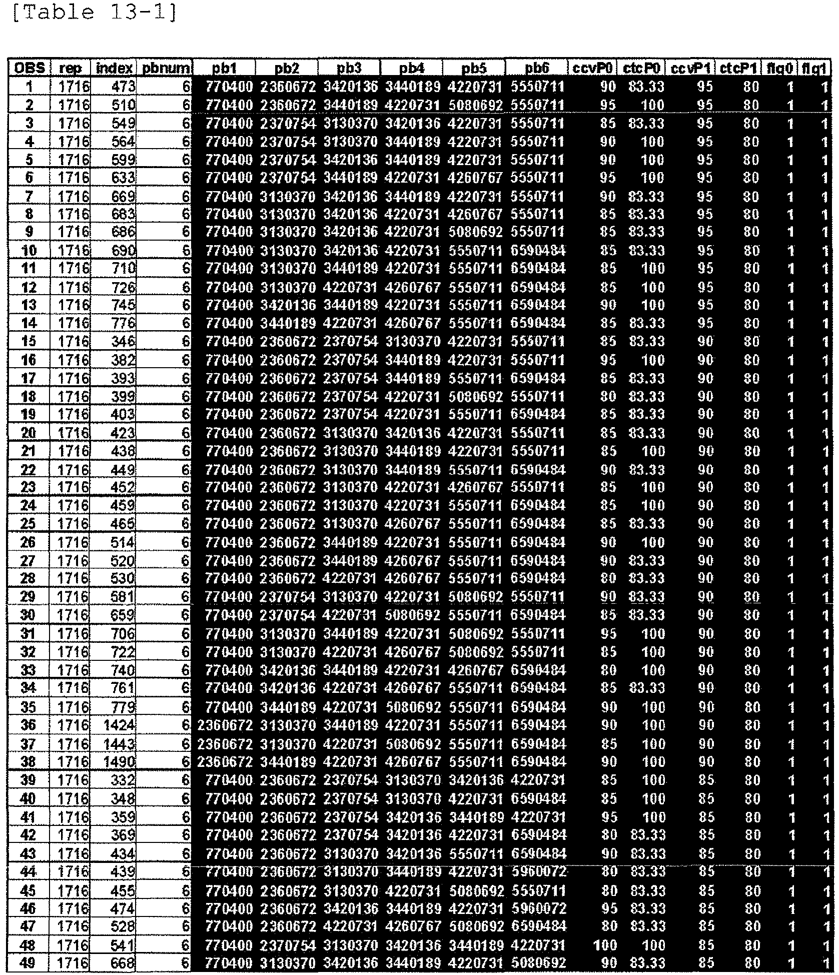

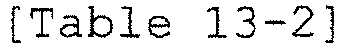

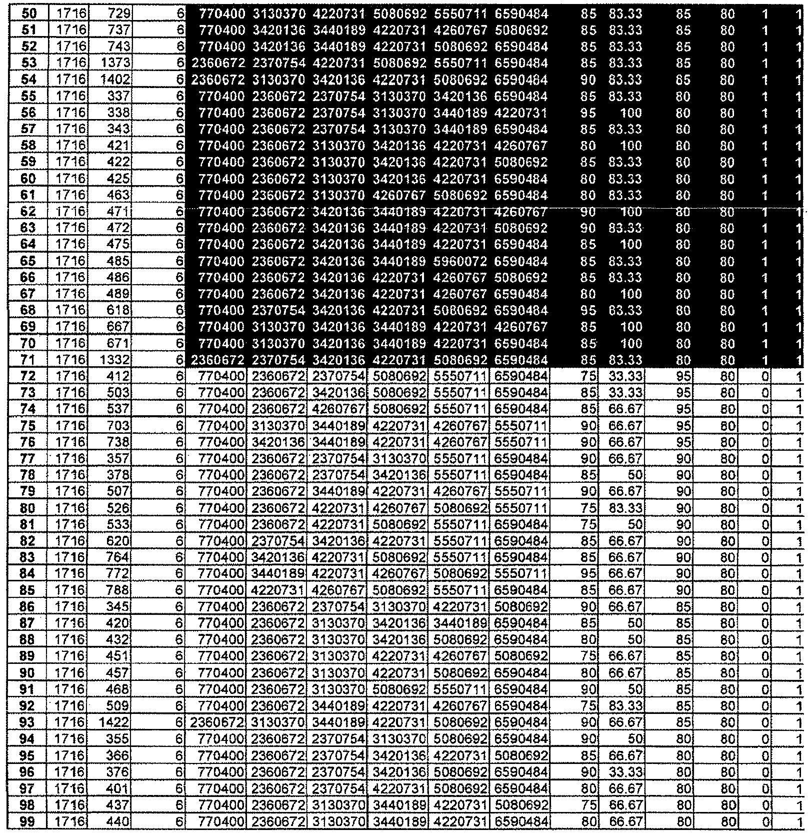

### (7) Discrimination rate based on 7 genes

The number of combinations of 7 genes from the 13 genes is 1715, and some of them are shown in Table 13. For example, the sets of 7 gene probes that permit prediction with a discrimination rate of 80% or more in training data (40 individuals) and test data (11 individuals) as to the discrimination of long life or short life are 63 sets [Table 14: 1 to 63].

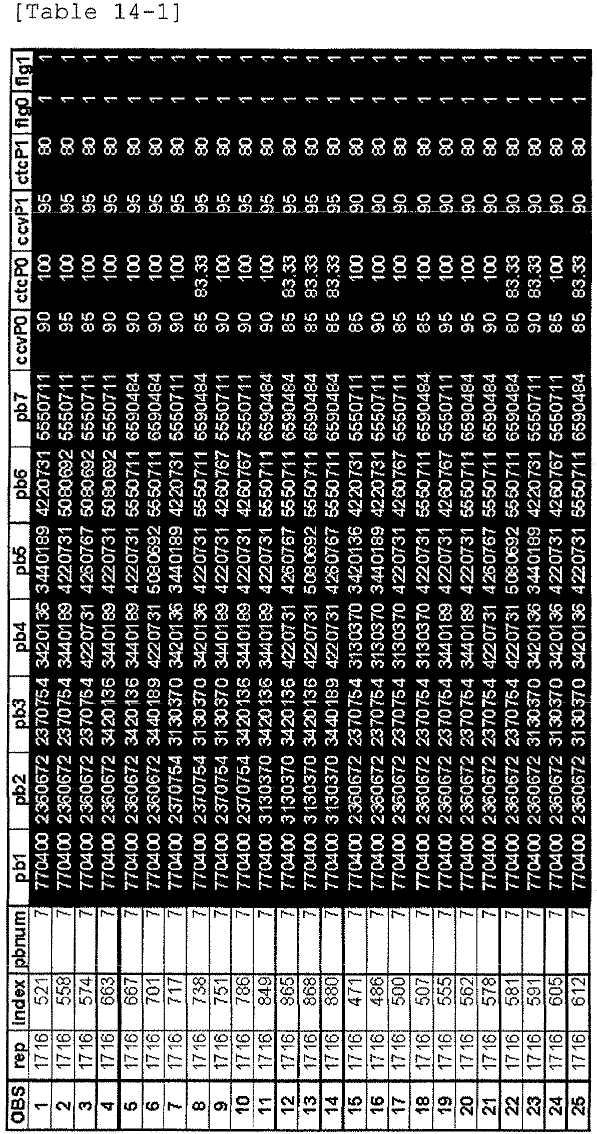

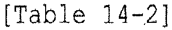

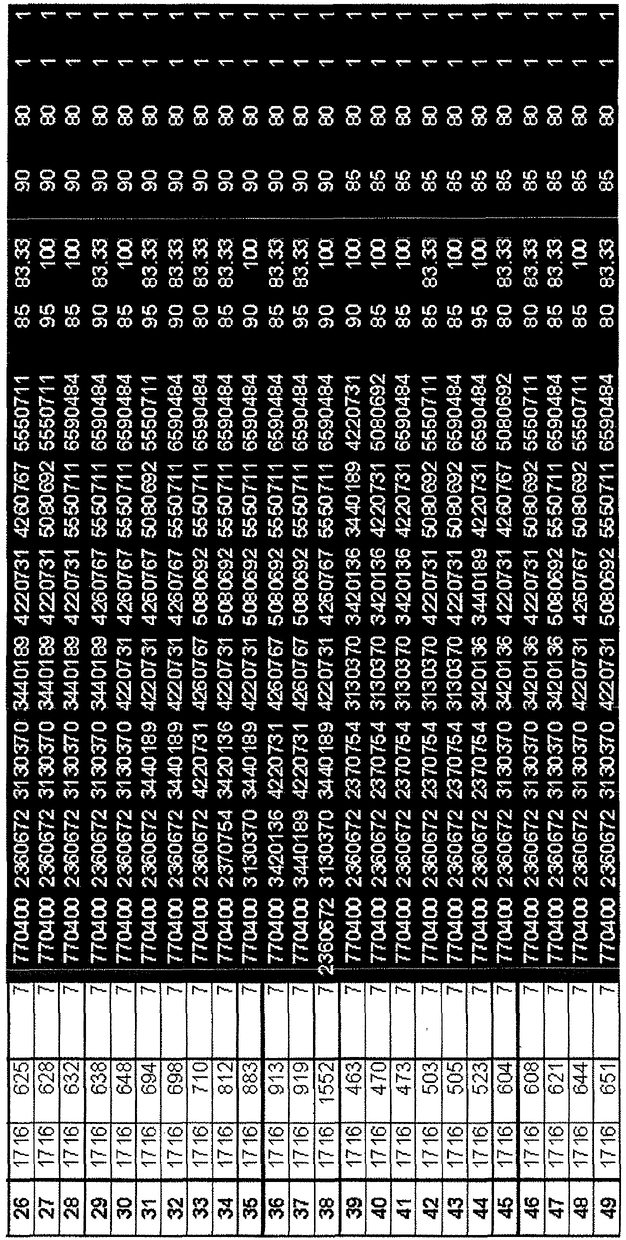

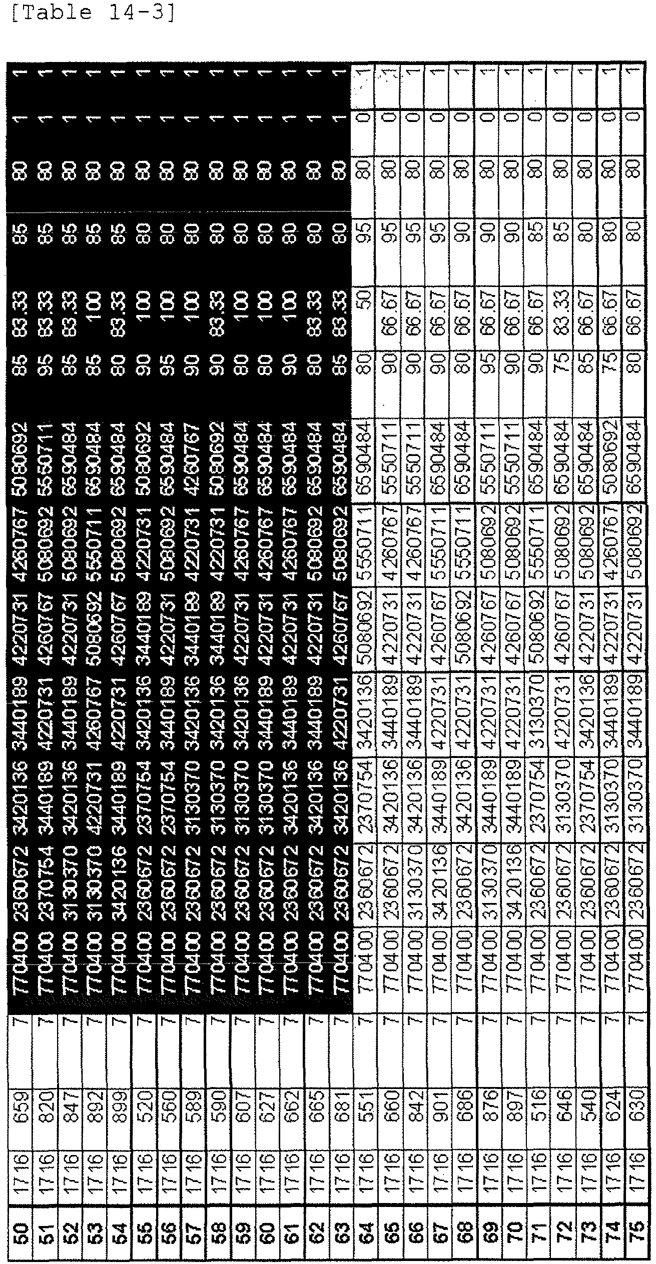

### (8) Discrimination rate based on 8 genes

The number of combinations of 8 genes from the 13 genes is 1287, and some of them are shown in Table 15. For example, the sets of 8 gene probes that permit prediction with a discrimination rate of 80% or more in training data (40 individuals) and test data (11 individuals) as to the discrimination of long life or short life include 45 sets [Table 15: 1 to 45].

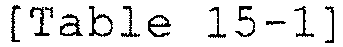

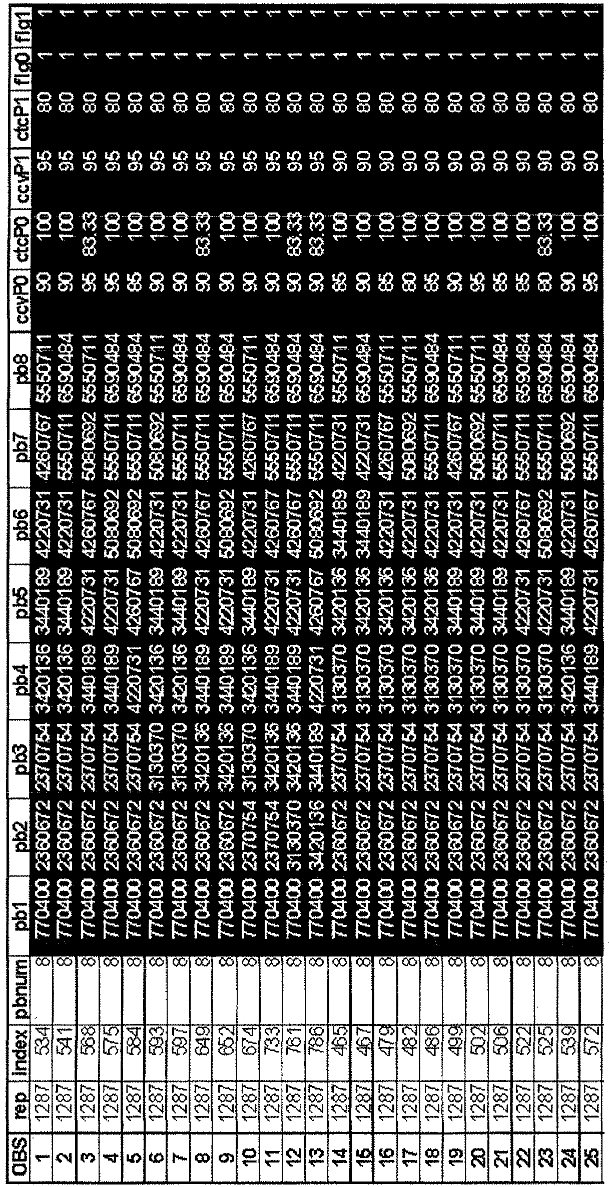

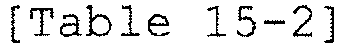

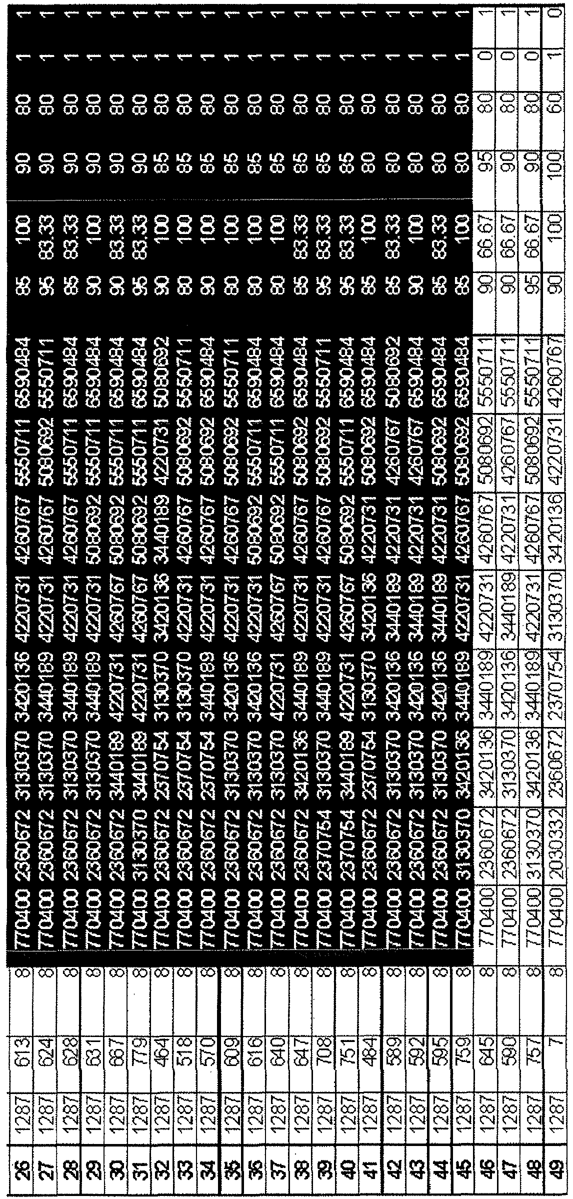

### (9) Discrimination rate based on 9 genes

The number of combinations of 9 genes from the 13 genes is 715, and some of them are shown in Table 16. For example, the sets of 9 gene probes that permit prediction with a discrimination rate of 80% or more in training data (40 individuals) and test data (11 individuals) as to the discrimination of long life or short life include 22 sets [Table 16: 1 to 22].

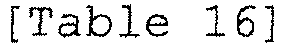

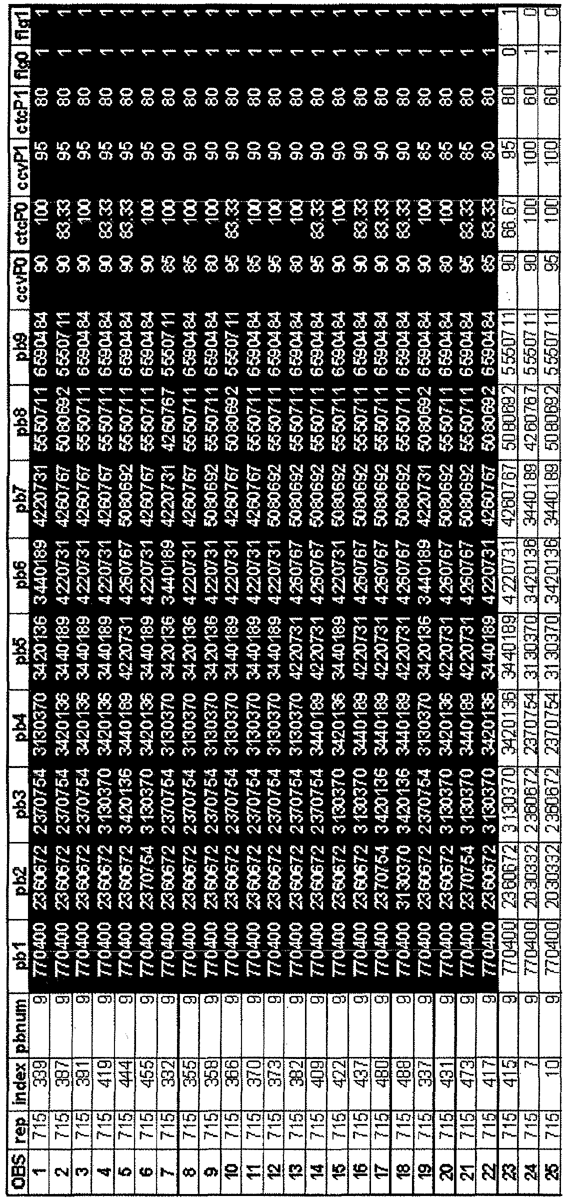

### (10) Discrimination rate based on 10 genes

The number of combinations of 10 genes from the 13 genes is 286, and some of them are shown in Table 17. For example, the sets of 10 gene probes that permit prediction with a discrimination rate of 80% or more in training data (40 individuals) and test data (11 individuals) as to the discrimination of long life or short life include 7 sets [Table 17: 1 to 7].

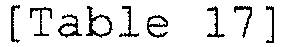

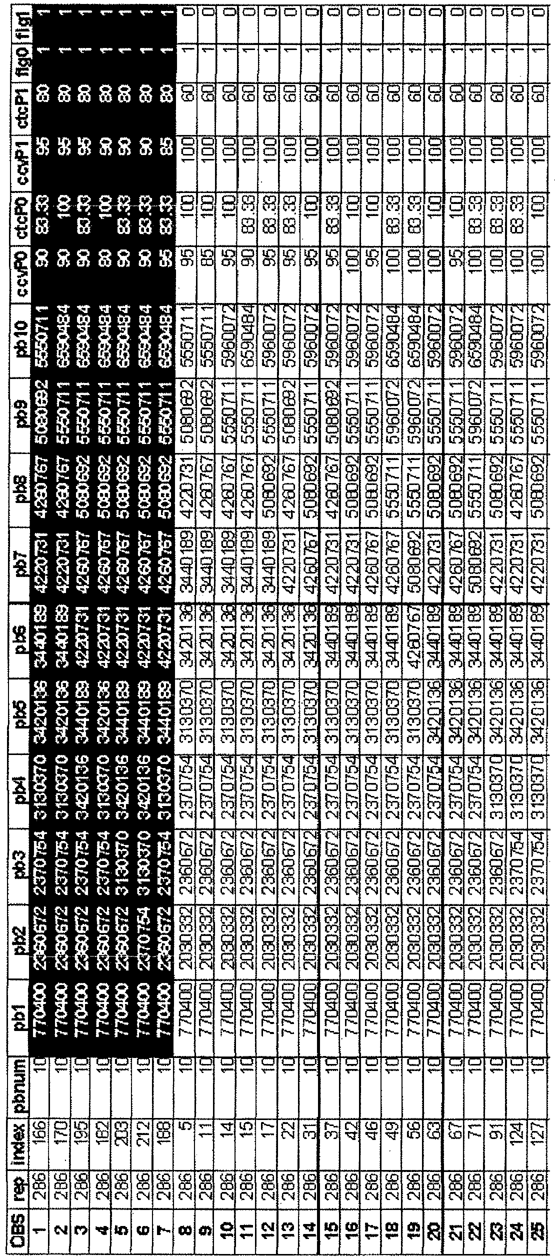

### (11) Discrimination rate based on 11 genes

The number of combinations of 11 genes from the 13 genes is 78. For example, the sets of 11 gene probes that permit prediction with a discrimination rate of 80% or more in training data (40 individuals) and test data (11 individuals) as to the discrimination of long life or short life include 1 set
[770400/2360672/2370754/3130370/3420136/3440189/4220731/4 260767/5080692/5550711/6590484.

A study was made on combinations of 12 or 13 genes selected from the 13 genes in the same way as above. No gene probe set was found to permit prediction with a discrimination rate of 80% or more in training data (40 individuals) and test data (11 individuals) as to the discrimination of long life or short life.

### (14) Selection of gene advantageous for discrimination

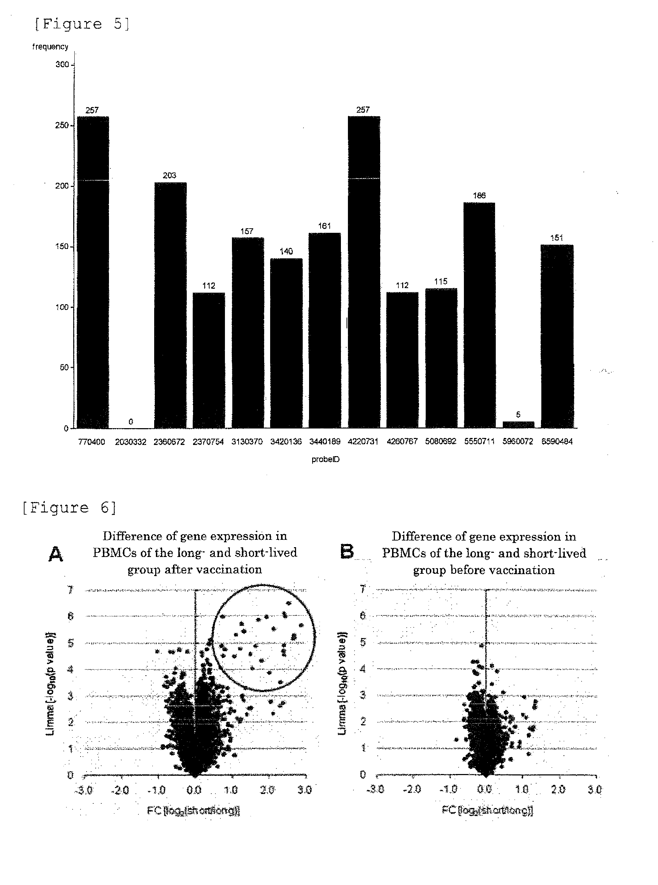

As described above, when 1, 2, ..., 13 genes are used from the 13 genes, 4 genes that offered a favorable discrimination rate (highly frequent genes used in the gene sets that resulted in a discrimination rate of 80% or more) were selected (probe ID NOs : 770400, 2360672, 4220731 and 5550711). Table 18 and Figure 5 show the appearance frequency of each probe obtained from the combinations of probes that result in both flg0 and flg1 of 1 (discrimination rate of 80% or more in both training data and test data). The combinations involving these 4 genes exhibited high prognostic predictability.

**[Table 18]**

| **OBS** | **Probe_ID** | **Symbol** | **Accession** | **frequency** |
|---|---|---|---|---|
| **1** | 770400 | LOC653600 | XM_928349.1 | 257 |
| **2** | 2030332 | PTPN18 | NM_014369.2 | 0 |
| **3** | 2360672 | TNFRSF19 | NM_148957.2 | 203 |
| **4** | 2370754 | G3BP2 | NM_012297.3 | 112 |
| **5** | 3130370 | ZNF83 | NM_018300.2 | 157 |
| **6** | 3420136 | C6orf222 | NM_001010903.3 | 140 |
| **7** | 3440189 | ZBTB20 | NM_015642.3 | 161 |
| **8** | 4220731 | P4HA1 | NM_000917.2 | 257 |
| **9** | 4260767 | GP1BA | NM_000173.4 | 112 |
| **10** | 5080692 | HLA-A29.1 | NM_001080840.1 | 115 |
| **11** | 5550711 | SYNE1 | NM_182961.2 | 186 |
| **12** | 5960072 | | BY797688 | 5 |
| **13** | 6590484 | NAP1L1 | NM_139207.1 | 151 |

### Example 2

### 1: Selection of immunologically relevant gene

In the same way as in Example 1, the short-lived group and the long-lived group were subjected to t-test with focusing on 748 immunologically relevant genes from the 16968 genes. As a result, top 100 genes with a small P-value (large significant difference) were selected as candidates (Table 19). The expression levels (fluorescence reader-measured values) of these 100 genes are shown in the columns "0" (short-lived group) and "1" (long-lived group) of Table 19.

**[Table 19-1]**

| **OBS** | **ProbeID** | **Symbol** | **Accession** | **_0** | **_1** | **_diT01** | **Method** | **Variances** | **tValue** | **DF** | **Probt** | **P_KW** | **flg1** | **flg2** | **flg3** | **FC** | **_lpval_t** | **_lpval_w** |
|---|---|---|---|---|---|---|---|---|---|---|---|---|---|---|---|---|---|---|
| **1** | 4830255 | DPP4 | NM_001935.3 | 6.8716 | 7.179 | -0.3074 | Pooled | Equal | -4.52 | 38 | <.0001 | 0.00019 | 1 | 1 | 2 | -0.3074 | 4.23172 | 3.7229 |
| **2** | 1110091 | TIAL1 | NM_001033925.1 | 7.6534 | 7.8873 | -0.2339 | Pooled | Equal | -3.9 | 38 | 0.0004 | 0.00141 | 1 | 1 | 2 | -0.23391 | 3.42498 | 2.84975 |
| **3** | 6940433 | STAT5B | NM 012448.3 | 8.5812 | 8.8213 | -0.2402 | Pooled | Equal | -3.67 | 38 | 0.0007 | 0.00088 | 1 | 1 | 2 | -0.24015 | 3.12747 | 3.05682 |
| **4** | 2640025 | HP | NM_005143.2 | 7.3689 | 6.9621 | 0.4068 | Pooled | Equal | 3.6 | 38 | 0.0009 | 0.00187 | 1 | 1 | 2 | 0.40682 | 3.03732 | 2.72907 |
| **5** | 3520601 | MPO | NM_000250.1 | 8.129 | 7.1635 | 0.9655 | Pooled | Equal | 3.39 | 38 | 0.0016 | 0.0008 | 1 | 1 | 2 | 0.96555 | 2.7846 | 3.09913 |
| **6** | 6550600 | MYC | NM_002467.3 | 7.5767 | 7.914 | -0.3374 | Pooled | Equal | -3.37 | 38 | 0.0017 | 0.00048 | 1 | 1 | 2 | -0.33736 | 2.75912 | 3.31516 |
| **7** | 3610735 | F12 | NM_000505.3 | 6.8105 | 6.7151 | 0.0954 | Pooled | Equal | 3.09 | 38 | 0.0037 | 0.00245 | 1 | 1 | 2 | 0.09539 | 2.43139 | 2.61106 |
| **8** | 5900100 | BCR | NM 021574.2 | 6.6621 | 6.5878 | 0.0743 | Pooled | Equal | 3.07 | 38 | 0.004 | 0.00204 | 1 | 1 | 2 | 0.07433 | 2.4012 | 2.68944 |
| **9** | 3130669 | SATB1 | NM_002971.2 | 7.0787 | 7.2949 | -0.2162 | Pooled | Equal | -3.05 | 38 | 0.0041 | 0.00803 | 1 | 1 | 2 | -0.21617 | 2.38317 | 2.09542 |
| **10** | 1500735 | CTSG | NM_001911.2 | 8.781 | 7.5263 | 1.2547 | Pooled | Equal | 3 | 38 | 0.0048 | 0.00869 | 1 | 1 | 2 | 1.25466 | 2.3209 | 2.06078 |
| **11** | 5420095 | MYC | NM_002467.3 | 8.2483 | 8.6315 | -0.3832 | Pooled | Equal | -2.91 | 38 | 0.0061 | 0.00451 | 1 | 1 | 2 | -0.38321 | 2.21614 | 2.34605 |
| **12** | 5670739 | AZU1 | NM_001700.3 | 7.3525 | 6.7459 | 0.6066 | Pooled | Equal | 2.89 | 38 | 0.0063 | 0.00741 | 1 | 1 | 2 | 0.60663 | 2.20215 | 2.13034 |
| **13** | 10358 | SPN | NM_001030288.1 | 8.4201 | 8.0743 | 0.3457 | Pooled | Equal | 2.86 | 38 | 0.0069 | 0.00803 | 1 | 1 | 2 | 0.34574 | 2.16135 | 2.09542 |
| **14** | 770021 | PRKRA | NM_003690.3 | 8.4218 | 8.6512 | -0.2294 | Pooled | Equal | -2.82 | 38 | 0.0075 | 0.00803 | 1 | 1 | 2 | -0.22936 | 2.12341 | 2.09542 |
| **15** | 2940767 | CEBPE | NM_001805.2 | 6.7703 | 6.625 | 0.1453 | Pooled | Equal | 2.78 | 38 | 0.0084 | 0.00224 | 1 | 1 | 2 | 0.14529 | 2.0756 | 2.65011 |
| **16** | 4250577 | HSPD1 | NM_002156.4 | 6.5931 | 6.5566 | 0.0365 | Pooled | Equal | 2.76 | 38 | 0.0088 | 0.02655 | 1 | 1 | 2 | 0.0365 | 2.05747 | 1.57598 |
| **17** | 940356 | IL15RA | NM_002189.2 | 6.8246 | 6.7111 | 0.1135 | Pooled | Equal | 2.76 | 38 | 0.0088 | 0.0186 | 1 | 1 | 2 | 0.1135 | 2.05697 | 1.73038 |
| **18** | 6250615 | PGLYRP1 | NM_005091.1 | 8.0051 | 7.3418 | 0.6634 | Pooled | Equal | 2.76 | 38 | 0.0088 | 0.01018 | 1 | 1 | 2 | 0.66337 | 2.05532 | 1.99238 |
| **19** | 4390398 | LCN2 | NM 005564.3 | 9.4014 | 8.4624 | 0.9391 | Pooled | Equal | 2.76 | 38 | 0.0089 | 0.02476 | 1 | 1 | 2 | 0.93905 | 2.04895 | 1.60628 |
| **20** | 5490403 | CD1E | NM_001042588.1 | 6.8164 | 6.9676 | -0.1512 | Pooled | Equal | -2.73 | 38 | 0.0094 | 0.01018 | 1 | 1 | 2 | -0.15121 | 2.02472 | 1.99238 |
| **21** | 6900634 | CD69 | NM_001781.1 | 8.5701 | 9.0537 | -0.4836 | Pooled | Equal | -2.65 | 38 | 0.0117 | 0.011 | 1 | 1 | 2 | -0.48356 | 1.93088 | 1.95862 |
| **22** | 6400176 | IRF7 | NM_004029.2 | 9.7006 | 9.3658 | 0.3348 | Pooled | Equal | 2.64 | 38 | 0.0118 | 0.02149 | 1 | 1 | 2 | 0.3348 | 1.92745 | 1.66776 |
| **23** | 2030767 | CD48 | NM_001 778.2 | 11.9662 | 12.2108 | -0.2446 | Pooled | Equal | -2.63 | 38 | 0.0124 | 0.02845 | 1 | 1 | 2 | -0.24 463 | 1.90778 | 1.54595 |
| **24** | 5360719 | MAPK9 | NM_002752.3 | 6.8029 | 6.9261 | -0.1232 | Pooled | Equal | -2.62 | 38 | 0.0124 | 0.01188 | 1 | 1 | 2 | -0.12321 | 1.90534 | 1.92515 |
| **25** | 5860075 | CAMP | NM 004345.3 | 9.9646 | 8.9318 | 1.0328 | Pooled | Equal | 2.61 | 38 | 0.0129 | 0.02 | 1 | 1 | 2 | 1.03277 | 1.89047 | 1.69892 |
| **26** | 3170543 | TIAL1 | NM 001033925.1 | 8.8499 | 9.0269 | -0.1771 | Pooled | Equal | -2.59 | 38' | 0.0135 | 0.02476 | 1 | 1 | 2 | -0.17707 | 1.86892 | 1.60628 |
| **27** | 360719 | CD44 | NM_000610.3 | 7.2686 | 7.5049 | -0.2363 | Pooled | Equal | -2.58 | 38 | 0.0139 | 0.01282 | 1 | 1 | 2 | -0.23628 | 1.8562 | 1.89196 |
| **28** | 5870138 | VWF | NM_000552.3 | 6.8198 | 6.6721 | 0.1477 | Pooled | Equal | 2.58 | 38 | 0.0139 | 0.05146 | 1 | 0 | 1 | 0.14772 | 1.85561 | 1.28851 |
| **29** | 6370435 | ETS1 | NM_005238.2 | 10.2 | 10.6878 | -0.4678 | Pooled | Equal | -2.57 | 38 | 0.0141 | 0.0058 | 1 | 1 | 2 | -0.46775 | 1.85086 | 2.23688 |
| **30** | 3170017 | MAP2K3 | NM 002756.3 | 8.5386 | 8.2263 | 0.3122 | Pooled | Equal | 2.54 | 38 | 0.0152 | 0.00451 | 1 | 1 | 2 | 0.31225 | 1.81911 | 2.34605 |
| **31** | 1400079 | CRHR1 | NM_004382.3 | 6.9215 | 6.8466 | 0.0749 | Pooled | Equal | 2.54 | 38 | 0.0153 | 0.01282 | 1 | 1 | 2 | 0.07494 | 1.81484 | 1.89196 |
| **32** | 1240450 | CD27 | NM_001242.4 | 8.27 | 8.6406 | -0.3706 | Pooled | Equal | -2.5 | 38 | 0.017 | 0.01383 | 1 | 1 | 2 | -0.37061 | 1.76963 | 1.85907 |
| **33** | 6960072 | HSPD1 | NM_002156.4 | 8.7781 | 9.056 | -0.2779 | Pooled | Equal | -2.49 | 38 | 0.0172 | 0.02655 | 1 | 1 | 2 | -0.27785 | 1.76391 | 1.57598 |
| **34** | 5260315 | ZBTB7B | NM_015872.1 | 6.901 | 6.7793 | 0.1217 | Pooled | Equal | 2.47 | 38 | 0.0183 | 0.011 | 1 | 1 | 2 | 0.12174 | 1.73678 | 1.95862 |
| **35** | 70605 | HSPD1 | NM_002158.4 | 9.9989 | 10.2759 | -0.2769 | Pooled | Equal | -2.45 | 38 | 0.019 | 0.01491 | 1 | 1 | 2 | -0.27691 | 1.72222 | 1.82646 |
| **36** | 5420441 | TNFSF9 | NM_003811.2 | 6.7756 | 6.6769 | 0.0987 | Pooled | Equal | 2.44 | 38 | 0.0195 | 0.0186 | 1 | 1 | 2 | 0.09871 | 1.70969 | 1.73038 |
| **37** | 270156 | UBE2N | NM 003348.3 | 9.546 | 9.757 | -0.2109 | Pooled | Equal | -2.43 | 38 | 0.02 | 0.02 | 1 | 1 | 2 | -0.21093 | 1.69802 | 1.69892 |
| **38** | 3610709 | PAG1 | NM_018440.3 | 8.3033 | 8.4991 | -0.1958 | Pooled | Equal | -2.42 | 38 | 0.0205 | 0.02476 | 1 | 1 | 2 | -0.19579 | 1.68762 | 1.60628 |
| **39** | 1770386 | MALT1 | NM_006785.2 | 8.068 | 8.2886 | -0.2205 | Pooled | Equal | -24 | 38 | 0.0214 | 0.00941 | 1 | 1 | 2 | -0.22 051 | 1.67057 | 2.02644 |
| **40** | 4780075 | CEACAM8 | NM_001816.2 | 7.982 | 7.2856 | 0.6964 | Pooled | Equal | 2.38 | 38 | 0.0222 | 0.02149 | 1 | 1 | 2 | 0.69644 | 1.65321 | 1.66776 |
| **41** | 1690626 | CMKLR1 | NM_004072.1 | 6.7231 | 6.6274 | 0.0956 | Pooled | Equal | 2.38 | 38 | 0.0223 | 0.011 | 1 | 1 | 2 | 0.09561 | 1.65075 | 1.95862 |

**[Table 19-21]**

| | | | | | | | | | | | | | | | | | | |
|---|---|---|---|---|---|---|---|---|---|---|---|---|---|---|---|---|---|---|
| **42** | 780465 | SLC11A1 | NM_000578.3 | 9.8466 | 9.4391 | 0.4075 | Pooled | Equal | 2.36 | 38 | 0.0235 | 0.0186 | 1 | 1 | 2 | 0.4075 | 1.629 | 1.73038 |
| **43** | 1430709 | SPACA3 | NM_173847.3 | 6.6516 | 6.5805 | 0.071 | Pooled | Equal | 2.29 | 38 | 0.0277 | 0.00533 | 1 | 1 | 2 | 0.07103 | 1.55822 | 2.27297 |
| **44** | 870193 | SP140 | NM_001005176.1 | 7.4131 | 7.2877 | 0.1255 | Pooled | Equal | 2.28 | 38 | 0.0286 | 0.06586 | 1 | 0 | 1 | 0.12546 | 1.54419 | 1.18141 |
| **45** | 5290040 | TNFRSF4 | NM_003327.2 | 6.5992 | 6.5555 | 0.0437 | Pooled | Equal | 2.24 | 38 | 0.0313 | 0.04831 | 1 | 1 | 2 | 0.04373 | 1.50467 | 1.31599 |
| **46** | 1050482 | CD46 | NM_172358.1 | 6.8321 | 6.9637 | -0.1316 | Pooled | Equal | -2.23 | 38 | 0.032 | 0.02655 | 1 | 1 | 2 | -0.13161 | 1.49515 | 1.57598 |
| **47** | 2140707 | SLPI | NM_003064.2 | 7.3095 | 6.923 | 0.3864 | Poole d | Equal | 2.21 | 38 | 0.0332 | 0.04248 | 1 | 1 | 2 | 0.38644 | 1.47913 | 1.37178 |
| **48** | 4560193 | CD44 | NM_001001392.1 | 8.9576 | 9.1696 | -0.212 | Pooled | Equal | -2.2 | 38 | 0.0342 | 0.03046 | 1 | 1 | 2 | -0.21201 | 1.46649 | 1.51622 |
| **49** | 4200746 | BPI | NM_001725.1 | 7.8696 | 7.3048 | 0.5649 | Pooled | Equal | 2.18 | 38 | 0.0352 | 0.03487 | 1 | 1 | 2 | 0.56488 | 1.45399 | 1.45759 |
| **50** | 4590026 | IMPDH2 | NM_000884.2 | 9.3239 | 9.5538 | -0.2299 | Pooled | Equal | -2.17 | 38 | 0.0366 | 0.02845 | 1 | 1 | 2 | -0.22987 | 1.43659 | 1.54595 |
| **51** | 5420091 | LTB | NM_002341.1 | 9.8693 | 10.162 | -0.2927 | Pooled | Equal | -2.14 | 38 | 0.0384 | 0.03046 | 1 | 1 | 2 | -0.29269 | 1.41519 | 1.51622 |
| **52** | 5910019 | C1QB | NM_000491.3 | 7.4045 | 7.1647 | 0.2398 | Pooled | Equal | 2.11 | 38 | 0.0417 | 0.04532 | 1 | 1 | 2 | 0.23979 | 1.37949 | 1.34375 |
| **53** | 6840435 | LILRB1 | NM_001081637.1 | 12.6694 | 12.9059 | -0.2366 | Pooled | Equal | -2.09 | 38 | 0.0429 | 0.04532 | 1 | 1 | 2 | -0.23657 | 1.3676 | 1.34375 |
| **54** | 6290343 | CRH | NM_000756.1 | 6.6596 | 6.623 | 0.0366 | Pooled | Equal | 2.07 | 38 | 0.0451 | 0.02476 | 1 | 1 | 2 | 0.03659 | 1.34582 | 1.60628 |
| **55** | 7200392 | C1QBP | NM_001212.3 | 9.8569 | 10.0245 | -0.1675 | Pooled | Equal | -2.06 | 38 | 0.046 | 0.06993 | 1 | 0 | 1 | -0.16752 | 1.33722 | 1.15532 |
| **56** | 4120379 | GFI1 | NM_005263.2 | 7.8526 | 7.6175 | 0.235 | Pooled | Equal | 2.06 | 38 | 0.0463 | 0.05479 | 1 | 0 | 1 | 0.23505 | 1.33486 | 1.26132 |
| **57** | 3990703 | IL10 | NM_000572.2 | 10.7567 | 11.1219 | -0.3652 | Pooled | Equal | -2.05 | 38 | 0.047 | 0.05146 | 1 | 0 | 1 | -0.36524 | 1.32783 | 1.28851 |
| **58** | 1230767 | IFITM2 | NM_006435.2 | 12.8638 | 12.476 | 0.3878 | Pooled | Equal | 2.05 | 38 | 0.0473 | 0.04831 | 1 | 1 | 2 | 0.38784 | 1.3249 | 1.31599 |
| **59** | 6400386 | MAP4K2 | NM_004579.2 | 9.8522 | 9.6326 | 0.2196 | Pooled | Equal | 2.05 | 38 | 0.0474 | 0.05479 | 1 | 0 | 1 | 0.21964 | 1.3242 | 1.26132 |
| **60** | 10142 | CD164 | NM_006016.3 | 6.8434 | 6.9864 | -0.143 | Pooled | Equal | -2.03 | 38 | 0.0489 | 0.02307 | 1 | 1 | 2 | -0.14303 | 1.31036 | 1.63688 |
| **61** | 3180494 | BCL2 | NM_000633.2 | 8.2881 | 8.6083 | -0.3202 | Pooled | Equal | -2.03 | 38 | 0.0493 | 0.0398 | 1 | 1 | 2 | -0.32016 | 1.30743 | 1.4001 |
| **62** | 290592 | CASP8 | NM_033356.3 | 6.7671 | 6.6696 | 0.0975 | Pooled | Equal | 2.03 | 38 | 0.0499 | 0.02845 | 1 | 1 | 2 | 0.09746 | 1.30167 | 1.54595 |
| **63** | 7200386 | GFI1B | NM_004188.3 | 6.6923 | 6.6295 | 0.0628 | Pooled | Equal | 2.01 | 38 | 0.0519 | 0.09893 | 0 | 0 | 0 | 0.06283 | 1.28501 | 1.00466 |
| 64 | 5090368 | HSPA4 | NM_198431.1 | 9.2162 | 9.405 | -0.1888 | Pooled | Equal | -1.99 | 38 | 0.0543 | 0.03726 | 0 | 1 | 1 | -0.18878 | 1.26483 | 1.42871 |
| **65** | 6330445 | CASP3 | NM_004346.3 | 6.6553 | 6.6183 | 0.037 | Pooled | Equal | 1.98 | 38 | 0.0548 | 0.12982 | 0 | 0 | 0 | 0.03704 | 1.2616 | 0.88665 |
| **66** | 4810333 | IL12RB1 | NM_153701.1 | 7.4262 | 7.2541 | 0.1721 | Pooled | Equal | 1.98 | 38 | 0.055 | 0.08342 | 0 | 0 | 0 | 0.17215 | 1.25985 | 1.07876 |
| **67** | 3520167 | CD63 | NM_001040034.1 | 8.9275 | 8.6779 | 0.2495 | Pooled | Equal | 1.98 | 38 | 0.0553 | 0.06586 | 0 | 0 | 0 | 0.24953 | 1.25701 | 1.18141 |
| **68** | 2490537 | TNFRSF1B | NM_001066.2 | 9.3305 | 9.0642 | 0.2663 | Pooled | Equal | 1.98 | 38 | 0.0554 | 0.05479 | 0 | 0 | 0 | 0.26631 | 1.25678 | 1.26132 |
| **69** | 5570730 | TICAM1 | NM_182919.1 | 8.2608 | 7.9802 | 0.2806 | Pooled | Equal | 1.98 | 38 | 0.0554 | 0.15167 | 0 | 0 | 0 | 0.28059 | 1.25666 | 0.8191 |
| **70** | 2900451 | MR1 | NM_001531.1 | 8.1442 | 7.9975 | 0.1467 | Pooled | Equal | 1.91 | 38 | 0.0557 | 0.12311 | 0 | 0 | 0 | 0.14673 | 1.25425 | 0.90971 |
| **71** | 3290441 | BMPR1A | NM_004329.2 | 6.8959 | 6.9693 | -0. 0734 | Pooled | Equal | -1.96 | 38 | 0.0575 | 0.06566 | 0 | 0 | 0 | -0.07343 | 1.24053 | 1.18141 |
| **72** | 6650242 | IFITM3 | NM 021034.2 | 11.7018 | 11.2156 | 0.4862 | Pooled | Equal | 1.94 | 38 | 0.0594 | 0.04248 | 0 | 1 | 1 | 0.48619 | 1.22644 | 1.37178 |
| **73** | 5310053 | LTB | NM_002341.1 | 12.2713 | 12.556 | -0.2846 | Pooled ' | Equal | -1.94 | 38 | 0.0603 | 0.09352 | 0 | 0 | 0 | -0.28465 | 1.21984 | 1.02909 |
| **74** | 1010246 | IFI6 | NM_022872.2 | 9.0927 | 8.5078 | 0.5849 | Pooled | Equal | 1.94 | 38 | 0.0603 | 0.06586 | 0 | 0 | 0 | 0.58489 | 1.21951 | 1.18141 |
| **75** | 3140242 | KIR2DL3 | NM_014511.3 | 9.2033 | 8.6994 | 0.504 | Pooled | Equal | 1.91 | 38 | 0.0635 | 0.08835 | 0 | 0 | 0 | 0.50399 | 1.19711 | 1.05378 |
| **76** | 2970201 | ABHD2 | NM_152924.3 | 6.9306 | 6.8658 | 0.0648 | Pooled | Equal | 1.9 | 38 | 0.0655 | 0.06586 | 0 | 0 | 0 | 0.06478 | 1.18408 | 1.18141 |
| **77** | 4180088 | ABL1 | NM_005157.3 | 7.3424 | 7.1924 | 0.15 | Pooled | Equal | 1.89 | 38 | 0.066 | 0.07421 | 0 | 0 | 0 | 0.14999 | 1.18021 | 1.12953 |
| **78** | 3800725 | SPHK2 | NM 020126.3 | 7.9083 | 7.7437 | 0.1646 | Pooled | Equal | 1.89 | 38 | 0.0666 | 0.10459 | 0 | n | 0 | 0.1646 | 1.17643 | 0.98052 |
| **79** | 5310754 | VNN1 | NM 004666.1 | 7.0161 | 6.888 | 0.128 | Pooled | Equal | 1.88 | 38 | 0.0679 | 0.0398 | 0 | 1 | 1 | 0.12802 | 1.16809 | 1.4001 |
| **88** | 430142 | HSPA4 | NM_002154.3 | 7.086 | 7.2329 | -0.147 | Pooled | Equal | -1.85 | 38 | 0.0717 | 0.05829 | 0 | 0 | 0 | -0.14695 | 1.14455 | 1.2344 |
| **81** | 1030270 | FPR1 | NM_002029.3 | 11.6868 | 11.3479 | 0.3389 | Pooled | Equal | 1.85 | 38 | 0.0725 | 0.09893 | 0 | 0 | 0 | 0.33894 | 1.1395 | 1.00466 |

**[Table 19-3]**

| | | | | | | | | | | | | | | | | | | |
|---|---|---|---|---|---|---|---|---|---|---|---|---|---|---|---|---|---|---|
| **82** | 540671 | ULRB2 | NM_001080978.1 | 9.4993 | 9.272 | 0.2273 | Pooled | Equal | 1.83 | 38 | 0.0744 | 0.02149 | 0 | 1 | 1 | 0.22727 | 1.12837 | 1.66776 |
| **83** | 610601 | LYST | NM_000081.2 | 7.7998 | 7.9303 | -0.1905 | Pooled | Equal | -1.82 | 38 | 0.0763 | 0.0398 | 0 | 1 | 1 | -0.1905 | 1.11768 | 1.4001 |
| **84** | 4810474 | IL18 | NM_001562.2 | 13.2815 | 13.4723 | -0.1908 | Pooled | Equal | -1.81 | 38 | 0.0774 | 0.08835 | 0 | 0 | 0 | -0.1 908 | 1.11102 | 1.05378 |
| **85** | 2810156 | IGF2R | NM_000876.2 | 9.8756 | 9.6428 | 0.2328 | Pooled | Equal | 1.81 | 38 | 0.0788 | 0.1105 | 0 | 0 | 0 | 0.23285 | 1.10327 | 0.95664 |
| **86** | 1190519 | MS4A1 | NM_021950.3 | 6.7216 | 6.8175 | -0.096 | Pooled | Equal | -1.8 | 38 | 0.0791 | 0.23397 | 0 | 0 | 0 | -0.09595 | 1.10207 | 0.63085 |
| **87** | 5390246 | CCR7 | NM_001838.2 | 9.9724 | 10.3942 | -0.42128 | Pooled | Equal | -1.8 | 38 | 0.0792 | 0.09352 | 0 | 0 | 0 | -0.42181 | 1.10921 | 1.02909 |
| **88** | 4250136 | LTB4R | NM_181657.1 | 7.8286 | 7.6741 | 0.1546 | Pooled | Equal | 1.79 | 38 | 0.0813 | 0.06993 | 0 | 0 | 0 | 0.15 458 | 1.08977 | 1.15533 |
| **89** | 610113 | TNFSF14 | NM_003807.2 | 7.4507 | 7.2152 | 0.2355 | Pooled | Equal | 1.78 | 38 | 0.0834 | 0.07421 | 0 | 0 | 0 | 0.23552 | 1.079 | 1.12953 |
| **90** | 3400392 | FPR2 | NM_001462.3 | 7.9307 | 7.6552 | 0.2754 | Pooled | Equal | 1.78 | 38 | 0.0836 | 0.1441 | 0 | 0 | 0 | 0.27 545 | 1.07776 | 0.84135 |
| **91** | 2060377 | TLR3 | NM_003265.2 | 6.6058 | 6.5672 | 0.0385 | Pooled | Equal | 1.77 | 38 | 0.084 | 0.05146 | 0 | 0 | 0 | 0.03854 | 1.07572 | 1.28851 |
| **92** | 2070037 | IC OS | NM 012092.2 | 8.6581 | 8.9752 | -0.317 | Pooled | Equal | -1.77 | 38 | 0.0847 | 0.09352 | 0 | 0 | 0 | -0.31702 | 1.07188 | 1.02909 |
| **93** | 830324 | FLT3LG | NM_001459.2 | 7.9726 | 8.1475 | -0.175 | Pooled | Equal | -1.77 | 38 | 0.0852 | 0.12982 | 0 | 0 | 0 | -0.17496 | 1.06948 | 0.88665 |
| **94** | 3610440 | MAF | NM_005360.3 | 9.0803 | 8.81 46 | 0.2657 | Pooled | Equal | 1.77 | 38 | 0.0853 | 0.09352 | 0 | 0 | 0 | 0.26566 | 1.0688 | 1.02909 |
| **95** | 6520215 | ANXA1 | NM_000700.1 | 12.4165 | 12.6172 | -0.2006 | Pooled | Equal | -1.76 | 38 | 0.0869 | 0.08342 | 0 | 0 | 0 | -0.20065 | 1.06109 | 1.07876 |
| **96** | 5220189 | PIK3AP1 | NM_152309.2 | 7.5028 | 1.6559 | -0.1531 | Pooled | Equal | -1.75 | 38 | 0.0876 | 0.12311 | 0 | 0 | 0 | -0.1531 | 1.05764 | 0.90971 |
| **97** | 620717 | CCL5 | NM_002985.2 | 13.3773 | 13.0768 | 0.3005 | Pooled | Equal | 1.75 | 38 | 0.0889 | 0.02845 | 0 | 1 | 1 | 0.30045 | 1.05105 | 1.54595 |
| **98** | 6110343 | CCL23 | NM_145898.1 | 7.3429 | 7.2314 | 0.1115 | Pooled | Equal | 1.74 | 38 | 0.0891 | 0.15954 | 0 | 0 | 0 | 0.11147 | 1.04993 | 0.79712 |
| **99** | 5490750 | RELA | NM_021975.2 | 7.1608 | 7.006 | 0.1547 | Pooled | Equal | 1.74 | 38 | 0.0893 | 0.09352 | 0 | 0 | 0 | 0.15475 | 1.04908 | 1.02909 |
| **100** | 160601 | ST6GA L1 | NM_173216.1 | 6.7798 | 6.8588 | -0.079 | Pooled | Equal | -1.74 | 38 | 0.0903 | 0.15167 | 0 | 0 | 0 | -0.07899 | 1.04414 | 0.8191 |

As a result of performing variable selection using the 100 immunologically relevant genes, 29 genes were selected (Table 20). The gene set of these 29 genes permitted prediction with a discrimination rate of 100% in training data (40 individuals) as to the discrimination of long life or short life. The discrimination of test data (11 individuals) based on this gene set resulted in a short life discrimination rate (ctcP0) of 83.33% and a long life discrimination rate (ctcP1) of 80% (Table 21).

**[Table 20]**

| **OBS** | **probeID** | **Symbol** | **Accession** |
|---|---|---|---|
| **1** | 610113 | TNFSF14 | NM_003807.2 |
| **2** | 610170 | EREG | NM_001432.2 |
| **3** | 870156 | CD1A | NM_001763.2 |
| **4** | 940356 | IL15RA | NM_002189.2 |
| **5** | 1110091 | TIAL1 | NM_001033925.1 |
| **6** | 1300274 | ANXA11 | NM_001157.2 |
| **7** | 1450008 | IL16 | NM_172217.1 |
| **8** | 2260731 | ERAP2 | NM_022350.2 |
| **9** | 2640025 | HP | NM_005143.2 |
| **10** | 2750324 | PRKCZ | NM_002744.4 |
| **11** | 2760500 | CD38 | NM_001775.2 |
| **12** | 3420026 | FAS | NM_152877.1 |
| **13** | 3520601 | MPO | NM_000250.1 |
| **14** | 4210612 | AP3D1 | NM_003938.5 |
| **15** | 4220152 | SIRPG | NM_018556.3 |
| **16** | 4290736 | MAP2K2 | NM_030662.2 |
| **17** | 4390241 | BCL2L11 | NM_207002.2 |
| **18** | 4830255 | DPP4 | NM_001935.3 |
| **19** | 5080608 | LAT | NM_014387.3 |
| **20** | 5420095 | MYC | NM_002467.3 |
| **21** | 5810685 | THBS1 | NM_003246.2 |
| **22** | 5900136 | CLEC4C | NM_130441.2 |
| **23** | 6220639 | HSF1 | NM_005526.2 |
| **24** | 6450390 | IL2RG | NM_000206.1 |
| **25** | 6520725 | TNFRSF14 | NM_003820.2 |
| **26** | 6580408 | CTSW | NM_001335.3 |
| **27** | 6900424 | TYK2 | NM_003331.3 |
| **28** | 7210543 | PLD2 | NM_002663.2 |
| **29** | 7610390 | NOD1 | NM_006092.1 |

**[Table 21]**

| pbnum | ccvP0 | ccvP1 | ctcP0 | ctcP1 | flg_ccv | flg_ctc |
|---|---|---|---|---|---|---|
| 29 | 100 | 100 | 83.33 | 80 | 1 | 1 |

From these 29 immunologically relevant genes, top 11 genes that were considered particularly relevant and exhibited the high correlation between survival time and gene expression were selected, and addition of a TNF-related gene (2360672) (a total of 12 genes) resulted in selecting 12 genes (Table 22).

**[Table 22]**

| **OBS** | **probelD** | **Symbol** | **Accession** |
|---|---|---|---|
| **1** | 610113 | TNFSF14 | NM_003807.2 |
| **2** | 940356 | IL15RA | NM_002189.2 |
| **3** | 1110091 | TIAL1 | NM_001033925.1 |
| **4** | 2360672 | TNFRSF19 | NM_148957.2 |
| **5** | 2640025 | HP | NM_005143.2 |
| **6** | 3420026 | FAS | NM_152877.1 |
| **7** | 3520601 | MPO | NM_000250.1 |
| **8** | 4830255 | DPP4 | NM_001935.3 |
| **9** | 5420095 | MYC | NM_002467.3 |
| **10** | 5960136 | CLEC4C | NM_130441.2 |
| **11** | 6450390 | IL2RG | NM_000206.1 |
| **12** | 6520725 | TNFRSF14 | NM_003820.2 |

### 2: Discrimination rate based on selected gene

### (1) Discrimination rate based on combinations of 1 to 12 genes

Of the 12 genes, one gene probe 4830255 (Table 23) permitted prediction with a high rate (ccvP0 + ccvP1 + ctcP0 + ctcP1 > 290) by itself. This gene probe permitted prediction with 90% probability in the training data (40 individuals) and 50% probability in the test data (11 individuals) as to the discrimination of short life and with 70% probability in the training data and 80% probability in the test data as to the discrimination of long life.

**[Table 23]**

| **pb1** | **ccvP0** | **ccvP1** | **ctcP0** | **ctcP1** | **sumP** | **flg_ccv** | **fig_ctc** |
|---|---|---|---|---|---|---|---|
| 4830255 | 90 | 70 | 50 | 80 | 290 | 0 | 0 |

Of the 12 genes, examples of 2 to 11 gene probes that permitted prediction with a high rate (ccvP0 + ccvP1 + ctcP0 + ctcP1 > 290) are shown in the following Tables:

**[Table 24]**

| Combination of 2 genes | | | | | | | | |
|---|---|---|---|---|---|---|---|---|
| **pb1** | **pb2** | **ccvP0** | **ccvP1** | **ctcP0** | **ctcP1** | **sumP** | **flg_ccv** | **flg_ctc** |
| 2360672 | 5420095 | 75 | 85 | 50 | 100 | 310 | 0 | 0 |
| 4830255 | 64503090 | 90 | 70 | 67 | 80 | 307 | 0 | 0 |
| 940356 | 4830255 | 85 | 70 | 67 | 80 | 302 | 0 | 0 |
| 6010113 | 4830255 | 90 | 70 | 50 | 80 | 290 | 0 | 0 |

**[Table 25]**

| Combination of 3 genes | | | | | | | | | |
|---|---|---|---|---|---|---|---|---|---|
| **pb1** | **pb2** | **pb3** | **ccvP0** | **ccvP1** | **ctcP0** | **ctcP1** | **sumP** | **flg_ccv** | **flg_ctc** |
| 940356 | 2360672 | 4830255 | 95 | 80 | 67 | 80 | 322 | 1 | 0 |
| 610113 | 1110091 | 2360672 | 85 | 80 | 50 | 100 | 315 | 1 | 0 |
| 610113 | 2360672 | 4830255 | 85 | 80 | 67 | 80 | 312 | 1 | 0 |
| 2360672 | 7640025 | 4830255 | 85 | 80 | 67 | 80 | 312 | 1 | n |
| 2360672 | 3420026 | 5420095 | 75 | 85 | 50 | 100 | 310 | 0 | 0 |
| 1110091 | 2380672 | 4830255 | 85 | 90 | 33 | 100 | 308 | 1 | 0 |
| 610113 | 4830255 | 6450390 | 90 | 70 | 67 | 80 | 307 | n | 0 |
| 2360672 | 3420026 | 4830255 | 90 | 85 | 50 | 80 | 305 | 1 | 0 |
| 2360672 | 4830255 | 6520725 | 90 | 85 | 50 | 80 | 305 | 1 | 0 |
| 610113 | 2360672 | 2640025 | 65 | 75 | 83 | 80 | 303 | 0 | 1 |
| 610113 | 940356 | 4830255 | 85 | 70 | 67 | 80 | 302 | 0 | 0 |
| 3420026 | 4830255 | 6450390 | 90 | 65 | 67 | 80 | 302 | 0 | n |
| 4830255 | 6450390 | 6520725 | 85 | 70 | 67 | 80 | 302 | 0 | 0 |
| 2360672 | 5420095 | 5960136 | 70 | 80 | 50 | 100 | 300 | 0 | 0 |
| 11110091 | 2360672 | 2640025 | 85 | 80 | 33 | 100 | 298 | 1 | 0 |
| 1110091 | 2380672 | 5420095 | 75 | 90 | 33 | 100 | 298 | 0 | 0 |
| 2380672 | 5420095 | 6460390 | 70 | 75 | 50 | 100 | 295 | 0 | 0 |
| 1110091 | 2360672 | 6459390 | 80 | 80 | 33 | 100 | 293 | 1 | 0 |
| 610113 | 3520601 | 5420095 | 70 | 90 | 33 | 100 | 293 | 0 | 0 |
| 2360672 | 3520601 | 5420095 | 75 | 85 | 33 | 100 | 293 | 0 | 0 |
| 3520601 | 5420095 | 5960136 | 70 | 90 | 33 | 100 | 293 | 0 | 0 |
| 610113 | 940356 | 2360672 | 70 | 75 | 67 | 80 | 292 | 0 | 0 |
| 2360672 | 2640025 | 6520725 | 65 | 80 | 67 | 80 | 292 | 0 | 0 |
| 3420026 | 4830255 | 5950136 | 90 | 70 | 50 | 80 | 290 | 0 | 0 |

**[Table 26]**

| Combination of 4 genes | | | | | | | | | | |
|---|---|---|---|---|---|---|---|---|---|---|
| **ph1** | **pb2** | **pb3** | **pb4** | **ccvP0** | **ccvP1** | **ctcP0** | **ctcP1** | **cumP** | **flg_ccv** | **flg_ctc** |
| 940356 | 2360872 | 4830255 | 6520725 | 85 | 70 | 83 | 80 | 328 | 0 | 1 |
| 610113 | 11100191 | 2360672 | 3420026 | 85 | 80 | 50 | 100 | 315 | 1 | 0 |
| 610113 | 11100191 | 2360672 | 5960136 | 85 | 80 | 50 | 100 | 315 | 1 | 0 |
| 610113 | 11100191 | 2360672 | 6520725 | 85 | 80 | 50 | 100 | 315 | 1 | 0 |
| 610113 | 2360672 | 2640025 | 4830256 | 85 | 80 | 67 | 80 | 312 | 1 | 0 |
| 2300672 | 2640025 | 4830255 | 6520725 | 85 | 80 | 67 | 80 | 312 | 1 | 0 |
| 610113 | 940356 | 2360672 | 4830255 | 80 | 75 | 67 | 80 | 31.2 | 0 | 0 |
| 940356 | 2360657.2 | 4830255 | 5960136 | 98 | 70 | 67 | 80 | 312 | 0 | 0 |
| 610113 | 1110091 | 2360672 | 6450390 | 80 | 80 | 50 | 100 | 310 | 1 | 0 |
| 610113 | 1110091 | 2360672 | 5420095 | 75 | 85 | 50 | 100 | 310 | 0 | 0 |
| 1110091 | 2360672 | 4830255 | 5960136 | 85 | 90 | 33 | 100 | 308 | 1 | 0 |
| 2360672 | 2640025 | 4830255 | 5960136 | 80 | 80 | 67 | 80 | 307 | 1 | 0 |
| 610113 | 2360672 | 4830255 | 6460390 | 85 | 75 | 67 | 80 | 307 | 0 | 0 |
| 610113 | 2360672 | 4830255 | 6520725 | 85 | 75 | 67 | 80 | 307 | 0 | 0 |
| 2360672 | 2640025 | 3420026 | 4830255 | 85 | 75 | 67 | 80 | 307 | 0 | 0 |
| 2360672 | 2640025 | 4830255 | 6450390 | 85 | 75 | 67 | 80 | 307 | 0 | 0 |
| 2360672 | 3420026 | 4830255 | 6450390 | 90 | 85 | 50 | 80 | 305 | 1 | 0 |
| 2360672 | 53420026 | 4830255 | 6520725 | 90 | 85 | 50 | 80 | 305 | 1 | 0 |
| 2360672 | 483025 | 6450390 | 6520725 | 90 | 85 | 50 | 80 | 305 | 1 | 0 |
| 2360672 | 3420026 | 5420095 | 5960136 | 70 | 85 | 50 | 100 | 305 | 0 | 0 |
| 2360672 | 3520601 | 5420095 | 5960136 | 70 | 85 | 50 | 100 | 305 | 0 | 0 |
| 610113 | 1110091 | 2360672 | 4830255 | 85 | 85 | 33 | 100 | 303 | 1 | 0 |
| 940356 | 1110091 | 2360672 | 3520601 | 90 | 80 | 33 | 100 | 303 | 1 | 0 |
| 1110091 | 3420026 | 3520601 | 4830255 | 90 | 80 | 33 | 100 | 303 | 1 | 0 |
| 940356 | 238067.2 | 2640025 | 3520601 | 85 | 85 | 50 | 80 | 300 | 1 | 0 |
| 940356 | 2360672 | 2640025 | 5420085 | 85 | 85 | 50 | 80 | 300 | 1 | 0 |
| 2360672 | 483025.5 | 5960138 | 6520725 | 90 | 80 | 50 | 80 | 300 | 1 | 0 |
| 2360672 | 3420026 | 5420095 | 5520725 | 70 | 80 | 50 | 100 | 300 | 0 | 0 |
| 610113 | 940356 | 1110091 | 2360672 | 85 | 80 | 33 | 100 | 298 | 1 | 0 |
| 1110091 | 2360672 | 2640025 | 6520725 | 85 | 80 | 33 | 100 | 298 | 1 | 0 |
| 1110091 | 2360672 | 3520601 | 5420095 | 90 | 80 | 33 | 100 | 298 | 1 | 0 |
| 1110091 | 236087.2 | 5420095 | 6450390 | 80 | 80 | 33 | 100 | 298 | 1 | 0 |
| 610113 | 2360672 | 2640025 | 5960136 | 80 | 75 | 83 | 80 | 298 | 0 | 0 |
| 610113 | 2360672 | 2640025 | 6450390 | 65 | 70 | 83 | 100 | 298 | 0 | 1 |
| 1110091 | 2360672 | 3420026 | 5420095 | 75 | 90 | 33 | 100 | 298 | 0 | 0 |
| 1110091 | 2360672 | 5420095 | 5960136 | 75 | 90 | 33 | 100 | 298 | 0 | 0 |
| 1110091 | 2360672 | 5420095 | 6520725 | 75 | 90 | 33 | 100 | 298 | 0 | 0 |
| 610113 | 2360672 | 2840025 | 3420025 | 75 | 75 | 67 | 80 | 297 | 0 | 0 |
| 610113 | 2640025 | 2640025 | 4830255 | 85 | 85 | 67 | 80 | 297 | 0 | 0 |
| 610113 | 3420026 | 4830255 | 6450390 | 85 | 65 | 67 | 80 | 297 | 0 | 0 |
| 610113 | 2360672 | 3420026 | 4830255 | 85 | 80 | 50 | 80 | 295 | 1 | 0 |
| 2360672 | 2640025 | 3520601 | 4830255 | 85 | 80 | 50 | 80 | 295 | 1 | 0 |
| 610113 | 940356 | 2360672 | 3420026 | 90 | 75 | 50 | 80 | 295 | 0 | 0 |
| 610113 | 940356 | 3520601 | 54830255 | 65 | 80 | 50 | 100 | 295 | 0 | 0 |
| 940358 | 2236067 | 2640025 | 4830255 | 90 | 75 | 50 | 80 | 295 | 0 | 0 |

**[Table 27]**

| Combination of 5 genes | | | | | | | | | | | |
|---|---|---|---|---|---|---|---|---|---|---|---|
| **pb1** | **pb2** | **pb3** | **pb4** | **pb5** | **ccvP0** | **ccvP1** | **ctcP0** | **ctcP1** | **sunP** | **flg_ccv** | **flg_ctc** |
| 610113 | 940356 | 2360672 | 4830255 | 483025 | 95 | 70 | 83 | 80 | 32.8 | 0 | 1 |
| 2360372 | 2640025 | 4830255 | 6450390 | 6520725 | 90 | 80 | 67 | 80 | 317 | 1 | 0 |
| 610113 | 1110091 | 2360672 | 3420026 | 5960136 | 85 | 80 | 50 | 100 | 315 | 1 | 0 |
| 610113 | 1110091 | 2360672 | 3420026 | 6520725 | 85 | 80 | 50 | 100 | 315 | 1 | 0 |
| 610113 | 1110091 | 2360672 | 5960136 | 6520725 | 85 | 80 | 50 | 100 | 315 | 1 | 0 |
| 610113 | 1110091 | 2360672 | 6450390 | 6520725 | 85 | 80 | 50 | 100 | 315 | 1 | 0 |
| 61113 | 2360672 | 2640025 | 4830255 | 8520725 | 85 | 80 | 67 | 80 | 312 | 1 | 0 |
| 2360672 | 2640025 | 4830255 | 4830255 | 6520725 | 85 | 80 | 67 | 80 | 312 | 1 | 0 |
| 2360672 | 2640025 | 3520601 | 5960136 | 6450390 | 85 | 80 | 67 | 80 | 312 | 1 | 0 |
| 610113 | 2360672 | 2640025 | 3420026 | 3520601 | 65 | 80 | 67 | 100 | 312 | 0 | 0 |
| 610113 | 1110091 | 1110091 | 2640025 | 5960136 | 80 | 80 | 50 | 100 | 310 | 1 | 0 |
| 610113 | 1110091 | 2360672 | 3420026 | 6450390 | 80 | 80 | 50 | 100 | 310 | 1 | 0 |
| 610113 | 1110091 | 2360672 | 3420026 | 6450390 | 75 | 5 | 50 | 100 | 310 | 0 | 0 |
| 610113 | 1110091 | 2360672 | 5420095 | 5960136 | 75 | 85 | 50 | 100 | 310 | 0 | 0 |
| 610113 | 1110091 | 2360672 | 5420095 | 6450390 | 75 | 85 | 50 | 100 | 310 | 0 | 0 |
| 1110091 | 2360672 | 4830255 | 6450390 | 6520725 | 85 | 90 | 33 | 100 | 308 | 1 | 0 |
| 610113 | 2360672 | 2640025 | 4830255 | 59601 | 86 | 80 | 67 | 80 | 307 | 1 | 0 |
| 610113 | 2360672 | 2640025 | 3420026 | 4830255 | 85 | 75 | 67 | 80 | 307 | 0 | 0 |
| 610113 | 2360672 | 2640025 | 4830255 | 6450390 | 85 | 75 | 80 | 80 | 307 | 0 | 0 |
| 2360672 | 2640025 | 3420026 | 4830255 | 6450390 | 85 | 75 | 67 | 80 | 307 | 0 | 0 |
| 2360672 | 2640025 | 3420026 | 4830255 | 6520725 | 85 | 75 | 67 | 80 | 307 | 0 | 0 |
| 2360672 | 2640025 | 4830255 | 5960136 | 6450390 | 85 | 75 | 67 | 80 | 307 | 0 | 0 |
| 940356 | 2360672 | 2640025 | 2640025 | 6450390 | 90 | 85 | 50 | 80 | 305 | 0 | 0 |
| 940356 | 2360672 | 2670025 | 4830255 | 8520725 | 95 | 80 | 50 | 80 | 305 | 1 | 0 |
| 1110091 | 2360672 | 2640025 | 4830255 | 5960136 | 90 | 85 | 85 | 80 | 80 | 1 | 0 |
| 1110091 | 2360672 | 2640025 | 4830255 | 6450390 | 90 | 95 | 50 | 90 | 305 | 1 | 0 |
| 2360672 | 3420026 | 4630255 | 6450390 | 6520725 | 90 | 85 | 50 | 80 | 305 | 1 | 0 |
| 610113 | 1110091 | 2360672 | 5420095 | 6520725 | 75 | 80 | 50 | 100 | 305 | 0 | 0 |

**[Table 28]**

| Combination of 6 genes | | | | | | | | | | | | |
|---|---|---|---|---|---|---|---|---|---|---|---|---|
| **pb1** | **pb2** | **pb3** | **pb4** | **pb5** | **pb6** | **ccvP0** | **ccvP1** | **ctcP1** | **sunP** | **flg_ccv** | **flg_ctc** | **fig_ctc** |
| 2360872 | 3420026 | 5420095 | 3420026 | 5420095 | 6450390 | 75 | 85 | 67 | 100 | 327 | 0 | 0 |
| 610113 | 1110081 | 2360672 | 5420085 | 5960136 | 6450390 | 70 | 85 | 67 | 100 | 322 | 0 | 0 |
| 610113 | 1110091 | 2360672 | 5420095 | 6450390 | 6520725 | 80 | 75 | 67 | 100 | 322 | 0 | 0 |
| 610113 | 1110091 | 2360672 | 4830255 | 6450390 | 6520725 | 85 | 85 | 50 | 100 | 320 | 1 | 0 |
| 610013 | 2360672 | 2640025 | 4630255 | 6450390 | 6520725 | 90 | 80 | 67 | 80 | 317 | 1 | 0 |
| 1110091 | 2360672 | 2640025 | 3420026 | 4830255 | 6450390 | 90 | 80 | 67 | 60 | 317 | 1 | 0 |
| 610113 | 1110081 | 2360672 | 3420026 | 5960136 | 6520725 | 95 | 80 | 50 | 100 | 315 | 1 | 0 |
| 610113 | 1110091 | 2360672 | 6450390 | 645390 | 6520725 | 85 | 80 | 50 | 100 | 315 | 1 | 0 |
| 610113 | 1110091 | 2350672 | 5960136 | 6450390 | 6520725 | 85 | 80 | 50 | 100 | 315 | 1 | 0 |
| 1110091 | 2380672 | 3420026 | 4830255 | 6450390 | 6520725 | 80 | 85 | 50 | 100 | 100 | 1 | 0 |
| 1110091 | 2360672 | 3520601 | 4830255 | 6450390 | 6520725 | 80 | 85 | 50 | 100 | 315 | 1 | 0 |
| 610113 | 2360672 | 2640025 | 3520601 | 4830255 | 6450390 | 85 | 80 | 67 | 80 | 312 | 1 | 0 |
| 2360672 | 2640025 | 3420026 | 3520601 | 4830255 | 6450390 | 85 | 80 | 67 | 80 | 312 | 1 | 0 |
| 2360572 | 2640025 | 3420026 | 3520601 | 4830255 | 8520725 | 85 | 80 | 67 | 80 | 312 | 1 | 0 |
| 610113 | 1110081 | 2360672 | 610113 | 6450380 | 6520725 | 90 | 75 | 67 | 80 | 312 | 0 | 0 |
| 610113 | 1110081 | 2360672 | 2640025 | 3420026 | 5960136 | 80 | 80 | 50 | 100 | 310 | 1 | 0 |
| 610113 | 111008 | 2380672 | 2640025 | 5960136 | 6520725 | 0 | 80 | 50 | 100 | 310 | 1 | 0 |
| 610113 | 1110091 | 2360672 | 3420026 | 5420095 | 5960136 | 75 | 85 | 50 | 100 | 310 | 0 | 0 |
| 610113 | 1110091 | 3420026 | 3520601 | 4830255 | 6520725 | 90 | 85 | 33 | 100 | 308 | 1 | 0 |
| 940356 | 1110091 | 2360672 | 2640025 | 3520601 | 6520725 | 90 | 85 | 33 | 100 | 308 | 1 | 0 |
| 1110091 | 2360672 | 4830255 | 5960136 | 6450390 | 6520725 | 85 | 90 | 33 | 100 | 308 | 1 | 0 |
| 610113 | 2360672 | 2640025 | 4830255 | 5950136 | 6520725 | 80 | 80 | 67 | 60 | 307 | 1 | 0 |
| 940356 | 1110091 | 3420026 | 3520601 | 4830255 | 5520725 | 100 | 100 | 17 | 100 | 307 | 1 | 0 |
| 2360672 | 2640025 | 3520601 | 4830255 | 8450390 | 6450390 | 80 | 80 | 67 | 80 | 307 | 1 | 0 |
| 610113 | 2360672 | 2640025 | 3420026 | 4830255 | 6450390 | 85 | 75 | 67 | 80 | 307 | 0 | 0 |
| 610113 | 2360672 | 2640025 | 4830255 | 5960136 | 6450390 | 95 | 75 | 67 | 80 | 307 | 0 | 0 |
| 610113 | 2360672 | 2640025 | 4830255 | 596016 | 6450390 | 85 | 75 | 67 | 80 | 307 | 0 | 0 |
| 940355 | 2360672 | 2640025 | 5960136 | 5450390 | 6520725 | 85 | 75 | 67 | 80 | 307 | 0 | 0 |
| 2360672 | 2640025 | 3420026 | 4830255 | 5960136 | 6450390 | 85 | 75 | 67 | 80 | 307 | 0 | 0 |
| 2360572 | 2640025 | 3420026 | 4830255 | 5960136 | 6520725 | 85 | 75 | 67 | 80 | 307 | 0 | 0 |
| 2360672 | 2640025 | 3420026 | 4830255 | 6450390 | 6520725 | 85 | 75 | 67 | 80 | 307 | 0 | 0 |
| 2360672 | 2640025 | 3520601 | 4830255 | 6450390 | 6450390 | 85 | 75 | 67 | 80 | 307 | 0 | 0 |
| 610113 | 940358 | 2360672 | 2640025 | 3520801 | 4830255 | 90 | 75 | 85 | 80 | 305 | 1 | 0 |
| 610113 | 940358 | 2360672 | 2640025 | 4830255 | 6520725 | 95 | 80 | 50 | 80 | 305 | 1 | 0 |
| 610113 | 1110091 | 2360672 | 2640025 | 4830255 | 5960136 | 90 | 80 | 85 | 80 | 305 | 1 | 0 |
| 610113 | 1110091 | 2640025 | 2640025 | 4830255 | 6450390 | 90 | 85 | 50 | 80 | 305 | 1 | 0 |
| 1110091 | 2380572 | 313520601 | 4830255 | 4630255 | 5450390 | 90 | 85 | 50 | 80 | 305 | 1 | 0 |

**[Table 29]**

| Combination of 7 genes | | | | | | | | | | | | | |
|---|---|---|---|---|---|---|---|---|---|---|---|---|---|
| **pb1** | **pb2** | **pb3** | **pb4** | **pb5** | **pb6** | **pv7** | **ccvP0** | **ccvP1** | **ctcP0** | **ctcP1** | **sunP** | **flg_ccv** | **flg_ctc** |
| 610113 | 1110091 | 3420026 | 3520601 | 4330255 | 6450390 | 6520725 | 90 | 85 | 50 | 100 | 325 | 1 | 0 |
| 610113 | 1110091 | 2360672 | 4830255 | 5960136 | 6450390 | 6520725 | 85 | 85 | 50 | 100 | 320 | 1 | 0 |
| 610113 | 2360672 | 2640025 | 3420026 | 4930255 | 4030255 | 6450390 | 90 | 80 | 67 | 60 | 317 | 1 | 0 |
| 1110091 | 2350672 | 2640025 | 3420026 | 4330255 | 5900136 | 6450390 | 90 | 80 | 67 | 80 | 317 | 1 | 0 |
| 610113 | 1110091 | 2360672 | 3420026 | 5420095 | 5960136 | 6450390 | 70 | 80 | 67 | 100 | 317 | 0 | 0 |
| 610113 | 1110091 | 2360672 | 3420026 | 5420095 | 6450390 | 6520725 | 75 | 75 | 67 | 100 | 317 | 0 | 0 |
| 610113 | 1110091 | 2360572 | 5420095 | 5960136 | 6450390 | 6520725 | 75 | 75 | 67 | 100 | 317 | 0 | 0 |
| 610113 | 1110091 | 2350672 | 3420026 | 4830255 | 6450390 | 6520725 | 80 | 85 | 50 | 100 | 315 | 1 | 0 |
| 1110091 | 2360672 | 2640025 | 3520601 | 4830255 | 6450390 | 6520725 | 80 | 85 | 50 | 100 | 315 | 1 | 0 |
| 610113 | 2360672 | 2640025 | 3420026 | 3520601 | 4830255 | 6450390 | 85 | 80 | 67 | 80 | 312 | 1 | 0 |
| 610113 | 2360672 | 2640025 | 3420026 | 3520601 | 4830255 | 5520725 | 85 | 80 | 67 | 80 | 312 | 1 | 0 |
| 940356 | 2360672 | 2640025 | 4830255 | 5420095 | 5960136 | 6520725 | 85 | 80 | 67 | 80 | 312 | 1 | 0 |
| 610113 | 2360672 | 2640025 | 4830255 | 5960136 | 6450390 | 6520725 | 90 | 75 | 67 | 80 | 312 | 0 | 0 |
| 2360672 | 2640025 | 3420026 | 4830255 | 5960136 | 6450390 | 6520725 | 90 | 75 | 67 | 80 | 312 | 0 | 0 |
| 610113 | 1110091 | 2360672 | 2640025 | 3420026 | 5950136 | 6520725 | 80 | 80 | 50 | 100 | 310 | 1 | 0 |
| 610113 | 1110091 | 2380672 | 3420028 | 5960138 | 8450390 | 5520725 | 80 | 80 | 50 | 100 | 310 | 1 | 0 |
| 610113 | 1110091 | 2360672 | 3520601 | 4830255 | 6450390 | 5520725 | 80 | 80 | 50 | 100 | 310 | 1 | 0 |
| 610113 | 2360672 | 2640025 | 3520601 | 4830255 | 6450390 | 8520725 | 80 | 80 | 67 | 80 | 307 | 1 | 0 |
| 2360672 | 2640025 | 3420026 | 3520601 | 4330255 | 6450390 | 6520725 | 80 | 80 | 67 | 80 | 307 | 1 | 0 |
| 610113 | 2380672 | 2640025 | 3420026 | 4830255 | 5960136 | 6520725 | 85 | 75 | 67 | 80 | 307 | 0 | 0 |
| 610113 | 2360672 | 2640025 | 3420026 | 4830255 | 6450390 | 6520725 | 85 | 75 | 67 | 80 | 307 | 0 | 0 |
| 610113 | 2360672 | 2640025 | 3520601 | 4830255 | 5980136 | 6450390 | 95 | 75 | 67 | 80 | 307 | 0 | 0 |
| 2360672 | 2640025 | 3420026 | 3520601 | 4330255 | 5960136 | 6450390 | 85 | 75 | 67 | 80 | 307 | 0 | 0 |
| 810113 | 1110091 | 2380672 | 2870025 | 4830255 | 5960136 | 5450390 | 90 | 85 | 50 | 80 | 305 | 1 | 0 |
| 940356 | 1110091 | 2360672 | 2640025 | 3420026 | 4830255 | 5960136 | 95 | 80 | 50 | 80 | 305 | 1 | 0 |
| 610113 | 1110091 | 2350672 | 2640025 | 3420026 | 3520601 | 5960136 | 75 | 80 | 50 | 100 | 305 | 0 | 0 |
| 610113 | 1110091 | 2360672 | 3420026 | 5420095 | 5960136 | 6520725 | 75 | 80 | 80 | 100 | 305 | | 0 |
| 610113 | 940358 | 1110091 | 2360672 | 3420026 | 3520501 | 6520725 | 90 | 80 | 33 | 100 | 303 | 1 | 0 |
| 610113 | 1110091 | 2360672 | 2640025 | 4830255 | 5960136 | 6520725 | 85 | 85 | 33 | 100 | 303 | 1 | 0 |
| 1110091 | 2360672 | 3420026 | 3520601 | 4830255 | 6450390 | 6520725 | 85 | 85 | 33 | 100 | 303 | 1 | 0 |
| 610113 | 940356 | 1110091 | 3520601 | 4330255 | 5960136 | 8520725 | 95 | 90 | 17 | 100 | 302 | 1 | 0 |

**[Table 30]**

| Combination of 8 genes | | | | | | | | | | | | | |
|---|---|---|---|---|---|---|---|---|---|---|---|---|---|
| **pb1** | **pb2** | **pb3** | **pb4** | **pb5** | **pb6** | **pb7** | **pb8** | **ccvP0** | **ccvP1** | **ctcP0** | **ctcP1** | **sunP** | **flg_ccv** |
| 610113 | 1110091 | 2360672 | 2640025 | 3430026 | 4630255 | 5960136 | 6450390 | 90 | 80 | 66.67 | 80 | 317 | 1 |
| 610113 | 1110091 | 3420026 | 3520801 | 4830255 | 5960136 | 6450390 | 6520725 | 85 | 80 | 50 | 100 | 315 | 1 |
| 610113 | 2360672 | 2640025 | 3420026 | 4830255 | 5960136 | 6450390 | 6520125 | 90 | 75 | 66.67 | 80 | 312 | 0 |
| 610113 | 1110091 | 2360672 | 3420036 | 4830255 | 5960136 | 6450390 | 6520725 | 80 | 80 | 50 | 100 | 310 | 1 |
| 610113 | 1110091 | 2640025 | 3420026 | 3520601 | 4830255 | 6450350 | 6520725 | 80 | 80 | 50 | 100 | 310 | 1 |
| 1110091 | 2360672 | 2640025 | 3520601 | 4831255 | 5960136 | 6450390 | 6520725 | 80 | 80 | 50 | 100 | 310 | 1 |
| 940356 | 1110061 | 2360672 | 2840025 | 3520601 | 5420095 | 5960136 | 6520725 | 80 | 85 | 33.33 | 100 | 308 | 1 |
| 610113 | 2360672 | 2640025 | 3420026 | 3520601 | 4630255 | 6450390 | 6520725 | 80 | 80 | 68.67 | 80 | 307 | 1 |
| 610113 | 940356 | 2360672 | 2640025 | 4830255 | 5420095 | 5960136 | 6520725 | 85 | 75 | 66.67 | 80 | 307 | 0 |
| 610113 | 2360872 | 2640025 | 3420026 | 3520601 | 4830255 | 5960136 | 6450390 | 85 | 75 | 68.67 | 80 | 307 | 0 |
| 610113 | 1110091 | 2360672 | 2640025 | 3420026 | 3520601 | 5980136 | 6520725 | 75 | 80 | 50 | 100 | 305 | 0 |
| 610113 | 1110091 | 2380872 | 2640025 | 3520601 | 4830255 | 6450390 | 6520725 | 80 | 75 | 50 | 100 | 305 | 0 |
| 610113 | 940356 | 1110091 | 2360672 | 2640025 | 3420026 | 4830256 | 5960136 | 95 | 80 | 50 | 80 | 305 | 1 |
| 610113 | 940356 | 1110091 | 2360672 | 264025 | 4830255 | 5960136 | 6450390 | 85 | 80 | 50 | 80 | 305 | 1 |
| 940356 | 1110091 | 2360672 | 2640025 | 3420025 | 4830255 | 5980136 | 6450390 | 85 | 80 | 50 | 80 | 305 | 1 |
| 610113 | 940356 | 1110091 | 2380672 | 2640025 | 3520601 | 5980136 | 650725 | 85 | 85 | 33.33 | 100 | 303 | 1 |
| 940356 | 1110091 | 2360872 | 2640025 | 3520601 | 5420095 | 6450390 | 6520725 | 85 | 85 | 33.33 | 100 | 303 | 1 |
| 610113 | 940356 | 1110091 | 2360672 | 2640025 | 3420026 | 3520601 | 6520725 | 90 | 80 | 33.33 | 100 | 303 | 1 |
| 940356 | 1110091 | 2360672 | 2640025 | 34220026 | 3520601 | 5420095 | 6520725 | 90 | 80 | 33.33 | 100 | 303 | 1 |
| 940356 | 1110091 | 2360672 | 3420026 | 4830255 | 5960136 | 6450380 | 6520725 | 90 | 80 | 33.33 | 100 | 303 | 1 |
| 610113 | 940356 | 1110091 | 2640025 | 3520601 | 4830255 | 5980136 | 6520725 | 95 | 90 | 16.67 | 100 | 302 | 1 |
| 610113 | 940356 | 1110091 | 2360872 | 3420026 | 3520601 | 4830256 | 6520725 | 100 | 85 | 16.67 | 100 | 302 | 1 |
| 610113 | 2360672 | 2640025 | 3520601 | 4830255 | 5960136 | 645039 | 6520725 | 80 | 75 | 66.67 | 80 | 302 | 0 |
| 2360672 | 2640025 | 3420026 | 3520601 | 4830255 | 5960136 | 6450390 | 6520725 | 80 | 75 | 66.67 | 80 | 302 | 0 |
| 610113 | 2360672 | 2640025 | 3420026 | 3520601 | 5420085 | 596013 | 6520725 | 70 | 80 | 50 | 100 | 300 | 0 |
| 610113 | 1110091 | 2360672 | 3420026 | 5420095 | 596013 | 6450390 | 6520725 | 75 | 75 | 50 | 100 | 300 | 0 |
| 1110091 | 2360672 | 2640025 | 3520601 | 420095 | 5960136 | 6450390 | 6520725 | 75 | 75 | 50 | 100 | 300 | 0 |
| 610113 | 940356 | 236072 | 2840025 | 5420085 | 5960136 | 6450380 | 6520725 | 85 | 85 | 50 | 80 | 300 | 1 |
| 610113 | 940356 | 2360672 | 2640025 | 3520601 | 4830255 | 5960136 | 6520725 | 90 | 80 | 50 | 80 | 300 | 1 |
| 610113 | 1110091 | 2360872 | 2640025 | 3420026 | 3520601 | 4830255 | 6450390 | 90 | 80 | 50 | 80 | 300 | 1 |

**[Table 31]**

| Combination of 9 genes | | | | | | | | | | | | | | | |
|---|---|---|---|---|---|---|---|---|---|---|---|---|---|---|---|
| **pb1** | **pb2** | **pb3** | **pb4** | **pb5** | **pb6** | **pb7** | **pb8** | **pb9** | **ccvP0** | **ccvP1** | **ctcP0** | **ctcP1** | **sumP** | **flg_ccv** | **flg_ctc** |
| 610113 | 940356 | 1110091 | 2360672 | 2640025 | 3520601 | 5420095 | 5960136 | 6520725 | 90 | 85 | 33 | 100 | 308 | 1 | 0 |
| 940356 | 1110091 | 2360672 | 2640025 | 3420026 | 3520601 | 5420095 | 5960136 | 6520725 | 90 | 85 | 33 | 100 | 308 | 1 | 0 |
| 610113 | 940356 | 1110091 | 2360672 | 2640025 | 3420025 | 4830255 | 5960136 | 6450390 | 95 | 80 | 50 | 80 | 305 | 1 | 0 |
| 610113 | 1110091 | 2360672 | 2640025 | 3520601 | 4830255 | 5960136 | 6450390 | 6520725 | 80 | 75 | 50 | 100 | 305 | 0 | 0 |
| 610113 | 1110091 | 2640025 | 3420025 | 3520601 | 4830255 | 5960136 | 6450390 | 6520725 | 80 | 75 | 50 | 100 | 305 | 0 | 0 |
| 610113 | 940356 | 1110091 | 2360672 | 2640025 | 3520601 | 5420095 | 6450390 | 6520725 | 85 | 85 | 33 | 100 | 303 | 1 | 0 |
| 610113 | 940356 | 1110091 | 2360672 | 3420026 | 48302255 | 5420095 | 6450390 | 8520725 | 95 | 85 | 33 | 100 | 303 | 1 | 0 |
| 610113 | 2350672 | 2640025 | 3420026 | 3520601 | 4830255 | 5960136 | 6450390 | 6520725 | 80 | 75 | 67 | 80 | 302 | 0 | 0 |
| 610013 | 940356 | 2360672 | 2640025 | 3520601 | 4830255 | 5960136 | 6450390 | 6520725 | 90 | 80 | 50 | 80 | 300 | 1 | 0 |
| 610013 | 940356 | 1110091 | 2360672 | 2640025 | 3420026 | 3520601 | 5420095 | 6520725 | 95 | 80 | 33 | 100 | 298 | 1 | 0 |
| 610013 | 940356 | 1110091 | 2360672 | 2640025 | 3420025 | 3520601 | 5960136 | 6520725 | 95 | 80 | 33 | 100 | 298 | 1 | 0 |
| 610013 | 1110091 | 2360672 | 2640025 | 3420025 | 3520601 | 4830255 | 6450390 | 6520725 | 85 | 80 | 33 | 100 | 298 | 1 | 0 |
| 940356 | 1110091 | 2360672 | 2640025 | 3420026 | 3520601 | 5420095 | 6450390 | 6520725 | 85 | 80 | 33 | 100 | 298 | 1 | 0 |
| 940356 | 1110091 | 2360672 | 2640025 | 3420026 | 3520601 | 5960136 | 6450390 | 6520725 | 80 | 95 | 33 | 100 | 298 | 1 | 0 0 |
| 610113 | 940356 | 1110091 | 2360672 | 3420026 | 4830255 | 5960136 | 6450390 | 6520725 | 90 | 75 | 33 | 100 | 298 | 0 | 0 |
| 610113 | 940356 | 1110091 | 2360672 | 2640025 | 3420026 | 3520601 | 4830255 | 6520725 | 90 | 85 | 17 | 100 | 297 | 1 | 0 |
| 610113 | 940356 | 1110091 | 2360672 | 3420026 | 3520601 | 5420095 | 5960136 | 6520725 | 90 | 90 | 17 | 100 | 297 | 1 | 0 |
| 610113 | 940356 | 1110091 | 2360672 | 3520601 | 4830256 | 5420095 | 5960136 | 6520725 | 95 | 85 | 17 | 100 | 297 | 1 | 0 |
| 940356 | 1110091 | 2360672 | 3420026 | 3520601 | 4830255 | 5420095 | 5960136 | 6520725 | 95 | 85 | 17 | 100 | 297 | 1 | 0 |
| 610113 | 1110091 | 2360672 | 2640025 | 3420026 | 3520601 | 4830255 | 5960636 | 6450390 | 85 | 80 | 50 | 80 | 295 | 1 | 0 |
| 940356 | 2360672 | 2640025 | 3420026 | 4830255 | 5420095 | 5980136 | 6450390 | 6520725 | 85 | 80 | 50 | 80 | 295 | 1 | 0 |
| 610113 | 940356 | 1110091 | 2360672 | 2640025 | 4830255 | 5420095 | 5960136 | 6520725 | 90 | 75 | 50 | 80 | 295 | 1 | 0 |
| 610113 | 940356 | 2360672 | 2640025 | 3420026 | 4830255 | 5960136 | 6450390 | 6520725 | 90 | 75 | 50 | 80 | 295 | 0 | 0 |
| 610113 | 1110091 | 2360672 | 2640025 | 3520601 | 5420095 | 5960136 | 6450390 | 6520725 | 75 | 70 | 50 | 100 | 295 | 0 | 0 |
| 1110091 | 2360672 | 2640025 | 3420026 | 3520601 | 5420095 | 5960136 | 6450390 | 6520725 | 75 | 70 | 50 | 100 | 295 | 0 | 0 |
| 610113 | 940356 | 1110091 | 2360672 | 2640025 | 3520601 | 5960136 | 6450390 | 6520725 | 80 | 80 | 33 | 100 | 293 | 1 | 0 |
| 610113 | 940356 | 1110091 | 2360672 | 3420026 | 3520601 | 5960136 | 6450390 | 6520725 | 80 | 80 | 33 | 100 | 293 | 1 | 0 |
| 1110091 | 2360672 | 2640025 | 3420026 | 3520601 | 4830255 | 5860136 | 6450390 | 6520725 | 80 | 80 | 33 | 100 | 293 | 1 | 0 |
| 610113 | 940356 | 1110081 | 2380872 | 2640025 | 3420026 | 3520601 | 6450390 | 6520725 | 85 | 75 | 33 | 100 | 293 | 0 | 0 |
| 610113 | 1110091 | 2360672 | 2640025 | 3520601 | 4830256 | 5420095 | 6450390 | 6520725 | 75 | 85 | 33 | 100 | 293 | 0 | 0 |
| 610113 | 1110091 | 2360672 | 3520601 | 4830255 | 5420096 | 5960136 | 6450390 | 6520725 | 75 | 85 | 33 | 100 | 293 | 0 | 0 |
| 1110091 | 2360672 | 2640025 | 3520601 | 4830255 | 5420096 | 5960136 | 6450390 | 6520725 | 75 | 85 | 33 | 100 | 293 | 0 | 0 |
| 610113 | 940356 | 1110091 | 2360672 | 2640025 | 3520601 | 4830255 | 6450390 | 6520725 | 90 | 85 | 17 | 100 | 292 | 1 | 0 |
| 610113 | 940356 | 1110091 | 2360672 | 3420025 | 3520601 | 4830255 | 5960136 | 6520725 | 90 | 85 | 17 | 100 | 292 | 1 | 0 |
| 610113 | 940356 | 1110091 | 2360672 | 3420026 | 3520601 | 4830255 | 6450390 | 6520725 | 90 | 85 | 17 | 100 | 292 | 1 | 0 |
| 610113 | 940356 | 1110091 | 2640025 | 3420026 | 3520601 | 5420095 | 5950136 | 6520725 | 90 | 85 | 17 | 100 | 292 | 1 | 0 |
| 610113 | 940356 | 1110091 | 3520601 | 4830255 | 5420095 | 5960136 | 6450390 | 6520725 | 85 | 80 | 17 | 100 | 292 | 1 | 0 |
| 940356 | 1110091 | 2360672 | 2640025 | 3420026 | 3520601 | 4830255 | 6450390 | 6520725 | 90 | 85 | 17 | 100 | 292 | 1 | 0 |
| 940356 | 1110091 | 2360672 | 2640025 | 3520601 | 4830255 | 5420095 | 5960136 | 6520725 | 90 | 85 | 17 | 100 | 292 | 1 | 0 |
| 940356 | 1110091 | 2360672 | 3520601 | 4830255 | 5420095 | 5960136 | 6450390 | 6520725 | 90 | 85 | 17 | 100 | 292 | 1 | 0 |
| 940356 | 1110091 | 3420026 | 3520601 | 4830255 | 5420095 | 5960136 | 6450390 | 6520725 | 85 | 90 | 17 | 100 | 292 | 1 | 0 |
| 610113 | 1110091 | 2360672 | 2640025 | 3420026 | 4830255 | 5420095 | 5960136 | 6450380 | 80 | 80 | 50 | 80 | 290 | 1 | 0 |
| 610113 | 940356 | 2350672 | 2640025 | 4830255 | 5420095 | 5960136 | 6450390 | 6520725 | 85 | 75 | 50 | 80 | 290 | 0 | 0 |
| 610113 | 1110091 | 2360672 | 2640025 | 3420026 | 4830255 | 5960136 | 6450390 | 6520725 | 85 | 75 | 50 | 80 | 290 | 0 | 0 |
| 940356 | 1110091 | 2360672 | 2640025 | 4630256 | 5420885 | 5960136 | 6450390 | 6520725 | 85 | 75 | 50 | 80 | 290 | 0 | 0 |

**[Table 32]**

| Combination of 10 genes | | | | | | | | | | | | | | | | |
|---|---|---|---|---|---|---|---|---|---|---|---|---|---|---|---|---|
| **pb1** | **pb2** | **pb3** | **pb4** | **pb5** | **pb6** | **pb7** | **pb8** | **pb9** | **pb10** | **ccvP0** | **ccvP1** | **ctcP0** | **ctcP1** | **sunP** | **fig-ccv** | **tg_ctc** |
| 610113 | 940356 | 1110091 | 2360672 | 2640025 | 3420025 | 3520601 | 5420095 | 5960136 | 6520725 | 90 | 85 | 33 | 100 | 308 | 1 | 0 |
| 610113 | 940356 | 1110091 | 2360672 | 2640025 | 4830255 | 5420095 | 5960136 | 6450390 | 6520725 | 85 | 85 | 33 | 100 | 303 | 1 | 0 |
| 610113 | 940356 | 1110091 | 2360672 | 2640025 | 3420025 | 3520601 | 5420095 | 6450390 | 6520725 | 85 | 80 | 33 | 100 | 298 | 1 | 0 |
| 610113 | 940356 | 1110091 | 2360672 | 3420025 | 3520601 | 4830255 | 5420095 | 5960136 | 6520725 | 95 | 85 | 17 | 100 | 297 | 1 | 0 |
| 610113 | 940356 | 2360672 | 2640025 | 3420025 | 4830255 | 5420095 | 5960136 | 6450390 | 6520725 | 85 | 80 | 50 | 80 | 295 | 1 | 0 |
| 610113 | 1110091 | 2360672 | 2640025 | 3420025 | 3520601 | 5420095 | 5960136 | 6450390 | 6520725 | 75 | 70 | 50 | 100 | 295 | 0 | 0 |
| 610113 | 940356 | 1110091 | 2360672 | 2640025 | 3420025 | 3520601 | 5960136 | 6450390 | 6520725 | 80 | 80 | 33 | 100 | 293 | 1 | 0 |
| 610113 | 940356 | 1110091 | 2640025 | 3420025 | 3520601 | 4830255 | 5960136 | 6450390 | 6520725 | 80 | 80 | 33 | 100 | 293 | 1 | 0 |
| 610113 | 1110091 | 2360672 | 2640025 | 3520601 | 4830255 | 5420095 | 5960136 | 6450390 | 6520725 | 75 | 85 | 33 | 100 | 293 | 1 | 0 |
| 610113 | 940356 | 1110091 | 2360672 | 2640025 | 3420025 | 3520601 | 4830255 | 6450390 | 6520725 | 90 | 85 | 17 | 100 | 292 | 1 | 0 |
| 610113 | 940336 | 1110091 | 2360672 | 2640025 | 3520601 | 4830255 | 5420095 | 5960136 | 6520725 | 90 | 85 | 17 | 100 | 292 | 1 | 0 |
| 610113 | 940356 | 1110091 | 2360672 | 3520601 | 4830255 | 5420095 | 5960136 | 6450390 | 6520725 | 90 | 85 | 17 | 100 | 292 | 1 | 0 |
| 940356 | 1110091 | 2360672 | 2640025 | 3420025 | 3520601 | 4930255 | 5420095 | 5960136 | 6520725 | 90 | 85 | 17 | 100 | 292 | 1 | 0 |
| 940356 | 1110091 | 2360672 | 2640025 | 3520601 | 4630255 | 5420095 | 5960136 | 6450390 | 6520725 | 90 | 85 | 17 | 100 | 292 | 1 | 0 |
| 940356 | 1110091 | 2360672 | 3420026 | 3520601 | 4830255 | 5420095 | 5960136 | 6450390 | 6520725 | 90 | 85 | 17 | 100 | 292 | 1 | 0 |
| 610113 | 940356 | 1110091 | 2360672 | 2640025 | 4830255 | 5420095 | 5960136 | 6450390 | 6520725 | 85 | 75 | 50 | 80 | 290 | 0 | 0 |

**[Table 33]**

| Combination of 11 genes | | | | | | | | | | | | | | | | | |
|---|---|---|---|---|---|---|---|---|---|---|---|---|---|---|---|---|---|
| **pb1** | **pb2** | **pb3** | **pb4** | **pb5** | **pb6** | **pb7** | **pb8** | **pb9** | **pb10** | **pb11** | **ccvP0** | **ccvP1** | **ctcP0** | **ctcP1** | **sumP** | **flg_ccv** | **flg_ctc** |
| 610113 | 940356 | 1110091 | 2380672 | 3420028 | 3520601 | 4830255 | 5420085 | 5960136 | 6450390 | 6520725 | 90 | 85 | 17 | 100 | 292 | 1 | 0 |

In the above-described tables, pb1 to pb11, ccvP0, ccvP1, ctcP0, ctcP1, sumP, flg_ccv, flg_ctc, etc. are defined as follows:
pb1: Probe 1
pb2: Probe 2
pb3: Probe 3
pb4: Probe 4
pb5: Probe 5
pb6: Probe 6
pb7: Probe 7
pb8: Probe 8
pb9: Probe 9
pb10: Probe 10
pb11: Probe 11
pb12: Probe 12
ccvP0: Rate of correct determination "short life → short life" obtained using training data
ccvP1: Rate of correct determination "long life → long life" obtained using training data
ctcP0: Rate of correct determination "short life → short life" obtained using test data
ctcP1: Rate of correct determination "long life → long life" obtained using test data
sumP: ccvP0 + ccvP1 + ctcP0 + ctcP1
flg_ccv: Both ccvP0 and ccvP1 were 80% or more flg_ctc: Both ctvP0 and ctvP1 were 80% or more

### Example 3

Progressive recurrent prostate cancer patients were classified into a long-lived group (16 cases) that survived for 900 days or longer after personalized peptide vaccination and a short-lived group (14 cases) that died within 300 days after the vaccination. The peptide vaccines were appropriately selected by a physician in consideration of host immunity present before the vaccination. Four peptides (3 mg each of the peptides) at the maximum were subcutaneously administered, together with Freund's incomplete adjuvants, to each patient once a week for 6 weeks. Blood was obtained from the patient before the vaccination and after the vaccination. Peripheral blood mononuclear cells (PBMCs) were prepared by density gradient centrifugation using Ficoll-Paque (GE Healthcare Life Sciences, Uppsala, Sweden). Genes that differed in expression in the peripheral blood mononuclear cells (including granulocytes, lymphocytes, etc.) of the patients between two groups (long-lived group and short-lived group) were analyzed in the same way as in Example 1 using DNA microarrays (Human WG-6 v3.0 Expression BeadChip manufactured by Illumina, Inc.). Microarray data was extracted using BeadStudio v3.0 software (Illumina, Inc.). Fold-change (FC) ranking and P-values obtained using Linear Models for Microarray Data (LIMMA) Bioconductor package were employed for evaluating the difference in gene expression between the long-lived group and the short-lived group. FC was calculated according to log₂FC = log₂ (S_{S}/S_{L}) wherein S_{L} represents the assay range of the target gene in the long-lived group-derived sample; and S_{S} represents the assay range of the target gene in the short-lived group-derived sample.
A volcano plot was prepared with the difference in expression level (log₂FC) as the abscissa and statistical significance (negative log P-value) as the ordinate (Figure 6). In Figure 6, for example, the area in the circle shows genes that largely differed in expression between the long-lived group and the short-lived group after the vaccination.
42 probes corresponding to 36 genes that satisfied the conditions of fold-change ranking (log₂FC < -1.0 or > 1.0) and P-value (P < 0.01) were identified. Of them, 1 gene (LTB) exhibited a decreased expression level in the short-lived group, whereas all the remaining 35 genes exhibited an increased expression level therein (Table 34).

**[Table 34]**

| **Gene symbol** | **Gene name** | **¹Fold change** | **²P-Value** | **³Expression** | **⁴Pre and Post** |
|---|---|---|---|---|---|
| LTB | lymphotoxin beta | -1.03 | 2.01.E-05 | | |
| OLR1 | oxidized low density lipoprotein receptor 1 | 1.04 | 3.76.E-03 | | |
| CEACAM1 | carcinoembryonic antigen-related cell adhesion molecule 1 | 1.07 | 3.09.E-05 | * | |
| ARG1 | arginase, liver | 1.10 | 4.66.E-06 | * | |
| MYL4 | myosin, light chain 4, alkali; atrial, embryonic | 1.14 | 7.10.E-03 | | |
| ALAS2 | aminolevulinate, delta-, synthase 2 | 1.20 | 9.35E-03 | ** | |
| SLPl | secretory leukocyte peptidase inhibitor | 1.22 | 1.58E-05 | * | |
| SELENBP1 | selenium binding protein 1 | 1.22 | 7.56.E-03 | | |
| SNCA | synuclein, alpha | 1.25 | 7.55.E-03 | | |
| AZU1 | azurocidin 1 | 1.25 | 1.89.E-06 | * | # |
| HMGXB4 | HMG box domain containing 4 | 1.27 | 1.07.E-03 | | |
| RNASE3 | ribonuclease, RNase A family, 3 | 1.28 | 9.83.E-04 | * | # |
| HBQ1 | hemoglobin theta 1 | 1.31 | 1.42.E-03 | ** | |
| MMP9 | matrix metallopeptidase 9 | 1.32 | 3.56.E-06 | * | |
| GYPE | glycophorin E | 1.36 | 5.00.E-06 | ** | |
| SNCA | synuclein, alpha | 1.39 | 4.85.E-03 | | |
| EPB42 | erythrocyte membrane protein band 4.2 | 1.45 | 3.00.E-03 | ** | |
| HP | haptoglobin | 1.50 | 2.58.E-05 | ** | |
| IFIT1L | interferon-induced protein with tetratricopeptide repeats 1-like | 1.51 | 2.89.E-03 | | |
| CD24 | CD24 molecule | 1.55 | 8.89.E-05 | * | |
| BPI | bactericidal/permeability-increasing protein | 1.64 | 1.29.E-05 | * | |
| CEACAM6 | carcinoembryonic antigen-related cell adhesion molecule 6 | 1.72 | 1.34.E-06 | * | # |
| PGLYRP1 | peptidoglycan recognition protein 1 | 1.80 | 4.59.E-05 | * | # |
| MPO | myeloperoxidase | 1.94 | 1.03.E-06 | * | # |
| OLFM4 | olfactomedin 4 | 2.01 | 1.34.E-04 | | |
| HBM | hemoglobin, mu | 2.05 | 1.67.E-03 | ** | |
| ALAS2 | aminolevulinate, delta-, synthase 2 | 2.11 | 4.62.E-03 | ** | |
| CEACAM8 | carcinoembryonic antigen-related cell adhesion molecule 8 | 2.13 | 2.97.E-06 | * | # |
| ERAF | erythroid associated factor | 2.29 | 2.33.E-03 | ** | |
| CA1 | carbonic anhydrase I | 2.31 | 3.00.E-04 | * | |
| HBD | hemoglobin, delta | 2.37 | 1.83.E-03 | ** | |
| LCN2 | lipocalin 2 | 2.40 | 1.94.E-05 | * | # |
| CTSG | cathepsin G | 2.40 | 7.60.E-07 | * | # |
| DEFA1 | defensin alphal 1 | 2.40 | 1.20.E-05 | * | # |
| CAMP | cathelicidin antimicrobial peptide | 2.41 | 2.81.E-05 | * | # |
| ELA2 | elastase 2, neutrophil | 2.44 | 9.74.E-07 | * | # |
| DEFA4 | defensin, alpha 4, corticostatin | 2.53 | 3.09.E-07 | * | # |
| DEFA3 | defensin, alpha 3, neutrophil-specific | 2.65 | 7.04.E-06 | * | # |
| DEFA1 | defensin, alpha 1 | 2.65 | 6.70.E-06 | * | # |
| DEFA1 | defensin, alpha 1 | 2.67 | 4.96.E-06 | * | # |
| DEFA1 | defensin, alpha 1 | 2.68 | 6.24.E-06 | * | # |
| DEFA1 | defensin, alpha 1 | 2.87 | 2.15.E-06 | * | # |
| log₂(FC): ²P-value (limma): ³Preferential expression; *Granulocyte, **Erythroid: ⁴ #Identified in both pre- and post-vaccine PBMCs | | | | | |

It is noteworthy that, of the 35 genes whose expression level was increased in the short-lived group, 20 were genes known to be expressed in granulocytes and in relation to their functions. For example, defensins (DEFA1, DEFA3, and DEFA4), ELA2, CTSG, CAMP, and MPO are known to be localized in granules within granulocytic cells. In addition, molecules, such as MMP9 and arginase, which play an important role in tumor growth or immunosuppression, were observed.

Different gene expressions were confirmed by renal-time PCR for 4 genes, i.e., DEFA1, DEFA4, CEACAM8 and MPO, among the genes whose expression level was larger in the short-lived group than in the long-lived group (Figure 7). The real-time PCR was performed using Thermal Cycler Dice Real Time System (Takara Bio Inc.) and also using SYBR Premix Ex Taq II kit (Takara Bio Inc.). The sequences of primers used for defensin alpha, myeloperoxidase (MPO), carcinoembryonic antigen-related cell adhesion molecule 8 (CEACAM8) and GAPDH are as described in the following (a) to (d):
(a) DEFA1
   PCR primer 1: 5'-CGGACATCCCAGAAGTGGTTG-3' (SEQ ID NO: 1)
   PCR primer 2: 5'-CCCTGGTAGATGCAGGTTCCATA-3' (SEQ ID NO: 2)
(b) DEFA4
   PCR primer 1: 5'-CACTCCAGGCAAGAGGTGATGA-3' (SEQ ID NO: 3)
   PCR primer 2: 5'-GAGGCAGTTCCCAACACGAAGT-3' (SEQ ID NO: 4)
(c) CEACAM8
   PCR primer 1: 5'-TGGCACATTCCAGCAATACACA-3' (SEQ ID NO: 5)
   PCR primer 2: 5'-ATCATGATGCTGACAGTGGCTCTA-3' (SEQ ID NO: 6)
(d) MPO
   PCR primer 1: 5'-CTGCATCATCGGTACCCAGTTC-3' (SEQ ID NO: 7)
   PCR primer 2: 5'-GATGCCTGTGTTGTCGCAGA-3' (SEQ ID NO: 8)
(e) GAPDH
   PCR primer 1: 5'-GCACCGTCAAGGCTGAGAAC-3' (SEQ ID NO: 9)
   PCR primer 2: 5'-TGGTGAAGACGCCAGTGGA-3' (SEQ ID NO: 10)

### Example 4

Genes that differed in expression between a long-lived group (20 cases) and a short-lived group (20 cases) were searched for using the peripheral mononuclear cells of patients before vaccination. Both gene expression levels (FC) and limma P-values were lower than those after vaccination (see Example 3). In fact, when genes were selected on the basis of the same values (log₂FC < - 1.0 or > 1.0 and P < 0.01) as those after vaccination, only 5 probes derived from 5 genes were identified as different expressed genes. By contrast, 23 probes derived from 19 genes were selected on the basis of values (log₂FC < -0.6 or > 0.6 and P < 0.05) less strict than the same values. Of these genes, 4 genes (PRKAR1A, LRRN3, PCDH17 and TTN) were decreased in the short-lived group, while 15 genes (LAIR2, RNASE3, CEACAM6, AZU1, HIST1H4C, PGLYRP1, CEACAM8, LCN2, MPO, CAMP, DEFA1, DEFA3, CTSG, DEFA4 and ELA2) were increased therein (Table 35).

**[Table 35]**

| **Gene symbol** | **Gene name** | **¹Fold change** | **²P-Value** | **³Expression** | **⁴Pre and Post** |
|---|---|---|---|---|---|
| PRKAR1A | protein kinase, cAMP-dependent, regulatory, type I, alpha | -0.82 | 4.89.E-02 | | |
| LRRN3 | leucine rich repeat neuronal 3 | -061 | 8.40.E-03 | | |
| PCDH17 | protocadherin 17 | -0.60 | 2.16.E-03 | | |
| TTN | titin | -0.60 | 7.55.E-03 | | |
| LAIR2 | leukocyte-associated immunoglobulin-like receptor 2 | 0.60 | 3.23.E-02 | | |
| RNASE3 | ribonuclease, RNase A family, 3 | 0.63 | 2.01.E-02 | * | # |
| CEACAM6 | carcinoembryonic antigen-related cell adhesion molecule 6 | 0.65 | 9.92.E-03 | * | # |
| AZU1 | azurocidin 1 | 0.66 | 6.37.E-03 | * | # |
| HISHT1H4C | histone cluster 1, H4c | 0.71 | 2.47.E-02 | | |
| PGLYRP1 | peptidoglycan recognition protein 1 | 0.72 | 7.49.E-03 | * | # |
| CEACAMB | carcinoembryonic antigen-related cell adhesion molecule 8 | 0.78 | 1.52.E-02 | * | # |
| LCN2 | lipocalin 2 | 1.00 | 5.26.E-03 | * | # |
| MPO | myeloperoxidase | 1.04 | 1.10.E-03 | * | # |
| CAMP | cathelicidin antimicrobial peptide | 1.09 | 6.78.E-03 | * | # |
| DEFA1 | defensin, alpha 1 | 1.17 | 3.15.E-02 | * | # |
| DEFA1 | defensin, alpha 1 | 1.20 | 1.76.E-02 | * | # |
| DEFA1 | defensin, alpha 1 | 1.26 | 1.76.E-02 | * | # |
| DEFA3 | defensin, alpha 3, neutrophil-specific | 1.27 | 1.65.E-02 | * | # |
| DEFA1 | defensin, apha 1 | 1.27 | 1.97.E-02 | * | # |
| DEFA1 | defensin, alpha 1 | 1.30 | 1.54.E-02 | * | # |
| CTSS | cathepsin G | 1.32 | 2.77.E-03 | * | # |
| DEFA4 | defensin, alpha 4, corticostatin | 1.33 | 2.06.E-03 | * | # |
| ELA2 | elastase 2, neutrophil | 1.36 | 1.64.E-03 | * | # |
| ¹log₂(FC): ²P-value (limma): ³Preferential expression; *Granulocyte: ⁴#Identified in both pre- and post-vaccine PBMCs | | | | | |

It is noteworthy that, of these 15 genes increased in the short-lived group, 13 were granulocyte-specific genes generally identified before and after vaccination.
A most important application of gene expression information based on microarrays is prediction of therapeutic effect. Thus, a study was made on whether gene expression profiles examined using cDNA microarrays in the peripheral mononuclear cells of patients before vaccination were useful in prognostic prediction after the peptide vaccination. A set of four genes (LRRN3, PCDH17, HIST1H4C and PGLYRP1) was selected by variable selection (the stepwise discriminant analysis method) from 23 probes (Table 35) that differed in expression in peripheral mononuclear cells in the 40 cases (long-lived group (20 cases) and short-lived group (20 cases)) before vaccination. This set was used to study prognostic prediction. As a result, the prognosis (long life or short life) after the vaccination could be predicted with respect to 32 patients (80%) out of the 40 patients. Sensitivity (%), specificity (%), positive predictive value, negative predictive value and accuracy (%) were 85% (17/20), 75% (15/20), 77% (17/22), 83% (15/18), and 80% (32/40), respectively (the upper table (Training) of Table 36). For validation, the determination was performed with new independent patients (13 individuals) as subjects using the 4 genes. As a result, the prognosis (long life or short life) after the vaccination could be predicted with respect to 12 patients (93%) out of the 13 patients (the lower table (Test) of Table 36). Sensitivity (%), specificity (%), positive predictive value, negative predictive value, and accuracy (%) were 100% (7/7), 83% (5/6), 88% (7/8), 100% (5/5), and 92% (12/13), respectively. In Table 36, the circled number represents the number of patients who were predicted to belong to the short-lived group and actually had short life, i.e., the number of cases in which the determination of poor prognosis before vaccination was correct, and the boxed number represents the number of patients who were predicted to belong to the long-lived group and actually had short life, i.e., the number of cases in which the determination of good prognosis before vaccination was correct.

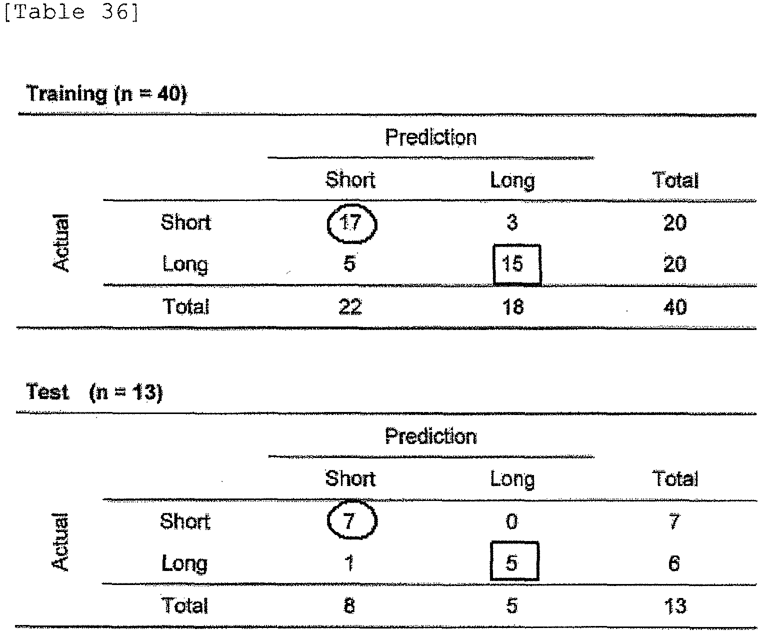

The levels of cytokines, chemokines and growth factors in the plasmas of patients before vaccination were detected using bead-based multiplex assay (xMAP; Luminex Corporation, Austin, TX). The levels of cytokines, chemokines and growth factors including IL-1Rα, IL-1β, IL-2, IL-2R, IL-4, IL-5, IL-6, IL-7, IL-8, IL-10, IL-12, IL-13, IL-15, IL-17, IFN-α, IFN-γ, TNF-α, G-CSF, GM-CSF, IP-10, RANTES, Eotaxin, MIP-1α, MIP-1β, MCP-1, MIG, VEGF, EGF, HGF and FGF basic were measured using a kit (Invitrogen Corporation: Human 30-Plex).
As a result, IL-6 was present at a high content in the plasmas of the short-lived group, compared with the long-lived group (Figure 8).

### Industrial Applicability

The present invention provides a prediction of patients (poor prognosis group) for whom immunotherapy is not expected to be effective and provides information useful in the selection of treatment methods for cancer patients.

The present application claims priority based on Japanese Patent Application No. 2010-147797. The contents thereof are incorporated herein by reference in its entirety.

[Sequencing Listing]

## Claims

1. A method for predicting effect of immunotherapy on a cancer patient, comprising a step of measuring an expression level of each gene in a gene set consisting of at least one gene selected from the group of genes shown in Table 1 or 19 in a sample obtained from the cancer patient before the immunotherapy.

2. The method according to claim 1, wherein the gene set comprises LOC653600, TNFRSF19, P4HA1 and SYNE1.

3. A method for predicting effect of immunotherapy on a cancer patient, comprising a step of measuring an expression level of each gene in a gene set consisting of at least one gene selected from the group of genes shown in Table 34 in a sample obtained from the cancer patient after the immunotherapy.

4. The method according to claim 3, wherein the gene set comprises DEFA1, DEFA4, CEACAM8 and MPO.

5. A method for predicting effect of immunotherapy on a cancer patient, comprising a step of measuring an expression level of each gene in a gene set consisting of at least one gene selected from the group of genes shown in Table 35 in a sample obtained from the cancer patient before the immunotherapy.

6. The method according to claim 5, wherein the gene set comprises LRRN3, PCDH17, HIST1H4C and PGLYRP1.

7. The method according to any of claims 1 to 6, further comprising a step of determining a prognosis of the patient by discriminant analysis using the expression level.

8. The method according to any of claims 1 to 7, for predicting a poor prognosis group.

9. The method according to any of claims 1 to 8, wherein the immunotherapy is peptide vaccine therapy.

10. The method according to any of claims 1 to 9, wherein the cancer is prostate cancer.

11. The method according to any of claims 1 to 10, wherein the sample obtained from the cancer patient is blood.

12. A gene set for predicting effect of immunotherapy on a cancer patient, consisting of at least one gene selected from the group of genes shown in Table 1, 19, 34 or 35.

13. The gene set according to claim 12, wherein the gene set consists of at least one gene selected from the group of genes shown in Table 2 or 22.

14. The gene set according to claim 12 or 13, wherein the gene set comprises LOC653600, TNFRSF19, P4HA1 and SYNE1.

15. The gene set according to any of claims 12 to 14, wherein the gene set comprises DEFA1, DEFA4, CEACAM8 and MPO.

16. The gene set according to any of claims 12 to 15, wherein the gene set comprises LRRN3, PCDH17, HIST1H4C and PGLYRP1.

17. A biomarker for predicting effect of immunotherapy on a cancer patient, consisting of a gene set according to any of claims 12 to 16.

18. A probe designed to specifically hybridize to at least one gene selected from the group of genes shown in Table 1, 19, 34 or 35.

19. A kit comprising a probe according to claim 18 and/or primers specifically hybridizing to at least one gene selected from the group of genes shown in Table 1, 19, 34 or 35.

20. The kit according to claim 19, wherein the probe and the primers are any combination of the following (1) to (4) :
(1) a primer consisting of the sequence of SEQ ID NO: 1, a primer consisting of the sequence of SEQ ID NO: 2, and a probe consisting of the sequence of SEQ ID NO: 3;
(2) a primer consisting of the sequence of SEQ ID NO: 4, a primer consisting of the sequence of SEQ ID NO: 5, and a probe consisting of the sequence of SEQ ID NO: 6;
(3) a primer consisting of the sequence of SEQ ID NO: 7, a primer consisting of the sequence of SEQ ID NO: 8, and a probe consisting of the sequence of SEQ ID NO: 9; and
(4) a primer consisting of the sequence of SEQ ID NO: 10, a primer consisting of the sequence of SEQ ID NO: 11, and a probe consisting of the sequence of SEQ ID NO: 12.

21. A method for predicting effect of immunotherapy on a cancer patient, comprising a step of measuring an expression level of IL-6 protein in blood obtained from the cancer patient before the immunotherapy.
